(19) Europäisches Patentamt
European Patent Office
Office européen des brevets

(11) EP 4 168 801 B1

(12) EUROPEAN PATENT SPECIFICATION

(45) Date of publication and mention of the grant of the patent:
**19.03.2025 Bulletin 2025/12**

(21) Application number: **21742044.7**

(22) Date of filing: **22.06.2021**

(51) International Patent Classification (IPC):
***G01N 33/50*** *(2006.01)*

(52) Cooperative Patent Classification (CPC):
**G01N 33/5091;** G01N 2800/367

(86) International application number:
**PCT/EP2021/066927**

(87) International publication number:
**WO 2021/259903 (30.12.2021 Gazette 2021/52)**

# (54) SPERM STRATIFICATION

SPERMA-STRATIFIZIERUNG

STRATIFICATION DE SPERMATOIZOÏDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2020 LU 101881**

(43) Date of publication of application:
**26.04.2023 Bulletin 2023/17**

(73) Proprietor: **Westfälische Wilhelms-Universität Münster**
**48149 Münster (DE)**

(72) Inventors:
- **SCHLATT, Stefan**
  **48149 Münster (DE)**
- **REDMANN, Klaus**
  **48149 Münster (DE)**
- **RAUL ADRIAN, Da Costa Graterol**
  **48149 Münster (DE)**

(74) Representative: **Schiweck Weinzierl Koch Patentanwälte Partnerschaft mbB**
**Ganghoferstraße 68 B**
**80339 München (DE)**

(56) References cited:
- **MOSKOVTSEV S. I. ET AL: "Sperm Dna Integrity: Correlation With Sperm Plasma Membrane Integrity In Semen Evaluated For Male Infertility", vol. 51, no. 1, 1 January 2005 (2005-01-01), US, pages 33 - 40, XP055783472, ISSN: 0148-5016, Retrieved from the Internet <URL:https://www.tandfonline.com/doi/pdf/10.1080/014850190512770?needAccess=true> DOI: 10.1080/014850190512770**
- **MD. SHAROARE HOSSAIN ET AL: "Flow cytometry for the assessment of animal sperm integrity and functionality: state of the art", ASIAN JOURNAL OF ANDROLOGY, vol. 13, no. 3, 11 April 2011 (2011-04-11), US, pages 406 - 419, XP055292750, ISSN: 1008-682X, DOI: 10.1038/aja.2011.15**
- **AL-ESSAWE ESSRAA M ET AL: "Improved cryosurvival of stallion spermatozoa after colloid centrifugation is independent of the addition of seminal plasma", CRYOBIOLOGY, ACADEMIC PRESS INC, US, vol. 81, 15 February 2018 (2018-02-15), pages 145 - 152, XP085386577, ISSN: 0011-2240, DOI: 10.1016/J.CRYOBIOL.2018.01.009**
- **HERBEMONT C ET AL: "Comment choisir le spermatozoïde en ICSI ?", GYNECOLOGIE OBSTETRIQUE & FERTILITE, ELSEVIER MASSON, FR, vol. 42, no. 11, 18 September 2014 (2014-09-18), pages 800 - 805, XP029091134, ISSN: 1297-9589, DOI: 10.1016/J.GYOBFE.2014.07.030**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 168 801 B1

- MOSKOVTSEV SERGEY I ET AL: "Testicular spermatozoa have statistically significantly lower DNA damage compared with ejaculated spermatozoa in patients with unsuccessful oral antioxidant treatment", FERTILITY AND STERILITY, vol. 93, no. 4, 1 March 2010 (2010-03-01), pages 1142 - 1146, XP029105710, ISSN: 0015-0282, DOI: 10.1016/ J.FERTNSTERT.2008.11.005

## Description

### FIELD OF THE INVENTION

The present invention relates to a method for stratifying spermatozoa in a sample obtained from a subject, comprising assaying the spermatozoa for membrane integrity; and assaying the spermatozoa for DNA fragmentation. Thereby, the spermatozoa stratification method allows an analysis of defined sperm categories and the prediction whether or not the spermatozoa will be functional. Further, a kit is described herein but not claimed for performing the method for sperm stratification.

### BACKGROUND OF THE INVENTION

It is known for a long time that sperm DNA damage is prevalent among infertile men. Sperm DNA integrity has thus become one of the most discussed biomarker in basic and clinical andrology in the last decades. This parameter contains a large informative content about the testicular function and is clinically significant in case of infertility, not only in terms of diagnostic, but also in terms of treatment selection. Furthermore, it negatively effects fertilization, embryo development, implantation and pregnancies in natural reproduction. Similar observations have been reported on clinical pregnancy, following treatments of *in vitro fertilization* (IVF) and *intracytoplasmic sperm injection* (ICSI).

The link between DNA fragmentation and negative clinical outcomes has been addressed using several methodologies, of which two flow cytometry methods can be highlighted, the *Terminal deoxynucleotidyl transferase* (TdT) *dUTP Nick-End Labeling* (TUNEL) and the *Sperm Chromatin Structure Assay* (SCSA). These are two used tests to measure sperm DNA damage, and although they provide values of sperm DNA fragmentation, they are based on different principles and have their own advantages and limitations. Further, for example, Moskovtsev et al. (Archives of Andrology, 51:33-40, 2005) also uses two tests to evaluate the relationship between sperm plasma membrane functional integrity - assessed by the hypo-osmotic swelling test (HOST) - and sperm DNA integrity - assesed by DNA fragmentation index (DFI). Unfortunately, there is no reference method and association not necessarily implies diagnostic accuracy. One of the main reasons for this observation is that most of the currently available methods of DNA evaluation do not consider the distinction between live or dead cells in terms of membrane integrity, and thus underestimate the amount of dead cells with intact DNA, when normally the membrane intact spermatozoa are the ones used and wanted for *assisted reproductive technologies* (ART). These limitations not only affect the diagnostic power of the assay, but also make it difficult to reveal the etiology of the sperm DNA fragmentation.

Thus, there remains a need in the art for a refined method of sperm stratification usable in clinics and fertility analysis. The evaluation of the DNA fragmentation in living populations of spermatozoa using an accurate and easy to apply method would improve the diagnostic of male infertility and guide the treatment efficiently. Moreover, it would also help to understand diverse cellular mechanism including apoptosis and oxidative stress at testicular, epididymal and/or spermatic level.

The technical problem underlying the present application is thus to comply with these needs. The technical problem is solved by providing the embodiments reflected in the claims, described in the description and illustrated in the examples and figures that follow.

### SUMMARY OF THE INVENTION

Described herein is a method for stratifying spermatozoa in a sample obtained from a subject, said method comprises

(a) assaying spermatozoa comprised by said sample for membrane integrity; and
(b) assaying spermatozoa comprised by said sample for DNA fragmentation,

wherein the stratification allows a prediction whether said subject will have spermatozoa which may be functional. It is described herein that the spermatozoa are stratified as membrane non-intact spermatozoa, having a DNA fragmentation index (%DFI) portion of at most about 5 %, membrane non-intact spermatozoa, having a DNA fragmentation index (%DFI) portion of at least about 1 %, membrane intact spermatozoa, having a DNA fragmentation index (%DFI) portion of at most about 5 %, and membrane intact spermatozoa, having a DNA fragmentation index (%DFI) portion of at least about 1 %, in particular the percentage of DNA fragmentation index (%DFI) of the sample lays between about 4 %DFI to about 75 %DFI. It is encompassed that the presence of spermatozoa, which are membrane intact spermatozoa, having a DNA fragmentation (%DFI) portion of at most about 5 %, may be indicative that said subject may have spermatozoa which may be functional. It is described herein that the presence of spermatozoa, which are membrane non-intact spermatozoa, having a DNA fragmentation (%DFI) portion of at most about 5 %, which are membrane non-intact spermatozoa, having a DNA fragmentation (%DFI) portion of at least about 1 %, and which are membrane intact spermatozoa, having a DNA fragmentation (%DFI) portion of at least about 1 %, may be predictive that said subject may have spermatozoa, which may not be functional. The method of the present invention is a method for stratifying spermatozoa comprised in a sample obtained from a subject, comprising (a) assaying spermatozoa comprised by said sample for membrane integrity; and (b) assaying spermatozoa comprised by said sample for DNA fragmentation, wherein the stratification allows a prediction whether said subject will have spermatozoa which may be functional, wherein spermatozoa are stratified as follows:

(i) membrane non-intact spermatozoa, having a DNA fragmentation index (%DFI) portion of at most about 5 %;
(ii) membrane non-intact spermatozoa, having a DNA fragmentation index (%DFI) portion of at least about 1 %;
(iii) membrane intact spermatozoa, having a DNA fragmentation index (%DFI) portion of at most about 5 %;
(iv) membrane intact spermatozoa, having a DNA fragmentation index (%DFI) portion of at least about 1 %;

wherein the percentage of DNA fragmentation index (%DFI) of said sample is between about 4 %DFI to about 75 % DFI, wherein the presence of spermatozoa pursuant to (iii) is indicative that said subject will have spermatozoa which may be functional, and wherein the presence of spermatozoa pursuant to (i), (ii) and/or (iv) is predictive that said subject will have spermatozoa, which may not be functional. It is envisaged that a sperm sample that may have a %DFI between about 4 %DFI to about 30 %DFI may contain predominantly spermatozoa, which may be functional. In the light of the present invention, a sperm sample that may have a %DFI between about 30 %DFI to about 75 %DFI may contain predominantly spermatozoa, which may not be functional. It is encompassed by the method of the present invention that if the subject should predominantly have spermatozoa, which may be membrane non-intact spermatozoa, having a DNA fragmentation index (%DFI) portion of at most about 5 %, which may be membrane non-intact spermatozoa, having a DNA fragmentation index (%DFI) portion of at least about 1 %, and which may be membrane intact spermatozoa, having a DNA fragmentation index (%DFI) portion of at least about 1 %, such spermatozoa may be used for IVF or ICSI.

In another aspect, the present invention relates to a method for determining whether functional spermatozoa are present in a sample with spermatozoa obtained from a subject, said method comprises assaying spermatozoa comprised by said sample for membrane integrity and assaying spermatozoa comprised by said sample for DNA fragmentation, and the presence of membrane intact spermatozoa having a DNA fragmentation index (%DFI) portion of at most about 5 % is predictive that said subject will have spermatozoa, which may be functional.

A further aspect of the present invention relates to a method for determining whether non-functional spermatozoa are present in a sample comprising spermatozoa obtained from a subject, said method comprising assaying spermatozoa comprised by said sample for membrane integrity and assaying spermatozoa comprised by said sample for DNA fragmentation, and the presence of membrane non-intact spermatozoa, having a DNA fragmentation index (%DFI) portion of at most about 5 % in said sample is predictive that said subject will have spermatozoa, which may not be functional, and the presence of membrane non-intact spermatozoa, having a DNA fragmentation index (%DFI) portion of at least about 1 % in said sample is predictive that said subject will have spermatozoa, which may not be functional, and/or the presence of membrane intact spermatozoa, having a DNA fragmentation index (%DFI) portion of at least about 1 % in said sample is predictive that said subject will have spermatozoa, which may not be functional. Preferably, the functional and/or non-functional spermatozoa are subject to one or more viability tests.

In view of the method of the present invention, the membrane integrity may be assayed by a dye differentiating between live and dead cells. It is envisaged that the DNA fragmentation may be assayed by a dye differentiating intact and fragmented DNA. In particular, it is envisaged by the method of the present invention that flow cytometry may be used for assaying said sample for membrane integrity and/or for assaying said sample for sperm DNA fragmentation.

Also it is encompassed by the method of the present invention that assaying a sample comprising spermatozoa for membrane integrity may include determining the number of spermatozoa not showing membrane integrity and/or determining the number of spermatozoa showing membrane integrity. Preferably, the method of the present invention comprises assaying a sample comprising spermatozoa for sperm DNA fragmentation including determining the number of spermatozoa comprising fragmented DNA and/or determining the number of spermatozoa not comprising fragmented DNA.

It is further envisaged that the method of the present invention may determine the number of membrane intact DNA fragmented spermatozoa, which may be set in ratio to the total number of membrane intact spermatozoa, thereby indicting the DNA and membrane integrity (DMI) ratio:

$$DMI\ ratio = \frac{membrane\ intact\ DNA\ fragmented\ spermatozoa}{total\ membrane\ intact\ spermatozoa}.$$

In particular, it is encompassed by the present invention that functional spermatozoa may be potentially fertile spermatozoa. In view of the method of the present invention, non-functional spermatozoa may be potentially infertile spermatozoa.

In view of the method of the present invention, it is encompassed that said method for stratifying or determining of spermatozoa preferably may comprise the steps of: 1) assaying spermatozoa for membrane integrity (a) and DNA fragmentation (b); 2) sorting the spermatozoa according to the dye used for (a) and (b); 3) selecting the majority of spermatozoa according to their FSC-A and SSC-A signal; 4) analyzing the fluorescence values in a histogram representing

(a) for individual spermatozoa; 5) analyzing the fluorescence values in a histogram representing
(b) for individual spermatozoa; 5)i) calculating the %DFI from the fluorescence values representing (b) for individual spermatozoa obtained in the previous step; 6) combining the fluorescence values of (a) and (b) obtained from analyzing the fluorescence values representing (a) and (b) respectively per sample in a bivariate logarithmic density plot of the fluorescence intensity; and 6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds. It is encompassed that calculating the percentage of the DNA fragmentation index (%DFI) per sample, may be established by determining the number of spermatozoa with moderate and high DNA fragmentation in relation to the number of spermatozoa with low DNA fragmentation from the fluorescence values in the histogram representing (b) of individual spermatozoa of said sample anylyzed in 5).

It is encompassed that the spermatozoa of said sample may thereby be stratified or determined as follows:

(i) membrane non-intact spermatozoa, having a DNA fragmentation index (%DFI) portion of at most about 5 %;

(ii) membrane non-intact spermatozoa, having a DNA fragmentation index (%DFI) portion of at least about 1 %;

(iii) membrane intact spermatozoa, having a DNA fragmentation index (%DFI) portion of at most about 5 %;

(iv) membrane intact spermatozoa, having a DNA fragmentation index (%DFI) portion of at least about 1 %; and wherein the percentage of DNA fragmentation index (%DFI) of said sample is between about 4 %DFI to about 75 % DFI.

In another aspect, the present invention relates to a method for stratifying or determining spermatozoa, wherein stratifying or determining of spermatozoa comprises 1) assaying spermatozoa for membrane integrity (a) and DNA fragmentation (b); 2) sorting the spermatozoa according to the dye used for (a) and (b); 3) selecting the majority of spermatozoa according to their FSC-A and SSC-A signal; 4) analyzing the fluorescence values in a histogram representing (a) for individual spermatozoa; 5) analyzing the fluorescence values in a histogram representing (b) for individual spermatozoa; 6) combining the fluorescence values of (a) and (b) obtained in the steps 4) and 5) per sample in a bivariate logarithmic density plot of the fluorescence intensity, and 6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds.

It is encompassed by the present invention that the spermatozoa may be stratified or determined as follows:

I. membrane non-intact spermatozoa having non-fragmented DNA, with a spermatozoa number between about 20 % and about 60 % of the total spermatozoa number, preferably about 35 % of the total spermatozoa number measured per sample;
II. membrane non-intact spermatozoa having fragmented DNA, with a spermatozoa number of at least about 1 % of the total spermatozoa number measured per sample;
III. membrane intact spermatozoa having non-fragmented DNA, with a spermatozoa number between about 45 % and about 75 % of the total spermatozoa number, preferably about 55 % of the total spermatozoa number measured per sample; and
IV. membrane intact spermatozoa having fragmented DNA, with a spermatozoa number of at least about 1 % of the total spermatozoa number measured per sample.

It is envisaged that the spermatozoa numbers of I to IV may allow the calculation of the DNA and membrane integrity (DMI) ratio, wherein the number of membrane intact DNA fragmented spermatozoa (IV) is set in ratio to the total number of membrane intact spermatozoa (III and IV), thereby indicating the DNA and membrane integrity (DMI) ratio as described herein.

In a further embodiment of the method of the present invention, a DNA intercalating dye may be applied for the evaluation of DNA fragmentation. Preferably, the DNA intercalating dye applied for the evaluation of DNA fragmentation is a fluorescent dye. Said fluorescent dye of the method of the present invention may be an intercalating dye applied for the evaluation of DNA fragmentation, preferably acridine orange (AO).

Further, it is envisaged that the dye differentiating live and dead cells may be reactive with amines. Said dye for differentiating live and dead cells for the method of the present invention may be reactive with amines and may preferably be a fluorescent dye. It is preferred that said dye for differentiating live and dead cells, which is reactive with amines, is a fluorescent dye. In particular, it is preferred that the fluorescent dye differentiating live and dead cells, which is reactive with amines, is a LIVE/DEAD Fixable Dead Cell Stain.

It is encompassed that the dye for assaying DNA fragmentation and the dye for assessing membrane integrity may have a different emission spectrum, thereby avoiding a spectral overlap.

In another aspect, the present invention relates to a method for stratifying spermatozoa comprised in a sample from a subject, said method comprising (a) assaying spermatozoa comprised by said sample for membrane integrity; (b) assaying spermatozoa comprised by said sample for DNA fragmentation; and thereby spermatozoa are stratified as membrane non-intact spermatozoa with prevalent double stranded DNA, membrane non-intact spermatozoa with prevalent single stranded DNA or RNA, membrane intact spermatozoa with prevalent double stranded DNA, and membrane intact spermatozoa with prevalent single stranded DNA or RNA.

It is envisaged that a DNA intercalating dye may be applied for the evaluation of single and double stranded DNA. In particular, it is envisaged that the DNA intercalating dye applied for the evaluation of single and double stranded DNA may be a fluorescent dye. In the method of the present invention, the dye differentiating membrane intact and membrane not-intact spermatozoa may be reactive with amines. It is encompassed that this dye differentiating membrane intact and membrane not-intact spermatozoa, which may be reactive with amines may be a fluorescent dye. Said dye for the evaluation of single and double stranded DNA and said dye differentiating membrane intact and membrane not-intact spermatozoa, which is reactive with amines, may have a different emission spectrum, thereby avoiding a spectral overlap.

It is encompassed that step (a) and step (b) may be performed simultaneously or subsequently irrespective of the order, whereas step (a) is preferably performed prior to step (b).

Preferably, the subject is a mammal, bird or fish.

Further described herein but not claimed is a kit for performing the method of the present invention which comprises a DNA intercalating dye for the evaluation of DNA fragmentation and a dye differentiating live and dead cells, and further comprises calibration beads and tubal fluid buffer of mammal, bird or fish. Further described herein but not claimed is that said kit comprises a DNA intercalating dye applied for the evaluation of DNA fragmentation, which may be a fluorescent dye. It is also described herein but not claimed that said kit comprises a fluorescent DNA intercalating dye applied for the evaluation of DNA fragmentation, which may be acridine orange (AO). Additionally, it is described herein but not claimed that said kit comprises a dye differentiating live and dead cells, which may be reactive with amines. It is further described herein but not claimed that said kit comprises a dye differentiating live and dead cells, which may be reactive with amines and may be a fluorescent dye. It is also described herein but not claimed that said kit comprises a fluorescent dye differentiating live and dead cells, which may be reactive with amines and may be a LIVE/DEAD Fixable Dead Cell Stain.

## BRIEF DESCRIPTION OF THE FIGURES

**Figure 1: Values of sperm membrane integrity obtained using independent probes and the LD+AO co-staining.**

(A) Distribution of percentages of intact membrane spermatozoa evaluated using propidium iodide (PI) or LIVE/DEAD combined with acridine orange.
(B) Correlation of membrane intact spermatozoa evaluated by using propidium iodide (PI) or LIVE/DEAD combined with acridine orange. MIS = Membrane Intact Spermatozoa.

**Figure 2: Method agreement analysis of the evaluation of sperm membrane integrity and DNA fragmentation.**

(A) Propidium iodide vs. LIVE/DEAD (+AO).
(B) SCSA vs AO (+LD). The difference between the measurements, as assessed by each method, was plotted against their average. The limits of agreement (d $\pm$ 2 SD) are given.

**Figure 3: Values of sperm DNA fragmentation obtained using independent probes and the LD+AO co-staining.**

(A) Percentages of spermatozoa with DNA fragmentation evaluated using SCSA or AO combined with LD. The box plot elements are defined as follows: center line: median; box limits: upper and lower quartiles; whiskers: minimum and maximum. Statistical analyzes showed no differences between the two groups P = 0.755 (membrane integrity) and P = 0.976 (DNA fragmentation).
(B) Correlation of DNA fragmentation index (%DFI) evaluated using acridine orange only or acridine orange combined with LIVE/DEAD.

**Figure 4: Method agreement analysis of the evaluation of DNA fragmentation index (DFI).** The difference between the measurements, as assessed by each method, was plotted against their average. The limits of agreement (d $\pm$ 2 SD) are given.

**Figure 5: Gating strategy and representative cytograms.**

(A) The gate applied to spermatozoa.
(B) Gate to calculate the level of DNA fragmentation. In the panel, the Red/Green fluorescence values of the Acridine orange (AO) staining for each cell are represented.
(C) Histogram of the calculated level of DNA fragmentation for each cell. The histogram contains the gate to quantify the percentage of spermatozoa with high DNA fragmentation.
(D) Spermatozoa stained with LIVE/DEAD™ Fixable Blue. The histogram depicts two subpopulations. The subpopulation to the left represents live spermatozoa (membrane intact) and one to the right represents dead spermatozoa (membrane non-intact).

**Figure 6: Combined staining plot and DNA and Membrane Integrity Ratio.**
The X-axis represents values of DNA fragmentation and the Y-axis fluorescence values of LIVE/DEAD™ Fixable Blue. Four sperm subpopulations can be identified. (Q1) membrane non-intact with no DNA fragmentation, (Q2) membrane non-intact with DNA fragmentation, (Q3) membrane intact with no DNA fragmentation, (Q4) membrane intact with DNA fragmentation.

**Figure 7: DNA and Membrane Integrity Ratio in** % (Formulation of the DNA and Membrane Integrity Ratio (DMI Ratio or DMI%) considering only membrane intact spermatozoa).

Figure **8:** Population's **distribution** on **extreme conditions.**

**(A)** Positive control for membrane integrity, Triton X-100 exposed spermatozoa; and
**(B)** for DNA fragmentation, UVB light 30 min exposed spermatozoa.

**Figure 9: Visualization of increasing degrees of membrane and DNA damage in the sperm population, measured by TUNEL + LIVE/DEAD.**

**(A)** Native sample;
**(B)** 20 % positive cells (positive control);
**(C)** 40 % positive cells;
**(D)** 60 % positive cells;
**(E)** 80 % positive cells; and
**(F)** 100 % positive cells.

**Figure 10: Visualization of increasing degrees of membrane and DNA damage in the sperm population.**

**(A)** Native sample;
**(B)** mixed sample 20% positive control (PC);
**(C)** mixed sample 40% PC;
**(D)** mixed sample 60% DFI;
**(E)** mixed sample 80% DFI; and
**(F)** 100% PC (exposed to UVB 312 nm for 10 min). The value comparison of DNA and Membrane Integrity Ratio vs. DNA fragmentation index (DMI|DFI %) is showed for each condition.

**Figure 11: Theoretical and experimental values of the co-staining AO + LD.**

**(A)** Values for membrane intact spermatozoa (MIS).
**(B)** Values for DNA fragmentation. n = 3, mean ± SD.

**Figure 12: Association between the values of DNA damage against the amount of DNA damage in the sperm population for TUNEL + LIVE/DEAD and LIVE/DEAD + acridine orange.**

**(A)** Acridine orange + LIVE/DEAD, TUNEL + LIVE/DEAD.
**(B)** Is showing the DIM Ratio sensitivity, plotting damaged cells against DMI.

**Figure 13: Evaluation of DNA integrity by acridine orange staining.**

**(A)** Stained spermatozoa under fluorescence microscopy showing different degrees of DNA fragmentation.
**(B)** Histogram representing individual values of DNA fragmentation in the evaluated sperm population.

## DETAILED DESCRIPTION OF THE INVENTION

Current sperm analytic methods misjudge the DNA fragmentation of spermatozoa, because necrotic and/or dead cells are included into the DNA fragmentation analysis, possibly resulting in a wrong clinical prediction of the male fertility. Herein presented is an advanced method for sperm stratification, which allows a refined assessment of the sperm quality. Not only the DNA fragmentation, but also the membrane integrity is analyzed, resulting in four sperm categories; membrane non-intact spermatozoa, having low levels of fragmented DNA, membrane non-intact spermatozoa, having high levels of fragmented DNA, membrane intact spermatozoa, having low levels of fragmented DNA, and membrane intact spermatozoa, having high levels of fragmented DNA. Importantly, this method allows the analysis of the DNA fragmentation of intact/ living spermatozoa and hence gives a more precise picture of the male fertility. To reach a precisely resolved sperm stratification as described herein, the spermatozoa are assayed for their DNA integrity in combination with their membrane integrity, as described herein by using preferably fluorescent dyes. The resulting data may be processed in a way described herein, and the DNA and Membrane Integrity Ratio (DMI) can be calculated.

The membrane integrity is analyzed by using a LIVE/DEAD staining, which is based on the reaction of a fluorescent reactive dye with cellular proteins (amines). The reactive dye can permeate into the damaged membrane of dead cells and stain both the interior and exterior amines, resulting in an intense staining, discriminating living and dead cells. A sperm DNA fragmentation evaluation is achieved by using a special DNA staining agent, called acridine orange (AO), which is capable of emitting light of different wavelengths when bound to single (damaged) or double stranded (intact) DNA, which allows an assessment of the sperm DNA damage. The sperm stratification method of the present invention is based on the finding that acridine orange and LIVE/DEAD assay protocols can be combined, resulting in a new valuable information source and further resulting in the calculated DNA membrane integrity ratio. This assay combination is only possible if the LIVE/DEAD staining is performed before the acridine orange staining, due to the very low pH required for the removal of chromatin and hence the accessibility of the DNA. Such a low pH could wash out other membrane stainings and interfere with the diagnostic results. Also, the posterior staining with acridine orange could have affected the outcome of the sperm membrane assay, which was investigated in detail. It was hence not known whether both protocols could be performed together and whether the combination would compromise the readout of either the DNA fragmentation assay or the membrane staining. Therefore, the compatibility of the protocols was investigated, showing no interferences. Furthermore, both stainings were optimized to a non-overlapping fluorophore emission wavelength to allow a simultaneous analysis of both stained dyes. The method of the present invention is cost effective, easy to perform, quick and applicable for a clinical use. Importantly, by using the method of the present invention, no cells are lost due to excessive manipulation (e.g., multiple centrifugations, DTT induced chromatin de-compaction, fixation, membrane permeabilization, antibody/fluorochrome incubation), and thus no membrane integrity or DNA fragmentation values are lost either, which is the case of other commonly used methods.

Further, the advantages of the method of the present invention over the common methods were investigated in great detail. A commonly used staining to detect dead cells is propidium iodide (PI), which is not permeant to living cells. The membrane integrity methods were compared using propidium iodide (PI) and LIVE/DEAD staining **(Fig. 1 and 2).** Further, the DNA fragmentation was analyzed by comparing acridine orange, and an acridine orange combination with LIVE/DEAD staining **(Fig. 3 and 4),** both showed comparable results answering whether the two assays combined in the present invention interfere with each other, which was not the case.

Finally, a combination of both, the membrane staining (e.g., LIVE/DEAD), and the DNA fragmentation staining (e.g., acridine orange) revealed a detailed picture of the four described sperm categories, namely (Q1) necrotic/ dead spermatozoa with low levels of DNA fragmentation, (Q2) necrotic/ dead spermatozoa with high levels of DNA fragmentation, (Q3) membrane intact spermatozoa with low levels of DNA fragmentation (functional), and (Q4) membrane intact spermatozoa with high levels of DNA fragmentation **(Fig. 6).** Especially, necrotic/ dead cells with fragmented DNA and membrane intact/alive cells with high levels of DNA fragmentation are not resolved by the other analytic methods, two cell populations, which can sum up to a cell count of about 40 % to 60 % in a standard sperm sample. Furthermore, the two cell populations of functional sperm and sperm with intact membrane, but defective DNA allow the calculation of a *DNA Membrane Integrity Ratio* **(Fig. 7)** and provide an advanced information source over the commonly used *DNA fragmentation index* as described herein.

To further validate the method of the present invention, typical positive controls for the membrane integrity **(Fig. 8A)** and the DNA fragmentation **(Fig. 8B)** were produced, by the exposure of spermatozoa to a detergent (Triton X-100) or UVB light for 30 minutes. The inventors compared the classical DNA TUNEL assay (which is based on the integration of labeled nucleotides by the enzymatic action of a *Terminal deoxynucleotidyl transferase* (TdT)) in combination with a LIVE/DEAD staining **(Fig. 9),** to the method of the present invention combining acridine orange and LIVE/DEAD staining **(Fig. 10).** In this experiment, the sperm was mixed with 0 to 100 % destroyed spermatozoa to assess the analytic power of the TUNEL + LIVE/DEAD in comparison to the method of the present invention, an acridine orange + LIVE/DEAD combination. While TUNEL + LIVE/DEAD recognized 98 % of the native sperm sample as intact, the combination of acridine orange and LIVE/DEAD staining revealed about 30 % of the sperm as spermatozoa with intact DNA, but with membrane damage. The method of the present invention was capable of resolving virtually all sperm cells of the 100 % destroyed sperm sample as necrotic with defective DNA, thus showing a high analytic sensitivity.

The lower accuracy of the TUNEL + LIVE/DEAD assay to evaluate membrane integrity and DNA damage is not surprising. It is well known that protocols with excessive sample manipulation like the TUNEL are prone to a random loss of cells, which results in an inaccurate evaluation of the cell vitality. It has been reported that the TUNEL assay tends to low sensibility attributed to the high compaction of sperm chromatin, which prevents the effective enzymatic action of the Terminal deoxynucleotidyl transferase. This issue is solved in the acridine orange + LIVE/DEAD assays by the use of a low pH solution, which denatures DNA at sites of DNA strand breaks.

Furthermore, a calculation of the DNA damage in relation to the positive control with a set percentage of damaged sperm revealed an accuracy of 97 % for the LIVE/DEAD + TUNEL combination, while the method of the present invention reached 99 % accuracy **(Fig. 12).** In other words, the method of the present invention is more accurate, when it comes to analyzing the DNA damage to spermatozoa, and has the advantage of resolving whether live or dead cells have damaged DNA.

The method of the present invention, given its practicality, can be used in research for understanding the cellular basis of the etiology of sperm DNA damage and the study of specific sperm subpopulations going through oxidative stress/ apoptotic-like processes. Furthermore, the method of the present invention has a huge potential to be implemented in clinical settings for the evaluation of unexplained andrological conditions, providing fast and accurate practical information.

### Spermatozoa assay

The present invention describes that spermatozoa are assayed, allowing the stratification of different sperm categories. Generally, the term "spermatozoa" refers to mature ejaculated male gametes, which are able to fertilize the female gamete counterpart. The term "spermatozoa" is used interchangeably with the term "cells" herein. In addition to that, the term "sperm" refers to spermatozoa and seminal plasma. The term "sperm" also encompasses subpopulations of sperm (e.g., sorted sperm, marked sperm, frozen sperm, purified sperm, motile sperm subpopulation, etc.). Said spermatozoa are comprised in a sperm sample obtained from a subject, which is commonly male. The subject of the present invention may be a mammal, bird or fish.

A "mammalian subject", a "mammal" or "mammalian" includes cats, dogs, horses, pigs, cows, goats, sheep, rodents (e.g., mice or rats, rabbits), primates (e.g., chimpanzees, monkeys, gorillas, and humans) and endangered mammals (such as non-human primates, elephants, rhinos, bears). The method can be carried out with sperm from domesticated animals, and livestock sperm of wild animals. Sperm samples may or may not be cryopreserved. Any mammal may be the subject of the present invention donating sperm. Preferably, the selected sperm sample is derived from a human subject and contains human spermatozoa.

A "bird" or aves is a group of endothermic vertebrates, characterized by feathers, toothless beaked jaws, the laying of hard-shelled eggs, a high metabolic rate, a four-chambered heart, and a strong yet lightweight skeleton and are to be understood as such in the context of the present invention. The method can be carried out with sperm from domesticated animals, and livestock sperm of wild animals. Sperm samples may or may not be cryopreserved. A kind of bird may be the subject described in the present invention donating sperm.

A "fish" refers to an animal group of gill-bearing aquatic craniate vertebrates that lack limbs with digits. They form a sister group to the tunicates, together forming the olfactores. Included in this definition are the living hagfish, lampreys, and cartilaginous and bony fish as well as various extinct related groups. The method can be carried out with sperm from domesticated animals, and livestock sperm of wild animals. Sperm samples may or may not be cryopreserved. A kind of fish may be the subject described in the present invention donating sperm.

In the following, the term "progeny" will refer to mammalian, bird and fish offspring.

A sperm sample is processed before applying the assay(s) of the present invention as defined under the section "semen processing" of the examples. In brief, the fresh semen sample is incubated for 30 minutes at 37°C and an aliquot is prepared to reach $1 \times 10^6$ cells/ml. The seminal plasma is removed and the aliquot is resuspended in human tubal fluid buffer. A deep-frozen sperm sample can be defrosted and prepared similarly.

By performing the method of the present invention, the spermatozoa are stratified and/or determined, meaning in other words, subdivided or classified into subgroups, cell populations or categories. As used herein, the term "stratifying spermatozoa" and/or "determining spermatozoa" refers to a spermatozoa sample, which is subdivided based on the presence or absence of a specific characteristic (unless context dictates otherwise). In this context, "stratified spermatozoa" and/or "determining spermatozoa" is a sample obtained by subjecting a source sperm sample/ spermatozoa to a selection for specific characteristics. It is therefore in a certain aspect enriched relative to the source sperm sample comprising spermatozoa, to obtain sperm/ spermatozoa having these specific characteristic(s). In the context of the present invention, these characteristics are an intact/ non-intact membrane and intact/ non-intact DNA, also referred to as fragmented/ non-fragmented DNA, which can be differentiated after assaying the sperm sample/ spermatozoa according to the method of the present invention.

A semen sample prepared in the above mentioned way is assayed according to the present invention if it is processed by a protocol distinguishing membrane intact from membrane non-intact spermatozoa (e.g., live and dead cells) with a dye as described herein, combined with a protocol for DNA fragmentation with a dye as described herein.

The term "membrane integrity" refers to the quality or state of the complete cell membrane, including viable cells with intact membrane (e.g., high membrane integrity) and all degrees of destroyed cell membranes (e.g., low or no membrane integrity). In the context of the present invention, the membrane integrity is determined by a staining, binding to cell membrane proteins (amines) on the cell surface, thereby differentiating between live and dead cells as described somewhere else herein. Other words used to describe the membrane integrity are "intact" membrane, not damaged membrane, not defective and living cell. As opposite terms are used "no membrane integrity", non-intact membrane, damaged membrane, defect membrane and a dead or necrotic cell, which are used interchangeably.

It is envisaged by the method of the present invention that spermatozoa, which are alive, display an intact membrane and have no DNA fragmentation, while necrotic cells are marked by their damaged cell membrane and/or fragmented DNA.

The term "fragmented DNA" refers to nuclear DNA with double or single strand breaks. In the context of the present invention, the DNA fragmentation is determined by a staining, binding covalently to the DNA and emitting light of different wavelength, when bound to single or double stranded DNA as described somewhere else herein. The same goal could also be established by using two different dyes marking single and double stranded DNA respectively, which are envisaged as an embodiment of the method of the present invention. Further, terms used to describe fragmented DNA may be destroyed DNA, damaged DNA, DNA fragmentation, non-intact DNA, single stranded DNA and defective DNA, which are used interchangeably.

Both parameters, the membrane integrity and the DNA fragmentation, are analyzed by a staining with at least one dye respectively.

Thus, the present invention relates in a first aspect to a method for stratifying spermatozoa in a sample obtained from a subject, said method comprising (a) assaying spermatozoa comprised by said sample for membrane integrity; and (b) assaying spermatozoa comprised by said sample for DNA fragmentation, and further the stratification allows a prediction whether said subject will have spermatozoa, which may be functional. Preferably, assay (a) and assay (b) are performed simultaneously or subsequently irrespective of the order, whereas assay (a) is preferably performed prior to assay (b).

Assaying a sperm sample comprising spermatozoa for membrane integrity and DNA fragmentation comprises in another embodiment staining the spermatozoa for membrane integrity and DNA fragmentation with at least two dyes, which are preferably fluorescent dyes.

## Stainings

Preferably, the membrane integrity is assayed by a dye differentiating between live and dead cells. It is envisaged that the dye differentiating live and dead cells may be reactive with amines. Further, it is envisaged that the dye differentiating live and dead cells may be reactive with amines, and is preferably a fluorescent dye. A dye marking the membrane integrity, which is preferably used in the context of the present invention, is the LIVE/DEAD Fixable Dead Cell Stain Kit. This kit is based on the reaction of a fluorescent reactive dye with cellular proteins (amines). These dyes cannot penetrate live cell membranes, so only cell surface proteins are available to react with the dye, resulting in dim staining. The reactive dye can permeate the damaged membranes of dead cells and stain both the interior and exterior amines, resulting in a more intense staining. The difference in the fluorescence intensity between live and dead cell populations is typically greater than 50-fold, thereby allowing complete, simultaneous discrimination between the two cell populations. In particular, said fluorescent dye for differentiating live and dead cells, which may be reactive with amines, may preferably be LIVE/DEAD Fixable Blue Stain. Since the reaction between LIVE/DEAD Fixable Blue Stain and the protein amines is covalent, the samples have the potential to be fixed, stored, and evaluated later at convenience. Further non-limiting examples of amine binding dyes are methoxycoumarin, dansyl, pyrene, Alexa Fluor 350, aminomethylcoumarin (AMCA), Marina Blue dye, dapoxyl dye, dialkylaminocoumarin, bimane, hydroxycoumarin, Cascade Blue dye, Pacific Orange dye, Alexa Fluor 405, Zombie Aqua™ Fixable Viability Kit, Zombie Green™ Fixable Viability Kit, Zombie NIR™ Fixable Viability Kit, Zombie Red™ Fixable Viability Kit, Zombie UV™ Fixable Viability Kit, Zombie Violet™ Fixable Viability Kit, Zombie Yellow™ Fixable Viability Kit, LIVE/DEAD™ Fixable Blue Dead Cell Stain Kit, LIVE/DEAD™ Fixable Aqua Dead Cell Stain Kit, LIVE/DEAD™ Fixable Yellow Dead Cell Stain Kit, LIVE/DEAD™ Fixable Green Dead Cell Stain Kit, LIVE/DEAD™ Fixable Red Dead Cell Stain Kit, LIVE/DEAD™ Fixable Far Red Dead Cell Stain Kit, LIVE/DEAD™ Fixable Near-IR Dead Cell Stain Kit, and LIVE/DEAD™ Fixable Violet Dead Cell Stain Kit. All the mentioned dyes may be used in the method of the present invention in further embodiments. Like the LIVE/DEAD, there are many other probes for the evaluation of membrane integrity. Most of them are large molecules that only enter the cell if the membrane is permeable, some of these molecules have also the capacity of reacting covalently to protein amines, providing the possibility of fixing the stain for further analysis. Thus, the use of LIVE/DEAD or any other specific vitality dye is not a limiting factor. The inventors selected the LIVE/DEAD blue, however, sperm stratification can be obtained by using other probes. Another potential compound with the same properties is the amine-reactive dye aminomethyl-coumarin (AMCA). AMCA is a fluorescent compound with an excitation peak at 346 nm and an emission peak at 434 nm, giving it a large Stokes shift of 88 nm. It can be excited using a 355 nm laser paired with a 450/50 nm bandpass filter. These and other alternatives can be used in the methods of the present invention as described herein.

It is envisaged that the DNA damage or fragmentation may be assayed by the method of the present invention by using a DNA intercalating dye, which may be applied for the evaluation of single and double stranded DNA. In the assay for the detection of damaged spermatozoa, DNA stained spermatozoa are analyzed. In a further embodiment of the method of the present invention, the DNA intercalating dye applied for the DNA fragmentation evaluation may be a fluorescent dye. Said DNA intercalating dye applied for the DNA fragmentation evaluation may be a fluorescent dye, and may be preferably acridine orange (AO). Acridine orange is a cell-permeant nucleic acid binding dye that emits green light when bound to double stranded DNA (dsDNA) and red light when bound to single stranded DNA (ssDNA) or RNA, which allows an assessment of the sperm DNA damage. Cells with dominantly green light emission resemble intact DNA and double stranded, whereas dominance of red light emission indicates the presence of fragmented and single stranded DNA.

Both, a staining protocol with a dye labelling single and double stranded DNA (e.g., acridine orange for the evaluation of DNA fragmentation) and a dye labelling the membrane integrity (e.g., LIVE/DEAD) are combined for the present invention and used in the method for spermatozoa stratification and/or determination. To allow the combination of the LIVE/DEAD assay with a further protocol, the inventors centrifuged and re-suspended the sperm samples in TNE Buffer (10 mM Tris-HCl, 150 mM NaCl, 1 mM EDTA) after following the LIVE/DEAD manual. This treatment, as well as the covalent binding of LIVE/DEAD staining, and the order of protocols, enable a combination of LIVE/DEAD with an acridine orange staining for the method of the present invention. Both dyes are used in a concerted manner on one sample, and analyzed in combination according to the emitted light of the labelling dyes.

Further stainings or staining mixtures may be formed by using one or more dye(s), which is/ are excitable through UV or visible light and selective for DNA. Exemplary UV light excitable dyes selective for DNA include Hoechst 33342 and Hoechst 33258, each of which is commercially available from Sigma-Aldrich (St. Louis, MO). Exemplary visible light excitable dyes include SYBR-14, which is commercially available from Molecular Probes, Inc. (Eugene, OR) and bisbensimide-BODIPY conjugate ("BBC") described in WO 02/41906. Each of these dyes may be used alone or in combination; alternatively, other cell-permeant UV and visible light excitable dyes may be used, alone or in combination with the aforementioned dyes, provided the dye does not detrimentally affect the sperm cells to an unacceptable degree when used in concentrations, which enable cell sorting as described elsewhere. However, it is preferred that one dye or two dyes selective for DNA separates between double and single stranded DNA and further that one dye or two dyes discriminate live and dead cells.

Alternatively, the staining mixture may be formed by using fluorescent polyamides, and more specifically polyamides with a fluorescent label or reporter conjugated thereto. Polyamides (PA) are distamycin-type ligands of DNA that bind the minor groove and are capable of sequence selective recognition. These polyamides emit light when hybridized to a nucleic acid containing a target or complementary sequence, but are in a non-hybridized state non-fluorescent (C.M. Dupureur *et al.,* 2012). Such polyamide label could in one scenario of the present invention be combined with another polyamide binding to double stranded DNA. Such a combination would enable the separation of single and double stranded DNA, and could as such be desirable in the context of the sperm stratification method of the present invention. Likewise, fluorescent sequences may also be used to label the sperm single stranded DNA in combination with a dye labelling double stranded DNA. Thus, fluorescent polyamides may be used alone or in combination with further fluorescent polyamides or dyes to labelled single and double stranded DNA. For the membrane integrity analyzes, however, another dye would be needed.

It is encompassed in the method of the present invention that the dye for assaying DNA fragmentation and the dye for assessing membrane integrity may have a different emission spectrum, thereby avoiding a spectral overlap. The labelling dyes, suitable for the method of the present invention, should preferably be compatible with flow cytometry analysis.

Flow cytometry

Generally, once the spermatozoa are assayed or co-stained according to the present invention, they may be analysed according to any known means that allows for distinction based upon a labelling dye, which is preferably a fluorescent dye. In particular, it is envisaged by the method of the present invention that flow cytometry may be used for characterizing the assayed spermatozoa for membrane integrity and/or assayed for sperm DNA fragmentation. Flow cytometry is a well known method in the art to distinguish mixed populations of cells based on the emitted light of differential staining with fluorescent or metal-bound dyes or binding to fluorescently labelled molecules, such as antibodies or nucleic acids. Flow cytometry enables the classification of cells into different cell populations based on the intensity of fluorescence radiation. Classification has been enhanced through the use of flow cytometers high speed, such as the MoFlo flow cytometer produced by Cytomation, Inc. (Ft. Collins, CO) and described in U.S. Pat No. 5,150,313; 5,602,039; 5,602,349 and 5,643,796 and in PCT No. WO 96/12171. Commonly used and well known methods include flow cytometry systems, as exemplified by and described in U.S. Patent Nos. 5,135,759, 5,985,216, 6,071,689, 6,149,867, and 6,263,745, International Patent Publications WO 99/33956 and WO 01/37655, and U.S. Patent Application Serial No. 10/812,351, and corresponding International Patent Publication WO 2004/088283.

Preferably, the cytometer used for the method of the present invention is equipped with the following Lasers; BP Filters: NUV 375 nm, 450/45 (for LIVE/DEAD) Blue 488 nm, 525/40 (green) and 690/50 (red). Even more preferably, the cytometer characteristics comprise Lasers BP Filters: 320-380 nm, 400-530 nm (for LIVE/DEAD), 515-575 nm (for acridine orange green) and 600-740 nm (for acridine orange red).

In the examples of the present invention, a flow cytometric analysis was conducted by using a high-resolution flow cytometer, CytoFLEX S (Beckman Coulter, Krefeld, Germany), equipped with four diode lasers, emitting at 375, 405, 488, and 638 nm; and 15 optical filters. The LIVE/DEAD dye was exited using a near UV laser 375 nm and its corresponding emitted fluorescence was detected with a 450/45 BP filter. For acridine orange the dye was exited with a 488 nm laser and two different filters 525/40 BP and 690/50 BP were used to detect green and red fluorescence respectively. FSC-A vs. SSC-A and 690/50 vs. 525/40 (dot-plots); 450/45, 690/50, 525/40 (histograms) were displayed and used to discriminate cells from cell-like particles. Either a standard sample or calibration beads were used to set the red and green photomultiplier tube (PMT) voltage gain. The mean red and green fluorescence values were set at about ~1300/10000 and about ~5000/10000 channels, respectively. For the LIVE/DEAD fluorescence (blue) the PMT were set at ~250/10000 channels.

Using calibration beads or a calibration or control sperm sample of a fertile donor, a histogram is initially plotted for calibration prior to the measurement of any unknown sample. Beads are small particles and so to say "cell dummies" of well-defined fluorescent intensity and sizes, which also can be used for PMT voltage optimization, compensation setup, cell counting, scale calibration and so on (Cossarizza et al., 2017). The beads may serve the purpose of the standard or calibration sample for fluorescence compensation and gating. The beads may comprise, e.g., the following characteristics: Active amine coated; 5 μm, displaying (448 nm), BODIPY TR (617 nm), and Oregon Green 488 (524 nm) fluorescence. The preparation of the beads may comprise: Mix the beads with 60 μl of staining media containing AMCA (448 nm), BODIPY TR (617 nm), and Oregon Green 488 (524), keep the beads protected from light and incubate for 30 min at 25 °C. After incubation, fix with 100 μl of 1 % PFA, 30 min. A "fertile donor" as referred to in the present invention means a subject, which has mostly functional spermatozoa and is capable of inducing a pregnancy and producing progeny/ children of average health status. Such a fertile donor may produce sperm having at least about 85 % spermatozoa with intact DNA and at least about 60 % living spermatozoa. Therefore, the sperm sample of a fertile donor may comprise fertile spermatozoa, which may be used as a calibration or control sample as described herein. Spermatozoa of a calibration sample are interchangeably termed spermatozoa of a control sample.

As described before, the method of the present invention for stratifying spermatozoa comprises assaying spermatozoa for their DNA fragmentation and membrane integrity by using at least two dyes and further comprises that these cells may be analyzed by flow cytometry or FACS sorting. Meaning, the sperm sample is prepared and diluted as mentioned, assayed for DNA fragmentation and membrane integrity, sorted, and the forward scatter (FSC or FSC-A) and the site scatter (SSC or SSC-A) signal is used to discriminate or gate between cells and cell-like particles. This is done by drawing a region on the plot called gate. Gates discriminate cell-like particles and cells and define, which cells are of further interest based on their position in the plot.

Gating processes may run automated or half automated by FACS operating programs. Many softwares share an "auto-compensation" feature. While these can be very powerful, they are based on automated gating algorithms in which the software identifies the positive and negative populations. Thereby signal maxima, for example of the fluorescence intensity can be read and gates can be placed automatically around cells of similar signal amplitude. Several semi-automated methods of fluorescence intensity registration are available (e.g., fdaNorm and gaussNorm). These methods attempt to move the actual data-points across samples to similar regions, thus allowing gates to be applied to all samples without adjustment. Both fdaNorm and gaussNorm register one channel at a time, and do not address multidimensional linkages between biological sub-populations. The herein described gating processes can however be performed via gating algorithms or manually as described herein.

The gated spermatozoa are then analyzed according to their fluorescence by displaying a histogram plotting the fluorescence signal for the DNA fragmentation against the cell count and the fluorescence signal for the membrane integrity against the cell count. In a preferred embodiment of the present invention, the DNA fragmentation is the green and red fluorescence signal of acridine orange, plotted against the spermatozoa cell count and the membrane integrity is a blue fluorescence signal for the LIVE/DEAD staining, plotted against the cell count. The information of these two histograms is combined in a bivariate logarithmic density plot. In a two parameter density plot, the two axes represent two detectors and cells/ spermatozoa are presented by dots. An example with final quadrant gates shows how four principal cell/ spermatozoa populations can be separated: quadrant Q1 and Q4 are cells strongly expressing one and only one color (single positives), Q2 - dissects cells with both red and blue positivity (double positives) and Q3 - are cells negative for both colors. Said bivariate logarithmic density plot of the present invention is displaying the fluorescence intensity of each spermatozoon for the acridine orange staining and the LIVE/DEAD staining. The scale unit of the combined bivalent logarithmic density plot is the fluorescence intensity, which has no dimension.

Because of the relative nature of the fluorescence intensity of the X- and Y-axis of the combined density plot, said values are described herein as percentage of fluorescence intensity. A percentage of the fluorescence intensity when used herein means the maximum fluorescence intensity measured for the same sample as 100%. The percentage of the fluorescence intensity is used to refer to certain cell populations, e.g. of spermatozoa stratified or determined by the method of the present invention. The fluorescence intensity of the membrane integrity refers to the Y-axis of the combined density plot and covers all measurable or detectable values measured for one sample. Meaning the lowest fluorescence value is assumed to be 0.1 % of the fluorescence intensity of the membrane integrity, while the highest value is taken as 100 % of the fluorescence intensity, representing the membrane integrity. Likewise, the fluorescence intensity of the DNA fragmentation (usually depicted on the X-axis of the combined density plot) covers the measured fluorescence values from lowest to highest value in percent. In a preferred embodiment of the present invention, the spermatozoa are stratified or determined by their fluorescence intensity representing the membrane integrity and DNA fragmentation.

The method further includes determining the number of living spermatozoa showing membrane integrity, dead spermatozoa showing no membrane integrity and the number of living and dead spermatozoa with or without DNA fragmentation. Hence, determining the number of living and dead spermatozoa with or without DNA fragmentation is possible by using the method for stratifying or determining spermatozoa of the present invention as described somewhere else herein. In view of the method of the present invention, it is especially encompassed that said method for stratifying or determining of spermatozoa preferably comprises 1) assaying spermatozoa for membrane integrity (a) and DNA fragmentation (b); 2) sorting the spermatozoa according to the dye used for (a) and (b); 3) selecting the majority of spermatozoa according to their FSC-A and SSC-A signal; 4) analyzing the fluorescence values in a histogram representing (a) for individual spermatozoa; 5) analyzing the fluorescence values in a histogram representing (b) for individual spermatozoa; 5)i) calculating the %DFI from the fluorescence values representing (b) for individual spermatozoa obtained in the previous step; 6) combining the fluorescence values of (a) and (b) obtained from analyzing the fluorescence values representing (a) and (b) respectively per sample in a bivariate logarithmic density plot of the fluorescence intensity; and 6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds, thereby stratifying or determining the spermatozoa number having a non-intact membrane and having fragmented DNA, determining the number of spermatozoa having an intact membrane and having non-fragmented DNA, and determining the number of spermatozoa having an intact membrane and having fragmented DNA. It is encompassed that calculating the percentage of the DNA fragmentation index (%DFI) per sample, may be established by determining the number of spermatozoa with moderate and high DNA fragmentation in relation to the number of spermatozoa with low DNA fragmentation from the fluorescence values representing (b) of individual spermatozoa of said sample.

## DNA fragmentation index (DFI)

The acridine orange DNA staining creates for each individual spermatozoon a unique spectral pattern of green and red fluorescence **(Fig. 13A).** The DNA fragmentation of individual cells can be calculated (GREEN [Channel] / (RED [Channel] + GREEN [Channel]) based on the increased ratio of red to red/green fluorescence. Following the obtained values of acridine orange fluorescence (green and red), a calculated value resulting from the acridine orange stained spermatozoa, separating single and double stranded DNA, is termed the "DNA fragmentation index" or "DFI". This DNA fragmentation index represents the readout of individual spermatozoa and has no dimension.

Using calibration beads or a calibration or control sperm sample of a fertile donor, a histogram is initially measured and analyzed for calibration prior to measurement of any unknown test sample comprising spermatozoa. A reference gate or line is drawn to separate between a population of green fluorescent beads/ spermatozoa (85 % or more of the entire population) in the control sample versus a yellow to red population assumed to represent the damaged beads/ spermatozoa (15 % or less).

The histogram representing the DNA fragmentation plotted against the cell count (e.g., green to red fluorescence of acridine orange staining, which provides for the X-axis of the combined density dot plot), can be used to calculate the percentage of DNA fragmentation index (%DFI) of a measured sample. The %DFI is calculated as the proportion of spermatozoa with high and moderate DNA fragmentation displayed on the right of the histogram **(Fig. 13)** and is the readout of the degree of DNA damage in the entire sample. In other words the %DFI is an expression of % of sperm with fragmented DNA (Everson *et al.,* 2016). In said histogram of the DNA fragmentation (e.g., green to red fluorescence signal) of individual spermatozoon is displayed, and the DNA fragmentation of the complete sample can be assessed on the X-axis ranging from low to high with no dimension **(Fig. 13B).** The percentage of DNA Fragmentation Index (%DFI) is calculated as the ratio of the number of spermatozoa with fragmented DNA to the number of all analyzed spermatozoa.

In relation to the number of spermatozoa of the analyzed sample, a portion of spermatozoa with moderate and high DFI or DNA fragmentation can be calculated. The definition of "moderate and high DFI" or "moderate and high DNA fragmentation" relates to a threshold established by using a calibration or control sample comprising spermatozoa or calibration beads. When using spermatozoa of a calibration sample comprising spermatozoa, said sample is measured first under the presumption that at least 85 % of spermatozoa have non-fragmented DNA and at least 60 % of the spermatozoa are living spermatozoa. According to this presumption, the spermatozoa of a calibration sample are used to establish the threshold, which is further used to analyze the measured test sample(s) in the following. This threshold is usually placed at about 30 units on the X-axis and relates thus to the fluorescence intensity of an analyzed marker. When using acridine orange, it relates to the fluorescence intensity of the red channel representing single stranded DNA or DNA fragmentation. It is however envisaged by the method of the present invention that said threshold can be placed by about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50 or more units. After the threshold is established, spermatozoa on the right of the threshold are considered as having a moderate to high DNA fragmentation index. For instance, in **Fig. 13B,** 8.49 % of the measured spermatozoa showed a higher DFI than the rest of the sample.

Clinical data revealed that another threshold can be defined for the %DFI, at which a clinically relevant decrease of fertility potential was observed and can be predicted (Evenson *et* al., 1999). Men producing semen samples with a DFI over 30 % could not achieve a pregnancy after 12 months. In contrast, a 15 % - 30 % DFI allowed initiation of pregnancies after 4-12 months. No delay was observed when a less than 15 % DFI was present. The DNA fragmentation index can however reach much higher percentages in samples with poor DNA quality. For the method of the present invention, the information of a DNA fragmentation index of spermatozoa comprised in a sample is combined with the information of living and dead cells in a two-parameter density plot of logarithmic scale.

Thus, in the methof for stratifying spermatozoa of the present invention, the spermatozoa are stratified as membrane non-intact spermatozoa, having a DNA fragmentation index (%DFI) portion of at most about 5 %, membrane non-intact spermatozoa, having a DNA fragmentation index (%DFI) portion of at least about 1 %, membrane intact spermatozoa, having a DNA fragmentation index (%DFI) portion of at most about 5 %, and membrane intact spermatozoa, having a DNA fragmentation index (%DFI) portion of at least about 1 %, and the percentage of DNA fragmentation index (% DFI) of the sample lays between about 4 %DFI to about 75 %DFI.

It is envisaged that a functional sperm sample may have a %DFI between about 4 %DFI to about 30 %DFI. In the light of the present invention, a non-functional sperm sample may have a %DFI between about 30 %DFI to about 75 %DFI. Thus, the %DFI is defined as follows: ≤ 15 % DFI = excellent to good sperm DNA integrity, > 15 to < 25 % DFI = good to fair sperm DNA integrity, > 25 to < 50 % DFI = fair to poor sperm DNA integrity, ≥ 50 % DFI = very poor sperm DNA integrity. The above mentioned values relate to natural and intrauterine insemination conceptions. The statistically significant DFI threshold for subfertility has been established at > 25 %. When used herein, the DNA integrity describes how well-preserved the DNA is, and thus the opposite to the DNA fragmentation. The DNA integrity describes a DNA in its natural physiological double helix state, without extensive double strand breaks, damaged base pairs or further non-physiological damages, which would interfere with the biological function of the DNA.

The percentage of DNA fragmentation index (%-DFI=((number of spermatozoa with fragmented DNA)/(number of spermatozoa with fragmented DNA+number of spermatozoa without fragmented DNA)x100%)) provided by the sole use of a DNA staining, does not distinguish between membrane intact and membrane defective spermatozoa, while only membrane intact spermatozoa are useable for conception or in *assisted reproductive technologies.* Accordingly, the DFI alone likely false estimates the risk of using a DNA damaged spermatozoa during such a reproductive treatment. A sperm sample is likely to contain spermatozoa with intact membrane and with or without DNA damage, and spermatozoa with defective membrane and with or without DNA damage. Importantly, the method of the present invention allows differentiating between spermatozoa with DNA damage having additional membrane damage. This means that the % DFI of a sample is mainly distributed between the group of spermatozoa having a non-intact membrane and having fragmented DNA, and spermatozoa having an intact membrane and having fragmented DNA. Said distribution of the % DFI between the spermatozoa groups having fragmented DNA and intact membrane/ non-intact membrane may not be in equal numbers (e.g., 5 % and 15 % instead of 10 % (ii), 10 % (iv)). Any distribution between the spermatozoa groups having fragmented DNA and intact membrane/ non-intact membrane is encompassed by the present invention.

In the context of the present invention, it is envisaged that "DNA fragmentation (%DFI) portion of at least about 1 %" may display a %DFI portion of about 1 %, about 2 %, about 3 %, about 4 %, about 5 %, about 6 %, about 7 %, about 8 %, about 9 %, about 10 %, about 11 %, about 12 %, about 13 %, about 14 %, about 15 %, about 16 %, about 17 %, about 18 %, about 19 %, about 20 %, about 21 %, about 22 %, about 23 %, about 24 %, about 25 %, about 26 %, about 27 %, about 28 %, about 29 %, about 30 %, about 31 %, about 32 %, about 33 %, about 34 %, about 35 %, about 36 %, about 37%, about 38 %, about 39 %, about 40 %, about 41 %, about 42 %, about 43 %, about 44 %, about 45 %, about 46 %, about 47 %, about 48 %, about 49 %, about 50 %, about 51 %, about 52 %, about 53 %, about 54 %, about 55 %, about 56 %, about 57 %, about 58 %, about 59 %, about 60 %, about 61 %, about 62 %, about 63 %, about 64 %, about 65%, about 66 %, about 67 %, about 68 %, about 69 %, about 70 %, about 71 %, about 72 %, about 73 %, about 74 %, about 75 % or more.

Spermatozoa stratified as membrane non-intact cells with intact DNA and functional spermatozoa should generally not display a DNA fragmentation, because they were selected for their fluorescence signal showing an intact DNA. However, it can not be excluded that spermatozoa stratified as (i) and (iii) may contain a very low percentage of cells, which could be considered as having some degree of natural DNA fragmentation, probably not interfering with the biological function of the DNA. In the context of the present invention, "DNA fragmentation (%DFI) portion of at most about 5 %" includes a %DFI portion of less than 1 %, about 1 %, about 2 %, about 3 %, about 4 %, and about 5 %, respectively. Spermatozoa defined in (i) are membrane non-intact spermatozoa and should preferably have a DNA fragmentation (% DFI) portion of less than about 1 % of the total %DFI of a sample. Spermatozoa defined in (iii) are membrane intact spermatozoa and should preferably have a DNA fragmentation (%DFI) portion of less than about 1 % of the total %DFI of a sample.

As mentioned before, a %DFI of about or less than about > 15 to < 25 % DFI is considered as a good to fair sperm DNA integrity, meaning about 85 % to 75 % of the spermatozoa are having no measurable DNA fragmentation. To include sperm samples of less fertile donors or donors with clinical symptomatic spermatozoa, it is encompassed by the present invention that assayed spermatozoa are having a percentage of DNA fragmentation index of at least about 4 % up to about 75 % or more of the total sperm sample.

**Flow cytometry data**

Calibration of the flow cytometer or FACS means that after sorting and selecting the spermatozoa according to their FSC-A versus SSC-A properties, spermatozoa comprised within a calibration or control sperm sample may be used to set the photomultiplier tube (PMT) voltage gain to yield comparable mean fluorescence level and DNA fragmentation values in each round of measurements. For acridine orange, these fluorescence levels may be red and green, however, other color channel may be suitable as well depending on the used dye. The mean red and green fluorescence values can be set at ~1300/10000 and ~5000/10000 channels, respectively. The same can be done for the LIVE/DEAD fluorescence of live spermatozoa (blue) at ~2500/10000 channels. In a similar way, calibration beads can be used instead of spermatozoa of a calibration sample. The green versus red fluorescence values of the acridine orange (AO) staining for each spermatozoon is displayed in a density plot and gated for their signal (excluding debris and chromatin abnormalities), creating a cytogram of the DNA fragmentation index, which may be used to determine the percentage of DNA fragmentation index (%DFI) of a sample as mentioned somewhere else herein. This may be established by identifying the spermatozoa with moderate to high DNA fragmentation.

Another histogram can be created, displaying the membrane integrity, plotted against the cell count, as mentioned somewhere else herein. In general, the subpopulation to the left of the X-axis of this histogram represents spermatozoa with intact membrane (e.g., living spermatozoa) according to their fluorescence signal, and the one to the right of the X-axis of the histogram represents membrane non-intact (e.g., dead) spermatozoa according to their fluorescence signal.

Finally, the values of the blue fluorescence channel for the membrane integrity (e.g., LIVE/DEAD) may be combined with the green to red fluorescence channel representing the spermatozoa with and without DNA fragmentation (e.g., acridine orange) in a combined density dot plot. A calibration sperm sample comprising spermatozoa is also envisaged to be used for establishing a fluorescence level threshold in the combined bivalent logarithmic density plot.

Spermatozoa of a calibration sample used for the calibration of the flow cytometer or FACS analysis refers to a sperm sample with about 85 % or more spermatozoa of the sperm sample having no DNA damage or fragmentation and at least about 60 % of the spermatozoa are living spermatozoa. Such a sperm sample may comprise about 85 %, about 90 %, about 95 % or more spermatozoa having no DNA damage or fragmentation, and at least about 60%, about 65 %, about 70%, about 75%, about 80 % or more spermatozoa are measured as living spermatozoa (e.g., having an intact membrane). Thus, it is encompassed that the individual fluorescence values representing assay (a) and assay (b) for individual spermatozoa, may be combined in a bivariate logarithmic density plot of the fluorescence intensity of (a) and (b) respectively, and that a fluorescence level threshold may be established for membrane intact and membrane non-intact spermatozoa and for spermatozoa with and without DNA fragmentation by using spermatozoa of a calibration sample or calibration beads.

A fluorescence level threshold, differentiating membrane intact (low LIVE/DEAD fluorescence) and membrane defective (high LIVE/DEAD fluorescence) spermatozoa of the combined density plot, and a fluorescence level threshold differentiating spermatozoa with non-fragmented DNA (acridine orange, green fluorescence (dsDNA)) and spermatozoa with fragmented DNA (acridine orange, red fluorescence (ssDNA or RNA)) of the combined density plot can be set as mentioned above by using a calibration or control sperm sample or calibration beads. Said threshold(s) define four regions of the combined density plot, which are referred to as quadrants. Said "quadrant gates" or "quadrants" (Q1 to Q4) display spermatozoa grouped, because of similar characteristics or fluorescence signal.

In one embodiment of the present invention, the quadrants may be: quadrant 1 comprising spermatozoa with non-intact membrane and no to almost no DNA fragmentation, quadrant 2 comprising spermatozoa with non-intact membrane and with DNA fragmentation, quadrant 3 comprising spermatozoa with intact membrane and no to almost no DNA fragmentation, and quadrant 4 comprising spermatozoa with intact membrane and with DNA fragmentation. The combined density plot can then be gated according to said threshold(s) for cell portions or cell numbers herein referred to as spermatozoa numbers, which allow the calculation of the cell count or percentages of spermatozoa, which have been stratified or determined according to the method of the present invention.

Thus, in view of the method of the present invention, the spermatozoa thereby may be stratified or determined as follows: I. membrane non-intact spermatozoa, having non-fragmented DNA, with a spermatozoa number between about 20 % and about 60 % of the total spermatozoa number, preferably about 35 % of the total spermatozoa number, measured per sample; II. membrane non-intact spermatozoa, having fragmented DNA, with a spermatozoa number of at least about 1 % of the total spermatozoa number measured per sample; III. membrane intact spermatozoa, having non-fragmented DNA, with a spermatozoa number between about 45 % and about 75 % of the total spermatozoa number, preferably about 55 % of the total spermatozoa number measured per sample; and IV. membrane intact spermatozoa, having fragmented DNA, with a spermatozoa number of at least about 1 % of the total spermatozoa number measured per sample. Spermatozoa having a non-intact membrane and having a DNA fragmentation, may be a percentage of spermatozoa between about 20 % and about 60%, preferably about 35 % of spermatozoa in said sample. Thus, spermatozoa having a non-intact membrane and having a DNA fragmentation, may be about 20 %, about 21 %, about 22 %, about 23 %, about 24 %, about 25 %, about 26 %, about 27 %, about 28 %, about 29 %, about 30 %, about 31 %, about 32 %, about 33 %, about 34 %, about 35 %, about 36 %, about 37%, about 38 %, about 39 %, about 40 %, about 41 %, about 42 %, about 43 %, about 44 %, about 45 %, about 46 %, about 47 %, about 48 %, about 49 %, about 50 %, about 51 %, about 52 %, about 53 %, about 54 %, about 55 %, about 56 %, about 57 %, about 58 %, about 59 %, about 60 % or more spermatozoa, in relation to the total spermatozoa cell count (100 %) per sample.

Spermatozoa having a non-intact membrane and having a DNA fragmentation may be up to at least about 1 % of the spermatozoa in said sample. Thus, spermatozoa having a non-intact membrane and having a DNA fragmentation may be about 1 %, about 2 %, about 3 %, about 4%, about 5 %, about 6 %, about 7 %, about 8 %, about 9 %, about 10 %, about 11 %, about 12 %, about 13 %, about 14 %, about 15 %, about 16 %, about 17 %, about 18 %, about 19 %, about 20 %, about 21 %, about 22 %, about 23 %, about 24 %, about 25 %, about 26 %, about 27 %, about 28 %, about 29 %, about 30 % or more spermatozoa, in relation to the total spermatozoa cell count (100 %) per sample.

Spermatozoa having an intact membrane and having a DNA fragmentation may be between about 45 % and about 75%, preferably about 55 % of the spermatozoa in said sample. Therefore, spermatozoa having an intact membrane and having a DNA fragmentation may be about 45 %, about 46 %, about 47 %, about 48 %, about 49 %, about 50 %, about 51 %, about 52 %, about 53 %, about 54 %, about 55 %, about 56 %, about 57 %, about 58 %, about 59 %, about 60 %, about 61 %, about 62 %, about 63 %, about 64 %, about 65 %, about 66 %, about 67 %, about 68 %, about 69 %, about 70 %, about 71 %, about 72 %, about 73 %, about 74 %, about 75 % or more spermatozoa, in relation to the total spermatozoa cell count (100 %) per sample.

Spermatozoa having an intact membrane and having a DNA fragmentation may be at least about 1 % of the spermatozoa in said sample. It is envisaged that spermatozoa having a non-intact membrane and having a DNA fragmentation may be about 1 %, about 2 %, about 3 %, about 4 %, about 5 %, about 6 %, about 7 %, about 8 %, about 9 %, about 10 %, about 11 %, about 12 %, about 13 %, about 14 %, about 15 %, about 16 %, about 17 %, about 18 %, about 19 %, about 20 %, about 21 %, about 22 %, about 23 %, about 24 %, about 25 %, about 26 %, about 27 %, about 28 %,

about 29 %, about 30 % or more spermatozoa, in relation to the total spermatozoa cell count (100 %) measured per sample. The cell count is then used to calculate the DMI ratio as described somewhere else herein. Further, in the present invention, the term "determining the percentage of spermatozoa" refers to the aforementioned percentage of spermatozoa calculated from the cell count. In particular, it is envisaged that the spermatozoa numbers of I to IV may allow the calculation of the DNA and membrane integrity (DMI) ratio, wherein the number of membrane intact DNA fragmented spermatozoa (IV) is set in ratio to the total number of membrane intact spermatozoa (III and IV), thereby indicating the DNA and membrane integrity (DMI) ratio as described somewhere else herein in more detail.

**DNA and Membrane Integrity (DMI) Ratio**

The method of the present invention may provide a *DNA and Membrane Integrity Ratio* (DMI), which makes it possible to assess and categorize DNA fragmentation in membrane intact spermatozoa only. This important difference, compared to the DNA fragmentation index (DFI), represents an increase in the diagnostic power, and the *DNA and membrane integrity* (DMI) ratio represents a more accurate information source. For these calculations, the cell count or number of spermatozoa is determined as described somewhere else herein. It is envisaged that the method of the present invention may determine the number of membrane intact to DNA fragmented spermatozoa, which may be set in ratio to the total number of membrane intact spermatozoa, thereby indicting the DNA and membrane integrity (DMI) ratio:

$$DMI\ ratio = \frac{membrane\ intact\ DNA\ fragmented\ spermatozoa}{total\ membrane\ intact\ spermatozoa}.$$

Meaning that the stratified or determined spermatozoa numbers may allow the calculation of the DNA and membrane integrity (DMI) ratio of the number of membrane intact DNA fragmented spermatozoa (e.g., token IV), which may be set in ratio to the total number of membrane intact spermatozoa (e.g., token III and IV), as described herein.

To calculate the percentage of *DNA and membrane integrity ratio,* the cell count or spermatozoa number in quadrant 4 of the two parameter density plot may be divided by the sum of the cell counts or spermatozoa number of quadrants 3 and 4 of the two parameter density plot and multiplied by 100 (%-DMI = [Number of cells in Q4/(Number of cells in Q3+ Number of cells in Q4)]x100%).

The DMI ratio of a sperm sample can be compared to control sample values. Thereby the DMI ratio gives a more detailed picture of the potential fertility situation of the subject than the DNA fragmentation index can. While the DNA fragmentation index (DFI) assesses the DNA fragmentation of already dead spermatozoa, the DMI ratio gives a clearly resolved picture of the DNA quality of living spermatozoa comprised in a sample. Thus, displaying a reliable clinical tool for the assessment of further steps and processing of the sperm and fertility treatment.

To simulate sperm samples of different qualities, untreated spermatozoa were mixed with known amounts of destroyed spermatozoa. The mixture was analyzed according to the method of the present invention and the DMI was calculated. The results show a clear correlation between increasing DMI values and the amount of destroyed spermatozoa in the total sample **(Fig. 12 B),** with a coefficient of determination of $R^2 = 0.97$. A low DMI value hints towards a spermatozoa sample with more membrane intact and DNA non-fragmented spermatozoa, which are predictably functional and presumably fertile, while a high DMI represents a spermatozoa sample with more membrane non-intact and DNA fragmented spermatozoa, which are predictably non-functional and presumably non-fertile.

**Spermatozoa functionality**

When used in the context of the present invention, a method for determining whether "functional" or "non-functional spermatozoa" are present in a sample refers to stratified spermatozoa, which are used to further predict the functionality of said stratified spermatozoa. Functional spermatozoa are, in the context of the present invention, stratified as membrane intact and having no DNA fragmentation, meaning no membrane and/or DNA damage can be detected according to the method of the present invention. Functional spermatozoa may be potentially fertile spermatozoa. "Potentially fertile" spermatozoa are presumably capable of inducing a pregnancy under standard conditions and can produce progeny/ children of average health status. The present invention relates to a method for stratifying spermatozoa in a sample obtained from a subject and the method comprises (a) assaying spermatozoa comprised by said sample for membrane integrity; and (b) assaying spermatozoa comprised by said sample for DNA fragmentation, wherein the stratification allows a prediction whether said subject will have spermatozoa, which may be functional, wherein the method is as defined in the appended claims.

In contrast, non-functional spermatozoa may display DNA and/or membrane damage, meaning spermatozoa are predicted as "non-functional" in the context of the present invention, if a non-intact membrane and/or DNA fragmentation can be detected by the method described herein. It is well known to the skilled artisan that sperm DNA damage is prevalent among infertile men and it is apparent that spermatozoa with non-intact membrane, which are dead or necrotic are not functional in that they cannot induce a pregnancy without clinical aid. Such non-functional spermatozoa are presumably not in the condition to induce a pregnancy or to produce progeny/ children of average health status. In other words, non-functional spermatozoa can be necrotic and/or have damaged DNA. In view of the method of the present invention, non-functional spermatozoa may be potentially infertile spermatozoa. "Potentially infertile" spermatozoa are presumably not capable of inducing a pregnancy under standard conditions. The described categories (i), (ii) and (iv) comprise either membrane damage, DNA damage or both, meaning that the presence of spermatozoa pursuant to (i), (ii) and/or (iv) may be predictive that said subject will have spermatozoa, which may not be functional. Accordingly, spermatozoa are stratified as follows: (i) membrane non-intact spermatozoa, having a DNA fragmentation index (% DFI) portion of at most about 5 % in said sample, (ii) membrane non-intact spermatozoa, having a DNA fragmentation index (%DFI) portion of at least about 1 % in said sample, (iii) membrane intact spermatozoa, having a DNA fragmentation index (%DFI) portion of at most about 5 % in said sample, (iv) membrane intact spermatozoa, having a DNA fragmentation index (%DFI) portion of at least about 1 % in said sample and wherein the presence of spermatozoa pursuant to (iii) is indicative that said subject will have spermatozoa which may be functional.

In one embodiment of the present invention, spermatozoa are distributed into four groups; membrane non-intact or necrotic spermatozoa having low level of DNA fragmentation, membrane non-intact or necrotic spermatozoa having high level of DNA fragmentation, membrane intact or living spermatozoa having low level of DNA fragmentation, membrane intact or living spermatozoa having high level of DNA fragmentation. In another embodiment, these four spermatozoa groups have the following staining signal: positive signal for LIVE/DEAD and mostly green signal for acridine orange, positive signal for LIVE/DEAD and mostly red signal for acridine orange, negative signal for LIVE/DEAD and mostly green signal for acridine orange and negative signal for LIVE/DEAD and mostly red signal for acridine orange. As mentioned above, three out of these four groups display membrane damage, DNA damage or both, which is taken as indicative for reduced spermatozoa functionality.

Spermatozoa with reduced functionality may be described as having low membrane integrity and having prevalent single stranded DNA or RNA. In another aspect, the method for stratifying spermatozoa comprised in a sample from a subject comprises (a) assaying spermatozoa comprised by said sample for membrane integrity; (b) assaying spermatozoa comprised by said sample for DNA fragmentation; and thereby the spermatozoa are stratified as membrane non-intact spermatozoa with prevalent double stranded DNA, membrane non-intact spermatozoa with prevalent single stranded DNA or RNA, membrane intact spermatozoa with prevalent double stranded DNA, and membrane intact spermatozoa with prevalent single stranded DNA or RNA. It is envisaged that the assay for (a) is preferably an assay staining amines on the cell surface, more preferably LIVE/DEAD, and that the assay for (b) is staining single and double stranded DNA, preferably with one or more dyes, more preferably the staining is acridine orange. For this embodiment, the spermatozoa are separated according to the fluorescent signal of the dyes used for (a) and (b) and thereby, the used assay allows the determination of the following four spermatozoa groups: dead cell (LIVE/DEAD positive) and mostly dsDNA (mostly green acridine orange signal), dead cell (LIVE/DEAD positive) and mostly ssDNA or RNA (mostly red acridine orange signal), living cell (LIVE/DEAD negative) and mostly dsDNA (mostly green acridine orange signal), and living cell (LIVE/DEAD negative) and mostly ssDNA or RNA (mostly red acridine orange signal). It is envisaged that the staining used for the membrane integrity does not bind DNA, to avoid interfering signals of the DNA fragmentation dye. By using the method of the present invention, it may be determined whether dsDNA or ssDNA is prevalent in the separate spermatozoa; however the %DFI may not be calculated. In another embodiment, the dye(s) used to separate single and double stranded DNA may combine labelled nucleic acid probes to mark single stranded DNA with a labelled DNA dye to mark double stranded DNA. The sole use of a DNA intercalating dye (e.g., SYBR14, ethidium bromide, TUNEL) would not resolve single and double stranded DNA. Additionally, the dye analyzing the spermatozoa viability and combined therewith is not intercalating into DNA.

A further aspect of the present invention relates to a method for determining whether non-functional spermatozoa are present in a sample comprising spermatozoa obtained from a subject, wherein the method comprises (a) assaying spermatozoa comprised by said sample for membrane integrity; and (b) assaying spermatozoa comprised by said sample for DNA fragmentation; wherein the presence of membrane non-intact spermatozoa having a DNA fragmentation index of at most about 5 % in said sample is predictive that said subject will have spermatozoa which may be non-functional, wherein the presence of membrane non-intact spermatozoa having a DNA fragmentation index (%DFI) portion of at least about 1 % in said sample is predictive that said subject will have spermatozoa which may not be functional, and/or wherein the presence of membrane intact spermatozoa having a DNA fragmentation index (%DFI) portion of at least about 1 % in said sample is predictive that said subject will have spermatozoa which may not be functional.

In another aspect, the present invention relates to a method for determining whether functional spermatozoa are present in a sample with spermatozoa obtained from a subject, the method comprises (a) assaying spermatozoa comprised by said sample for membrane integrity; and (b) assaying spermatozoa comprised by said sample for DNA fragmentation; wherein the presence of membrane intact spermatozoa having a DNA fragmentation index (%DFI) portion of at most about 5 % in said sample is predictive that said subject will have spermatozoa which may be functional. As mentioned before, spermatozoa with intact membrane and low DNA fragmentation index are predicted to be functional and are presumably potentially fertile meaning they are capable of inducing a pregnancy. It is encompassed by the present invention that these spermatozoa with at most about 5 % DNA fragmentation index are having a DNA fragmentation index between about 0.5 % and about 5 % as defined elsewhere herein.

Hence, a "prediction" whether a subject will have spermatozoa, which may or may not be functional, according to the present invention, is based upon the DNA fragmentation and membrane integrity status of the spermatozoa, measured by the method of the present invention. An assessment of the spermatozoa functionality is made according to these two parameters. Spermatozoa predicted to be functional, are presumably potentially fertile, which means that with the method of the present invention no DNA or membrane damage could be found. A prediction of the spermatozoa functionality and the potential fertility is aiming at the ability of said spermatozoa to induce a pregnancy, preferably under natural conditions, however, a complete analysis of the overall fertility situation of a subject would need to include further parameters like the movement and shape of said spermatozoa. However, in case the spermatozoa of a subject are predicted to contain a majority of non-functional spermatozoa and/or display a high %DFI and/or a high DMI ratio, further viability tests may be advisable.

## Further viability tests

The functional and/or non-functional spermatozoa determined by the method of the present invention may be subject to one or more viability tests. These sperm viability tests may be feasible for sperm consisting of a mixture of functional and/or non-functional spermatozoa as defined herein. It is envisaged that the sperm used for the stratification or determination method of the present invention can be further analyzed. This includes another aliquot of the same semen sample, which was analyzed by the method of the present invention as well as another semen sample from the same e.g. and identical subject. "More viability tests" means, for example, a spermatozoa motility test, an analysis of the acrosome status, morphology and morphometry analysis of the spermatozoa as well as an analysis of the sperm heads, which are known to a person skilled in the art. It further includes the use of a sperm sample for the purposes of research, or for the use in *assisted reproductive technology* (ART) such as *in vitro fertilization* (IVF)*, artificial insemination* (Al), *intracytoplasmic sperm injection* (ICSI, as well as other techniques using enucleated cells), and *multiple ovulation and embryo transfer* (MOET, as well as other embryo transfer techniques).

## Further embodiments

In another embodiment of the present invention, the method for stratifying or determining of spermatozoa may not comprise the determination of the %DFI or DFI of a sample, but the determination of the DNA fragmentation of individual spermatozoa. In this scenario, it is encompassed that said method for stratifying or determining of spermatozoa preferably may comprise 1) assaying spermatozoa for membrane integrity (a) and DNA fragmentation (b); 2) sorting the spermatozoa according to the dye used for (a) and (b); 3) selecting the majority of spermatozoa according to their FSC-A and SSC-A signal; 4) analyzing the fluorescence values in a histogram representing (a) for individual spermatozoa; 5) analyzing the fluorescence values in a histogram representing (b) for individual spermatozoa; 5)i) calculating the %DFI from the fluorescence values representing (b) for individual spermatozoa obtained in the previous step; 6) combining the fluorescence values of (a) and (b) obtained from analyzing the fluorescence values representing (a) and (b), respectively, per sample in a bivariate logarithmic density plot of the fluorescence intensity; and 6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds. It is encompassed that calculating the percentage of the DNA fragmentation index (%DFI) per sample, may be established by determining the number of spermatozoa with moderate and high DNA fragmentation in relation to the number of spermatozoa with low DNA fragmentation from the fluorescence values representing (b) of individual spermatozoa of said sample. It is encompassed that the spermatozoa of said sample may be thereby stratified or determined as follows: (i) membrane non-intact spermatozoa, having a DNA fragmentation index (%DFI) portion of at most about 5 %; (ii) membrane non-intact spermatozoa, having a DNA fragmentation index (% DFI) portion of at least about 1 %; (iii) membrane intact spermatozoa, having a DNA fragmentation index (%DFI) portion of at most about 5 %; (iv) membrane intact spermatozoa, having a DNA fragmentation index (%DFI) portion of at least about 1 %; and the percentage of DNA fragmentation index (%DFI) of said sample is between about 4 %DFI to about 75 %DFI. In features (ii) and (iv), the percentage of %DFI is related to "membran non-intact spermatozoa" or "membran intact spermatozoa", while 4 %DFI - 75 %DFI relates to %DFI of the total sample.

In another aspect, the present invention relates to a method for stratifying or determining spermatozoa, wherein stratifying or determining of spermatozoa comprises 1) assaying spermatozoa for membrane integrity (a) and DNA fragmentation (b); 2) sorting the spermatozoa according to the dye used for (a) and (b); 3) selecting the majority of spermatozoa according to their FSC-A and SSC-A signal; 4) analyzing the fluorescence values in a histogram representing (a) for individual spermatozoa; 5) analyzing the fluorescence values in a histogram representing (b) for individual spermatozoa; 6) combining the fluorescence values of (a) and (b) obtained in the steps 4) and 5) per sample in a bivariate logarithmic density plot of the fluorescence intensity, and 6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds. It is encompassed that the spermatozoa of said sample may thereby be stratified or determined as follows: I. membrane non-intact spermatozoa having non-fragmented DNA, with a spermatozoa number between about 20 % and about 60 % of the total spermatozoa number, preferably about 35 % of the total spermatozoa number measured per sample; II. membrane non-intact spermatozoa having fragmented DNA, with a spermatozoa number of at least about 1 % of the total spermatozoa number measured per sample; III. membrane intact spermatozoa having non-fragmented DNA, with a spermatozoa number between about 45 % and about 75 % of the total spermatozoa number, preferably about 55 % of the total spermatozoa number measured per sample; and IV. membrane intact spermatozoa having fragmented DNA, with a spermatozoa number of at least about 1 % of the total spermatozoa number measured per sample.

It is envisaged that the spermatozoa numbers of I to IV may allow the calculation of the DNA and membrane integrity (DMI) ratio, wherein the number of membrane intact DNA fragmented spermatozoa (IV) is set in ratio to the total number of membrane intact spermatozoa (III and IV), thereby indicating the DNA and membrane integrity (DMI) ratio as described herein.

In a further embodiment of the method of the present invention, a DNA intercalating dye may be applied for the evaluation of DNA fragmentation. Preferably, the DNA intercalating dye applied for the evaluation of DNA fragmentation is a fluorescent dye. Said fluorescent dye of the method of the present invention may be an intercalating dye applied for the evaluation of DNA fragmentation, preferably acridine orange (AO).

Further, it is envisaged that the dye differentiating live and dead cells may be reactive with amines. Said dye for differentiating live and dead cells for the method of the present invention, which may be reactive with amines, may be preferably a fluorescent dye. In particular, said dye for the method of the present invention is preferably a fluorescent dye differentiating live and dead cells, which may be reactive with amines, more preferably a LIVE/DEAD Fixable Dead Cell Stain.

In yet another embodiment of the present invention, the method of stratifying or determining of spermatozoa may not comprise the use of acridine orange and LIVE/DEAD staining, but of substitutional dyes marking single and double stranded DNA with different fluorophores and staining the amines on the cell membrane to mark living and dead cells. In this scenario the method comprises 1) assaying spermatozoa for membrane integrity (a) and DNA fragmentation (b); 2) sorting the spermatozoa according to the dye used for (a) and (b); 3) selecting the majority of spermatozoa according to their FSC-A and SSC-A signal; 4) analyzing the fluorescence values in a histogram representing (a) for individual spermatozoa; 5) analyzing the fluorescence values in a histogram representing (b) for individual spermatozoa; 5)i) calculating the %DFI from the fluorescence values, representing (b) for individual spermatozoa obtained in the previous step; 6) combining the fluorescence values of (a) and (b) obtained from analyzing the fluorescence values representing (a) and (b), respectively per sample in a bivariate logarithmic density plot of the fluorescence intensity; and 6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds. It is encompassed that calculating the percentage of the DNA fragmentation index (%DFI) per sample, may be established by determining the number of spermatozoa with moderate and high DNA fragmentation in relation to the number of spermatozoa with low DNA fragmentation from the fluorescence values representing (b) of individual spermatozoa of said sample. The method may further comprise determining the number of spermatozoa stratified as membrane non-intact spermatozoa with prevalent double stranded DNA; membrane non-intact spermatozoa with prevalent single stranded DNA or RNA; membrane intact spermatozoa with prevalent double stranded DNA; and membrane intact spermatozoa with prevalent single stranded DNA or RNA. It is envisaged that a DNA intercalating dye may be applied for the evaluation of single and double stranded DNA. In particular, it is envisaged that the DNA intercalating dye applied for the evaluation of single and double stranded DNA may be a fluorescent dye. In the method of the present invention, the dye differentiating membrane intact and membrane not-intact spermatozoa may be reactive with amines. It is encompassed that this dye differentiating membrane intact and membrane not-intact spermatozoa, which may be reactive with amines, may be a fluorescent dye. Said dye for the evaluation of single and double stranded DNA and said dye differentiating membrane intact and membrane not-intact spermatozoa, reactive with amines, may have a different emission spectrum, thereby avoiding a spectral overlap.

**Kit** (in the following embodiments of a kit are described which are not part of the invention)

It is further described herein a commercial package or kit for performing the method of the present invention,

which comprises a DNA intercalating dye for the evaluation of DNA fragmentation and a dye differentiating live and dead cells, further comprising calibration beads and (human) tubal fluid (HTF) buffer. The HTF was the first specific medium in 1985 for human IVF and since then is one of the most used in ART labs for the culture of embryos (Quinn et al., in 1985). Said kit may comprise at least a DNA intercalating dye for the evaluation of DNA fragmentation and at least a dye differentiating live and dead cells, at least a suspension of calibration beads, and at least a tubal fluid buffer of mammal, bird or fish, for example a human tubal fluid buffer as reported by Strünker et al. (2012) consisting of (in mM): 97.8 NaCl, 4.7 KCl, 0.2 $MgSO_4$, 0.37 $KH_2PO_4$, 2 $CaCl_2$, 0.33 Na-pyruvate, 21.4 Na-lactic acid, 2.8 glucose and 21 HEPES, adjusted to pH 7.35 with NaOH. This kit may be filled in one or more packages.

The DNA intercalating dye of the kit applied for the evaluation of DNA fragmentation may be a fluorescent dye and may be acridine orange (AO), while the dye differentiating live and dead cells may be reactive with amines and may be for example a fluorescent dye, which may be for example a LIVE/DEAD Fixable Dead Cell Stain. It is further described herein that the kit comprises a DNA intercalating dye applied for the evaluation of DNA fragmentation, which may be for example a fluorescent dye. It is further described herein that the kit comprises a fluorescent DNA intercalating dye applied for the evaluation of DNA fragmentation, which may be acridine orange (AO). Further, it is described herein that the kit comprises a dye differentiating live and dead cells, which may be reactive with amines. It is also described herein that the kit comprises a dye differentiating live and dead cells, which may be reactive with amines and may be a fluorescent dye. It is further described herein that the kit comprises a fluorescent dye differentiating live and dead cells, which may be reactive with amines and may be a LIVE/DEAD Fixable Dead Cell Stain. As mentioned before, the spermatozoa are assayed with LIVE/DEAD and acridine orange fluorescent staining and may be processed by flow cytometry as described herein. Further, the kit may also comprise a manual on how to analyze the sample, may use the calibration beads and how to calculate the DMI ratio.

To ensure the repeatability of the method of the present invention, calibration beads may be part of the kit described herein. These calibration beads may be used to set the photomultiplier tube (PMT) voltage gain. The advantage of beads is that no biological material is required, and they are easy to prepare and use. Following the manufacturer’s protocols, for many fluorochromes, beads provide accurate spillover values (SOVs), which means the amount of signal, measured in median fluorescent intensity, that a fluorochrome emits in a secondary detector specific for a different fluorochrome (Cossarizza et al., 2017).

The kit as described herein may be particularly useful for applications in mammals, fish, bird and humans, according to the evaluation methods described herein. More particularly, the kit may be helpful for laboratory and diagnostic purposes in humans before artificial insemination procedures. The kit as described herein and the methods of the invention may be used in combination with other assays and/or kits.

**Summary**

Herein the inventors provide the use of a new protocol that extends the ability to determine sperm DNA fragmentation of the SCSA with a vital stain, in this case, the LIVE/DEAD™ Fixable Blue Stain, to distinguish different sperm populations based on membrane integrity in a simple and easy-to-perform manner. However, the inclusion of this dye implies an incubation time of 30 min, and the previous removal of seminal plasma to avoid unspecific binding to seminal plasma proteins. In this regard, it was not known whether these differences in the staining protocol could compromise the readout of the DNA fragmentation test or if the nucleus denaturation with a low-pH (1.2) detergent solution and the posterior staining with AO could also affect the assessment sperm membrane integrity.

In the present application, the inventors’ co-staining was therefore tested against the independent-use of two established staining procedures such as PI staining for membrane integrity and conventional SCSA. As seen in **Figure 1A** and **3A,** no significant differences were observed between the methods. Moreover, the method agreement analysis shows comparable results among the tested methods **(Fig. 2).** By this way, the inventors ruled out any cross-interference between the staining protocols of the co-staining, and given the high R square values obtained from theoretical and experimental values **(Fig. 11),** it was proven that the method of the present invention is sensitive and its results are accurate and reliable.

At the moment, all other methods available for assessing sperm DNA integrity have limited diagnostic and prognostic power. In a meta-analysis, performed by Collins et al., the predictive value of sperm DNA integrity tests was evaluated for ART pregnancy in 22 studies and 2162 cycles. The results showed low specificity for all evaluated assays. Similar results were reported in a more recent study. This discrepancy lays mainly in the fact that most of the tests to evaluate sperm DNA integrity do not consider the membrane integrity of the studied sperm population, when only the spermatozoa with intact membrane are being willingly subsequently used in ART. Thus, information concerning the etiology of the problem is limited and the risk of using a DNA damaged spermatozoa during in vitro fertilization might be overestimated, causing the application of unnecessary, highly invasive, and expensive treatments.

Most of these techniques were introduced decades ago. Since then, few major changes have been implemented for their improvement. In the specific case of the SCSA, this method was introduced almost 40 years ago, and since then only minor changes regarding the nuclear denaturation have been applied. In this sense, the inventors' co-staining represents a significant modification that can improve its predictive power, and offers a more accurate and specific readout of the magnitude of the DNA damage in the sperm population.

On the other hand, the TUNEL assay, introduced in 1993, has undergone several modifications mainly to increase its sensitivity; but although being improved, the lack of a unified methodology of this test makes it difficult to compare data. This approach also offers the possibility of being combined with a LIVE/DEAD fixable dye for the simultaneous assessment of membrane integrity and DNA damage, which can provide more information than individual probes, but it also has its limitations. The TUNEL+LD requires multiple methodological steps, with which the protocol duration can take more than one day (due to overnight fixation, depending on the protocol) and the accuracy of the values of membrane integrity and DNA fragmentation could be compromised due to excessive manipulation (e.g., multiple centrifugations, DTT induced chromatin de-compaction, fixation, membrane permeabilization, antibody/ fluorochrome incubation), especially when performed by a personnel with limited experience.

The invention's double-staining counts with great practicability, given that the reaction between LD and the protein amines is covalent, the samples have the potential to be fixed, stored, and evaluated later at convenience. This feature, together with its simplicity and short processing-time (45 min) make it ideal to be used not only in clinical settings, but also for research purposes to understand the etiology of sperm DNA damage and to study specific sperm subpopulations going through oxidative stress/ apoptotic-like processes.

Like the TUNEL+LD combination, the inventors' AO+LD staining, provides the distinction of four sperm subpopulations **(Fig. 6),** but unlike the TUNEL+LD, excessive sample handling is not necessary and the formulation of an accurate "DNA and Membrane Integrity Ratio (DMI)" is possible **(Fig. 7).** This novel parameter makes the assessment and categorization of DNA fragmentation in membrane intact spermatozoa possible, which are potentially, one of the most harmful cells when using ARTs. Currently, the only method to evaluate sperm DNA fragmentation with "established" clinical thresholds, SCSA, does not consider membrane integrity in its DFI calculation; therefore the risk of using spermatozoa with defective DNA during ART is overestimated, when the DNA defective spermatozoa belong to the death fraction of ejaculate, like in the **Figure 10C**-E. Therefore, the DMI ratio can accurately reveal the real risk of using DNA fragmented spermatozoa during ARTs, facilitating decision-making for medical professionals, regarding which treatment is most appropriate for each couple. Thus, less expensive and less invasive treatments, like in vitro fertilization or artificial insemination, can be applied instead of Intracytoplasmic sperm injection (ICSI), avoiding unnecessary procedures, saving time, money and relieving stress to the couple by optimizing their ART treatment.

In this way, the inventors' co-staining has a huge potential to be implemented in research and clinical settings for the evaluation of unexplained andrological conditions, providing fast and accurate practical information to decide the best treatment to apply.

In conclusion, the results presented in the present invention validate the use of the inventors' innovative co-staining AO + LD as a feasible tool for the simultaneous evaluation of sperm membrane integrity and DNA fragmentation. This staining method consists of a simple protocol, with no spectral overlap and potential for fixation. This easy-to-perform assessment allows the identification of spermatozoa with intact membrane and no DNA fragmentation and the fraction of potentially harmful membrane intact-DNA fragmented spermatozoa, providing a more accurate and simple analysis compared to other methods. The herein presented protocol can open novel concepts to understand sperm damage, improve diagnosis in spermatology and given its simplicity, it has the potential to be applied in clinical settings in the short-term future.

**Further Definitions**

It is noted that as used herein, the singular forms "a", "an", and "the", include plural references unless the context clearly indicates otherwise.

Unless otherwise indicated, the term "at least" preceding a series of elements is to be understood to refer to every element in the series. The term "at least one" refers, if not particularly defined differently, to one or more such as two, three, four, five, six, seven, eight, nine, ten or more.

The term "and/or" wherever used herein includes the meaning of "and", "or" and "all or any other combination of the elements connected by said term".

The term "less than" or in turn "more than" does not include the concrete number.

For example, less than 20 mean less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step. When used herein, the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

The term "about" preceding a respective value means plus or minus 10 % of that value, preferably plus or minus 5 %, more preferably plus or minus 2 %, most preferably plus or minus 1 %, and also includes the value itself.

Throughout the description and claims of this specification, the singular encompasses the plural unless the context otherwise requires. In particular, where the indefinite article is used, the specification is to be understood as contemplating plurality as well as singularity, unless the context requires otherwise.

A better understanding of the present invention and of its advantages will be gained from the following examples, offered for illustrative purposes only.

## EXAMPLES OF THE INVENTION

### Example 1: Simultaneous detection of sperm membrane integrity and DNA fragmentation by flow cytometry: a novel and rapid tool for sperm analysis

The need to better understand sperm physiology and updating the conventional seminal evaluation, has led to consider sperm DNA integrity as one of the most promising biomarkers in basic and clinical andrology. However, the lack of an easy to apply method has prevented this parameter to be exploited at his full potential for research and in clinical settings. In the present application, the inventors provide a novel, rapid and easy to perform co-staining of acridine orange and LIVE/DEAD fixable dye, in which the inventors of the present invention combine the principle of the Sperm Chromatin Structure Assay to evaluate DNA fragmentation in membrane intact spermatozoa. The assay was validated against the use of independent proves, to rule out cross interference between them. The results demonstrated the accuracy and practicality of this assay. This new protocol provides a more accurate analysis of the DNA status, led to novel concepts to understand sperm damage and improves diagnosis in andrology in the immediate future.

### MATERIALS AND METHODS

#### Semen Collection

Human normozoospermic semen samples, according to the guidelines of the World Health Organization, were collected from thirteen (n = 13) donors of 25-35 years old by masturbation after 3-5 days of sexual abstinence. Written, informed consent was obtained for all the participants. Ethical approval was given by the local ethics committee.

#### Semen Processing

After the liquefaction of the samples (30 min), each of them was divided into two aliquots; the first aliquot was diluted ($10\times10^6$ spermatozoa/ml) in Human Tubal Fluid buffer (HTF) and divided again into two for the separate evaluation of membrane integrity by propidium iodide (PI) and DNA fragmentation by SCSA. The second aliquot was centrifuged (300 g, 5 min) to remove the seminal plasma and re-suspended in HTF ($10\times10^6$ spermatozoa/ml) to be co-stained using LIVE/DEAD™ Fixable Blue Dead Cell Stain kit (Invitrogen™) and AO for the simultaneous evaluation of membrane integrity and DNA fragmentation. For each condition, the final sperm concentration during the assay was $1\times10^6$ spermatozoa/ml.

#### Evaluation of Sperm Membrane Integrity

For the assessment of membrane integrity using PI-only, 1 ml of sperm suspensions ($1\times10^6$ spermatozoa/ml), was stained at 37°C with 1 μl of PI stock solution (2.5 mg/ml). The samples were incubated in the dark for 10 min at 37°C before examination via flow cytometry.

#### Evaluation of Sperm DNA fragmentation

The SCSA protocol has been described by Evenson elsewhere. Briefly, a total of $1\times10^6$ cells were treated with a low-pH (1.2) detergent solution for 30 seconds and then stained with 6 mg/L purified AO in phosphate-citrate buffer, pH 6.0. When excited at 488 nm, AO intercalated with double-stranded DNA emits green fluorescence and AO associated with single-stranded DNA emits red fluorescence. No-fragmented (green fluorescence) versus fragmented DNA (red fluorescence) cytograms were used to determine the percentage of DNA fragmentation of the entire sperm population (% DNA fragmentation index or %DFI), which was quantified based on the increased ratio of red/[red + green] fluorescence. A standard sample (≤ 15 %DFI) was used as control at the beginning of each round of experiments to verify threshold differentiating no-fragmented DNA spermatozoa (main population of cells) and fragmented spermatozoa (cells outside of the main population).

**Simultaneous evaluation of sperm membrane integrity and DNA fragmentation**

A two steps protocol was implemented to achieve the co-staining for the simultaneous evaluation of membrane integrity and DNA fragmentation with LD and AO respectively. In the first step, according to the specifications of the manufacturer, sperm suspensions were incubated in the dark for 30 min at 37°C with the LD, to be then washed 1× (300 g, 5 min, 37 °C) to remove the excess dye before being re-suspended in 300 μl of TNE Buffer. LD is an amine-reactive dye that reacts covalently with exposed protein amines, external amines when the cell is alive and external and internal when the cell is dead, reflecting, therefore, the membrane integrity by differential fluorescent intensity. This dye with an excitation maximum of ~350 nm, ideal for UV lasers, and emission of ~450 nm, leaves free the channels of the blue 488 nm laser for the green and red fluorescence emissions of the AO, avoiding in this way any spectral overlap issues and eliminating the need of color compensation. The second step consists of staining the cells with AO following a similar protocol as the SCSA, which was described above.

**Flow Cytometry**

The analysis by Flow cytometry were performed using a high-resolution flow cytometer, CytoFLEX S (Beckman Coulter, Krefeld, Germany), equipped with four diode lasers emitting at 375, 405, 488, and 638 nm; and 15 optical filters. The PI was excited at 488 nm and the emitted fluorescence was detected using a 690/50 BP filter. The LD dye was exited using a near UV laser 375 nm and its corresponding emitted fluorescence was detected with a 450/45 BP filter. For the AO, the dye was excited with a 488 nm laser and two different filters 525/40 BP and 690/50 BP were used to detect green and red fluorescence respectively. Fluorescence information regarding 10 000 spermatozoa was collected for each sample/ condition. Unstained and single stained control samples were used to set quadrants and as well to determine background fluorescence. For the co-staining LD+AO, no color compensation was needed. Additional positive controls such as Triton X-100 0.1 % permeabilized-spermatozoa (for PI and LD) and UVB 312 nm light exposed spermatozoa 30 min (for AO) were also used to confirm the limits of the quadrants. Files were exported and analyzed using FCS Express 6 Plus Research Edition (FCS Express V 6; De Novo Software).

**Experimental Design**

The co-staining LD + AO requires the previous removal of seminal plasma (300 g, 5 min at 37°C) and 30 min of incubation before the sample can be evaluated. Therefore, to confirm that there are no effects from centrifugation and incubation time, paired measurements were carried out. In this sense, to evaluate the similarity of the measurements of the methods, membrane integrity, and DNA fragmentation were evaluated independently at time 0 using PI and SCSA respectively, in native ejaculates, which were diluted for concentration normalization. These values were compared with the ones obtained from the use of the inventors co-staining LD + AO after 30 minutes in washed samples. Furthermore, to test the accuracy and sensitivity of the co-staining, sperm suspensions were mixed in different proportions with a positive control sample of 100 % of DNA and membrane integrity damage (exposed to UVB 312 nm for 10 min), based on the dilution of the positive control (20, 40, 60, 80 and 100%) and the native values of the samples, theoretical values of membrane integrity and DNA fragmentation were calculated for each condition. Three additional samples were used for this experiment.

**Statistical analysis**

The data was assessed using a one-tailed T-test for independent samples. Differences were considered significant when $P < 0.05$. The results are displayed as box and whisker plots. Bland and Altman plots were constructed to evaluate the similarity of the results as follows: the differences between paired measurements on the same samples were calculated, and the mean of the differences (d) was used to estimate the average bias of one method relative to the other. The 95 % limit of agreement was calculated as x 2 SD, where SD is the standard deviation of the differences between paired measurements. All calculations were performed using Microsoft Excel for Windows 10. To evaluate the sensitivity of the method, R square values were calculated after performing linear regression for the experiments using theoretical and experimental values of membrane integrity and sperm DNA fragmentation.

## RESULTS

### Evaluation of Sperm Membrane Integrity

The objective of this experiment was to assess whether there is any difference in the values of membrane integrity, when they are evaluated using the inventors' co-staining (LD+AO, including centrifugation and a 30 min incubation), in contrast to the direct evaluation of this parameter at Time 0 using PI. The results of this comparison can be observed in **Figure 1A** and **1B,** where the values of membrane integrity obtained with PI and LD in combination with AO are represented in box and whiskers plots **(Fig. 1A).** As shown, the ranges between the measurements of both methods are similar and no significant differences were detected P = 0.755. Moreover, the Bland and Altman Plots also revealed that both assays produced similar results since all paired values fall within the limits of agreement **(Fig. 2A).**

### Evaluation of Sperm DNA fragmentation

DNA fragmentation values obtained from the use of SCSA and the co-staining AO + LD were also compared. Also, in this case, no significant differences were observed between the values of DNA fragmentation obtained from both methods (P = 0.976) **(Fig. 3A).** Given these results, it is not surprising that the method agreement shows 100 % of the values within the limits of agreement **(Fig. 2B).**

### Simultaneous evaluation of sperm membrane integrity and DNA fragmentation

The sperm population was selected using their FSC versus SSC properties **(Fig. 5A)** and green versus red fluorescence cytograms **(Fig. 5B)** were used to determine the levels of DNA fragmentation of the sperm population after transformation of the data **(Fig. 5C).** Finally, the values of fluorescence for the LD **(Fig. 5D)** were combined with the levels of DNA fragmentation in a dot plot **(Fig. 6).**

To analyze the values of DNA fragmentation and membrane integrity in a combined way, classical dot plots were used **(Fig. 6),** where each dot represents a cell, while the limits of each quadrant were established based on unstained/non-damaged and single stained (positive) control samples. In this way, when the values DNA fragmentation (X-axis) and membrane integrity (Y-axis) are displayed, it is possible to identify four sperm subpopulations: (Q1) membrane non-intact with no DNA fragmentation, (Q2) membrane non-intact with DNA fragmentation, (Q3) membrane intact with no DNA fragmentation, (Q4) membrane intact with DNA fragmentation. The sum of the values of the right quadrants (Q2 + Q4) is equivalent to the DNA fragmentation index (DFI) provided by the conventional SCSA. Moreover, with the information of the lower quadrants (Q3 and Q4), provided by the method of the present invention, a new parameter, the DNA and Membrane Integrity Ratio (DMI) can be formulated **(Fig. 7);** making possible the assessment and categorization of DNA fragmentation in membrane intact spermatozoa. The power of this new parameter can be more clearly appreciated in **Figure 10.**

In **Figure 10,** the versatility of the combined staining plots is evident when evaluating samples with increasing values of membrane and DNA fragmentation (higher proportion of positive control), where the dots corresponding to damaged sperm "migrate" towards the upper quadrants in proportion with the magnitude of the damage in the sperm population. Additionally, the usefulness of the DMI ratio can be better appreciated when compared to the DFI values for each of the conditions (DMIIDFI%): 6.4118.40 % (native), 2.25|20.58 % (20 % +ctrl), 3.77|47.72 % (40 % +ctrl), 4.85|63.42 % (60 % +ctrl), 6.24186.42 % (80 % +ctrl) and 99.18|99.93 % (100 % +ctrl). In this sense, it is possible to observe how for each condition the values of DIM ratio show only the extent of DNA fragmentation in the membrane intact fraction of membrane intact spermatozoa, which is the population of interest, while the DFI shows the extent of DNA fragmentation of the entire sperm population.

The evaluation of samples with different proportions of damage was also used to further prove the accuracy of the method of the present invention, as an additional test to prove no random cell loss due to sample handling **(Fig. 11).** Starting from known DNA fragmentation values in native samples and positive controls, mixtures of these samples were prepared and theoretical DNA fragmentation and membrane damage were calculated to compare them with their corresponding experimental values. In **Figure 11,** it is possible to observe how theoretical and experimental values are well fitted to the line in both membrane integrity ($R^2$ = 0.9959) **(Fig. 11A)** and DNA fragmentation ($R^2$ = 0.9843) **(Fig. 11B).**

### Example 2: Sperm DNA fragmentation by TUNEL+LIVE/DEAD™ and Acridine Orange+LIVE/DEAD™

The Terminal deoxynucleotidyl transferase (TdT) dUTP Nick-End Labeling (TUNEL) and the Sperm Chromatin Structure Assay (SCSA) are the two most used flow cytometry methods for sperm DNA integrity assessment. Each of these assays is based on different principles. On one side the TUNEL is based on the integration of labeled nucleotides by the enzymatic action of a deoxynucleotidyl transferase (TdT) and on the other side the SCSA is based on the incorporation of acridine orange and its metachromatic properties (fluorescence change), after using a low-pH-protocol to denature DNA at sites of DNA strand breaks. These methods are the precursors of the TUNEL + LIVE/DEAD and the acridine orange + LIVE/DEAD, respectively, in which a vitality dye to evaluate membrane integrity, the LIVE/DEAD stain, was added to improve the specificity of the test. In theory, given that the staining protocol to evaluate membrane integrity is the same for both improved methods, their differences should only depend on technical aspects related to the evaluation of the sperm DNA damage. In this sense, since TUNEL and SCSA evaluate a different aspect of the DNA damage they have their own advantages and limitations. To test the accuracy of both improved methods of the present invention, the inventors designed an experiment in which untreated sperm suspensions were mixed in different proportions with a positive control sample of 100 % of DNA and membrane integrity damage (exposed to UVB 312 nm for 10 min or DNase 1 mg/ml 37 °C 1.5 h), both cell suspensions had the same sperm concentration ($1 \times 10^6$ sperm/ml) and based on the dilution of the positive control (0, 20, 40, 60, 80 and 100 %) and the native values of the samples for both parameters, theoretical values of membrane integrity and DNA damage were calculated for each condition. The difference between theoretical and experimental percentage values of membrane integrity and DNA damage was evaluated for both methods, the staining protocol for DNA evaluation was performed following the description of the Ribeiro et al. and Evenson et al. publications, for the TUNEL+ LIVE/DEAD and the LIVE/DEAD + acridine orange, respectively. Three seminal samples from Human normozoospermic donors in accordance to the World Health Organization guidelines were used for this experiment. The samples were collected by masturbation after 3 - 5 days of sexual abstinence. Written, informed consent was obtained for all the participants. Ethical approval was given by the local ethics committee.

## MATERIALS AND METHODS

### Semen Collection

Human normozoospermic semen samples in accordance to World Health Organization, were collected from ten (n = 10) donors (aged 25-35 years old) by masturbation after 3 - 5 days of sexual abstinence. Written, informed consent was obtained for all the participants. Ethical approval was given by the local ethics committee.

### Semen Processing

After liquefaction of the sample (30 mins at 37 °C in incubator), the sperm concentration is evaluated using an improved Neubauer chamber according to the guidelines of the WHO, 2010. An aliquot of the sperm sample is prepared to reach a sperm concentration of $1 \times 10^6$ cells/ml. To remove the seminal plasma, the sperm suspension is centrifuged (300 g, 5 min) and re-suspended ($1 \times 10^6$ spermatozoa/ml) in human tubular fluid (HTF) containing (in mM): 97.8 NaCl, 4.7 KCl, 0.2 $MgSO_4$, 0.37 $KH_2PO_4$, 2 $CaCl_2$, 0.33 Na-pyruvate, 21.4 Na-lactic acid, 2.8 glucose and 21 HEPES, adjusted to pH 7.35 with NaOH.

### Simultaneous evaluation of sperm membrane integrity and DNA fragmentation

A two-step protocol was implemented to achieve the co-staining for the simultaneous evaluation of membrane integrity and DNA fragmentation with LIVE/DEAD and acridine orange, respectively. In the first step, according to the specifications of the manufacturer, sperm suspensions were incubated in the dark for 30 min at 37 °C with the LIVE/DEAD, to be then washed 1× (300 g, 5 min, 37 °C) to remove the excess dye before being re-suspended in 300 μl of TNE Buffer. The second step consists of staining the cells with acridine orange according to the SCSA protocol as described above.

### LIVE/DEAD staining (Invitrogen Kit)

According to the manufacturer recommendations, 1 μL of LIVE/DEAD Fixable Blue Dead Cell Stain kit (Invitrogen) is added to each ml of sperm suspension ($1 \times 10^6$ cells/ml) in order to reach a final concentration of 0.1 % of the dye. The cell suspension is mixed gently and incubated for 30 minutes in the dark at 37 °C in the incubator.

In extension of this kit procedure, the sample is again centrifuged (300 g, 5 min) and re-suspended ($1 \times 10^6$ cells/ml) in TNE Buffer (mM): 10 Tris·Cl, 150 NaCl, 1 EDTA.

### Acridine orange staining (according to Evenson et *al.,* 1980 and 2013)

A total of $1 \times 10^6$ cells are treated for 30 seconds, with a low-pH (1.2) detergent solution: 0.08 N HCl, 150 mM NaCl, 0.1 % Triton X-100, adjusted to pH 1.2 with HCl and/or NaOH. Immediately after the 30 seconds of acid-detergent treatment, the cells are exposed to a staining solution containing: 6 mg/L purified acridine orange, 0.1 M citric acid, 0.2 M $Na_2HPO_4$, 1 mM disodium EDTA, 150 mM NaCl, adjusted to pH to 6.0 with NaOH.

**Flow Cytometry**

Flow cytometric analysis was conducted using a high-resolution flow cytometer, CytoFLEX S (Beckman Coulter, Krefeld, Germany), equipped with four diode lasers emitting at 375, 405, 488, and 638 nm; and 15 optical filters. Propidium Iodide (PI) was excited at 488 nm and the emitted fluorescence was detected using a 690/50 BP filter. The LIVE/DEAD dye was exited using a near UV laser 375 nm and its corresponding emitted fluorescence was detected with a 450/45 BP filter. For the acridine orange, the dye was exited with a 488 nm laser and two different filters 525/40 BP and 690/50 BP were used to detect green and red fluorescence respectively. Acridine orange equilibration buffer (400 μl acid-detergent solution + 1.2 ml acridine orange staining solution) was flushed through the cytometer for ~15 min prior to calibrate the instrument. After 5 min of incubation time, the standard sample was used to establish comparable instrument settings. FSC-A vs. SSC-A and 690/50 vs. 525/40 (dot-plots); 450/45, 690/50, 525/40 (histograms) were displayed. Either a standard sample or calibration beads were used to set the red and green photomultiplier tube (PMT) voltage gain. The mean red and green fluorescence values should be set at ~1300/10.000 and ~5000/10.000 channels, respectively. For the blue fluorescence of the live spermatozoa, the PMT were set at ~2500/10000. The fluorescence information of at least 5000 spermatozoa from each sample/condition were collected. For the co-staining LIVE/DEAD + acridine orange no color compensation was needed. Additional positive controls, such as Triton X-100 0.1 % permeabilized spermatozoa (for propidium iodide (PI) and LIVE/DEAD) and UVB light exposed spermatozoa 30 min (for acridine orange) were included. Files were exported and analyzed using FCS Express 6 Plus Research Edition (FCS Express V 6; De Novo Software).

**Experimental Design**

At difference with the evaluation of membrane integrity and DNA fragmentation by individual probes (e.g., propidium iodide (PI) and acridine orange only), the co-staining LIVE/DEAD + acridine orange requires the previous removal of seminal plasma (300 g, 5 min at 37 °C) and 30 min of incubation before the sample can be evaluated. Therefore, to confirm that there are no effects from centrifugation and incubation time, paired measurements were carried out. In this sense, to evaluate the correlation between the measurement, membrane integrity and DNA fragmentation were evaluated independently at time 0 using propidium iodide (PI) and acridine orange, respectively, in diluted native ejaculates (for concentration normalization). These values were compared with the ones obtained from a co-staining of LIVE/DEAD + acridine orange 30 minutes in washed samples.

**Statistical Analyzes**

The data was assessed using a two tailed T-test. Differences were considered significant when $P < 0.05$. The results are displayed as box and whisker plots. Correlations among assays were determined using linear regression showing R square values. Bland and Altman Plots, a method agreement analysis technique, of the two staining protocols were constructed. The differences between paired measurements on the same samples were calculated, and the mean of the differences (d) was used to estimate the average bias of one method relative to the other. The 95 % limit of agreement was calculated as x 2 SD, where SD is the standard deviation of the differences between paired measurements. All calculations were performed using Microsoft Excel for Windows 10.

**RESULTS**

Characteristic combined staining plots are shown for TUNEL + LIVE/DEAD **(Fig. 9)** and LIVE/DEAD + acridine orange **(Fig. 10),** in which similar patterns can be recognized, in both cases the dots corresponding to damaged sperm "migrate" towards the upper quadrants in proportion with the magnitude of the damage in the sperm population. A more detailed analysis of these experiments can be seen in **Figure 11,** where the mean difference between theoretical and experimental percentage values of membrane integrity and DNA damage was evaluated for each condition and plotted against zero. The upper and lower limits represent two times the standard deviation of the differences of both methods. In this figure, it is possible to observe how the measurements obtained with the LIVE/DEAD + acridine orange co-staining keep a lower difference for both membrane integrity and DNA integrity when compared to the values of the TUNEL + LIVE/DEAD protocol. This is particularly true for the values regarding membrane integrity, of which several of them fall outside the confidence limits. Likewise, concerning the measurements of DNA integrity, although all the values fall within the confidence limits, it is clear how the difference between experimental values and theoretical values increases proportionally to the amount of DNA damage in the sperm population (higher proportion of positive control cells). This last observation is evident when visualizing the association between the values of DNA damage provided for each method against the amount of DNA damage in the sperm population based in the used proportion of positive cells **(Fig. 12).** In the **Figure 12,** it can be observed, that even though both methods show a very high correlation ($R^2 = 0.99$ for LIVE/DEAD + acridine orange and $R^2 = 0.97$ for the TUNEL + LIVE/DEAD), it is also clear that the LIVE/DEAD + acridine orange co-staining has a higher sensibility.

**Example 3: Comparative Example**

This example is to be understood as a comparative Example in view of Moskovtsev S. I. et al. (ARCHIVES OF ANDROLOGY, vol. 51, no. 1, 2005, pages 33-40) and all other existing methods of the state-of-the-art, wherein independent probes for evaluating the mentioned parameters are used.

In Moskovtsev S. I. *et al.,* the evaluation of sperm DNA integrity (SCSA) and their membrane integrity (HOS test) are assessed independently (for each sample, one aliquot was used for DNA fragmentation and another aliquot for membrane integrity). Therefore, their categorization consists of only independent percentages corresponding to each parameter. This presents a huge difference to the methods of the present invention as described herein.

To highlight these differences between the method of Moskovtsev S. I. *et al.* and the method of the present invention, the inventors applied the analytical approach used in Moskovtsev S. I. *et al.* to one of their samples. By this way, all the values that were possible to obtain considering membrane integrity and DNA fragmentation were: live cells %, dead cells %, DNA fragmented cells %, and non-DNA fragmented cells %.

The used sample had the following total values: 52.5 % live cells; 47.5 % dead cells; 27.21 % of cells with DNA fragmentation, 72.79 % with non-DNA fragmentation. Such a sample with a DNA fragmentation index of 27.21 % is considered as high (> 25 %) (Giwercman, et al. 2010). However, such values do not represent a real stratification, because it is not possible to know the status of both parameters at the single-cell level (e.g., if the DFI is associated to live or dead spermatozoa), so that this example represents the limitation of the approach used in Moskovtsev S. I. *et al.*

In contrast thereto, method of the present invention considers the two parameters simultaneously, which resolves the specificity of the assay into individual cells, allowing their stratification into four sperm categories. By this way, the same sample mentioned above has been resolved as follows and gave the following values: 6.83 % of live cells with DNA fragmentation; 45.67 % of live cells without DNA fragmentation; 20.38 % of dead cells with DNA fragmentation, 27.12 % of dead cells without DNA-fragmentation. Consequently, it was possible to recognize that most of the sperm DNA fragmentation of the sample is associated with dead spermatozoa (20.38 %).

This information can also be gained from the Table 1 following directly below:

**Table 1.** Analytical differences between the method of the present invention and single staining. The method of the present invention (light grey) allows the stratification of the sample in four categories. Thus, membrane integrity and DNA fragmentation correlate at a single-cell level. Single parameter evaluation (dark grey) is limited to independent total percentages corresponding to each parameter with which sample stratification is not possible.

| | No DNA fragmentation (%) | DNA fragmentation (%) | Total |
|---|---|---|---|
| Live (%) | 45.67 | 6.83 | 52.5 |
| Dead (%) | 27.12 | 20.38 | 47.5 |
| Total | 72.79 | 27.21 | 100 |

This information is critical for the implementation of assisted reproductive technologies (ART), in which, if a sample is associated with a high DFI, the option to be considered is normally intracytoplasmic sperm injection (ICSI), which is highly invasive and expensive. However, if the DNA defective spermatozoa belong to the dead fraction of the ejaculate, as can be seen from this example, less invasive and more accessible treatments such as in-vitro fertilization (IVF) or Intrauterine insemination (IUI) can be applied, because for such treatments only live spermatozoa are used. The detection of these kinds of situations is not possible if independent probes are used (as, e.g., in the case of Moskovtsev S. I. *et al.*). By this way, the insights provided by the co-staining of the present invention would facilitate decision-making for medical professionals with regards to treatment selection, avoiding unnecessary procedures, saving time, money and relieving stress to the couple by optimizing their ART treatment.

Furthermore, since the stratification provided by the method of the present invention allows a deeper dissection of the live spermatozoa, it is possible to formulate a new parameter that describes the extent of DNA fragmentation in that fraction. Such parameter is the DMI% as defined herein.

In this respect, since dead and DNA fragmented cells imply a risk of fertilization failure, the DMI% represents the chances of using defective spermatozoa in ART. In the case of this example, the percentage of live cells with DNA fragmentation (DMI %) is 14.37 % ([6.83/6.83+45.67]x100), which is comprehensibly lower than the previously mentioned DFI value (27.21 %). Consequently, the DMI % allows also the appraisements of the functionality of sperm cells in the sample. The high accuracy of this parameter is only possible with the unique co-staining protocol and the method of the present invention. Only the methods of the present invention allow measuring the pure population of spermatozoa really having damaged DNA. The method of the present invention is further capable of analyzing DNA fragmentation in live fractions of spermatozoa without the need for a previous selection step. This highlights the advantages of the method of the present invention, using e.g. co-staining, over the use of single dyes and suggests its utility for the evaluation of the effectiveness of sperm selection protocols and media.

**References:**

1. Cissen M, Wely Mv, Scholten I, Mansell S, Bruin JPd, Mol BW, Braat D, Repping S, Hamer G. Measuring Sperm DNA Fragmentation and Clinical Outcomes of Medically Assisted Reproduction: A Systematic Review and Meta-Analysis. PloS one 2016;11:e0165125-e0165125.

2. Collins JA, Barnhart KT, Schlegel PN. Do sperm DNA integrity tests predict pregnancy with in vitro fertilization? Fertility and Sterility 2008;89:823-831.

3. Cossarizza A et al., Guidelines for the use of flow cytomerty and cell sorting in immunological studies. Eur J Immunol. 2017. 47:1584-1797.

4. Evenson D, Darzynkiewicz Z, Melamed M. Relation of mammalian sperm chromatin heterogeneity to fertility. Science 1980;210:1131-1133.

5. Evenson DP. Sperm Chromatin Structure Assay (SCSA®). In: Carrell DT, Aston KI, editors. Spermatogenesis: Methods and Protocols. Totowa, NJ: Humana Press; 2013, p 147-164.

6. Evenson DP. The Sperm Chromatin Structure Assay (SCSA®) and other sperm DNA fragmentation tests for evaluation of sperm nuclear DNA integrity as related to fertility. Animal Reproduction Science 2016, 169:56-75.

7. Ribeiro SC, Muratori M, De Geyter M, De Geyter C. TUNEL labeling with BrdUTP/anti-BrdUTP greatly underestimates the level of sperm DNA fragmentation in semen evaluation. PLOS ONE 2017;12:e0181802.

8. WHO. WHO laboratory manual for the examination and processing of human semen. 5 Th Edition ed. Geneva - Switzerland: WHO Press, World Health Organization; 2010.

9. Thermo Fisher Scientific. LIVE/DEAD™ Fixable Blue Dead Cell Stain Kit, for UV excitation. 2019, July 28.

10. Moskovtsev SI et al. Sperm Dna Integrity: Correlation With Sperm Plasma Membrane Integrity In Semen Evaluated For Male Infertility: ARCHIVES OF ANDROLOGY, Vol. 51, No. 1, 2005, pages 33-40.

11. Quinn P, Kerin JF, Warnes GM. Improved pregnancy rate in human in vitro fertilization with the use of a medium based on the composition of human tubal fluid. Fertil Steril. 1985;44:493-498.

12. Strünker, T., Goodwin, N., Brenker, C. et al. The CatSper channel mediates progesterone-induced $Ca^{2+}$ influx in human sperm. Nature 471, 382-386 (2011).

13. Giwercman A, Lindstedt L, Larsson M, et al. Sperm chromatin structure assay as an independent predictor of fertility in vivo: a case-control study. Int. J. Androl. 2010;33:221-7.

**Claims**

1. A method for stratifying spermatozoa comprised in a sample obtained from a subject, comprising

   (a) assaying spermatozoa comprised by said sample for membrane integrity; and
   (b) assaying spermatozoa comprised by said sample for DNA fragmentation,
   wherein the stratification allows a prediction whether said subject will have spermatozoa which may be functional, wherein spermatozoa are stratified as follows:

   (i) membrane non-intact spermatozoa, having a DNA fragmentation index (%DFI) portion of at most about 5 %;
   (ii) membrane non-intact spermatozoa, having a DNA fragmentation index (%DFI) portion of at least about 1 %;
   (iii) membrane intact spermatozoa, having a DNA fragmentation index (%DFI) portion of at most about 5 %;
   (iv) membrane intact spermatozoa, having a DNA fragmentation index (%DFI) portion of at least about 1 %;

   wherein the percentage of DNA fragmentation index (%DFI) of said sample is between about 4 %DFI to about 75 %DFI, wherein, the presence of spermatozoa pursuant to (iii) is indicative that said subject will have spermatozoa which may be functional, and wherein the presence of spermatozoa pursuant to (i), (ii) and/or (iv) is predictive that said subject will have spermatozoa, which may not be functional.

2. The method of claim 1, wherein a sperm sample that has a %DFI between about 4 %DFI to about 30 %DFI contains predominantly spermatozoa, which may be functional, or wherein a sperm sample that has a %DFI between about 30 %DFI to about 75 %DFI contains predominantly spermatozoa, which may not be functional.

3. The method of any one of the preceding claims, wherein, if the subject has predominantly spermatozoa of category (i), (ii) and/or (iv), such spermatozoa are to be used for IVF or ICSI.

4. A method for determining whether functional spermatozoa are present in a sample obtained from a subject, comprising

   (a) assaying spermatozoa comprised by said sample for membrane integrity; and
   (b) assaying spermatozoa comprised by said sample for DNA fragmentation;

   wherein the presence of membrane intact spermatozoa having a DNA fragmentation index (%DFI) portion of at most about 5 % in said sample is predictive that said subject will have spermatozoa, which may be functional.

5. A method for determining whether non-functional spermatozoa are present in a sample from a subject, comprising

   (a) assaying spermatozoa comprised by said sample for membrane integrity; and
   (b) assaying spermatozoa comprised by said sample for DNA fragmentation;
   wherein the presence of membrane non-intact spermatozoa having a DNA fragmentation index (%DFI) portion of at most about 5 % in said sample is predictive that said subject will have spermatozoa, which may not be functional,
   wherein the presence of membrane non-intact spermatozoa having a DNA fragmentation index (%DFI) portion of at least about 1 % in said sample is predictive that said subject will have spermatozoa, which may not be functional, and/or
   wherein the presence of membrane intact spermatozoa having a DNA fragmentation index (%DFI) portion of at least about 1 % in said sample is predictive that said subject will have spermatozoa, which may not be functional.

6. The method of any one of the preceding claims, wherein functional and/or non-functional spermatozoa are subject to one or more viability tests, and/or

   wherein membrane integrity is assayed by a dye differentiating between live and dead cells, and/or
   wherein DNA damage is assayed by a sperm DNA fragmentation test, and/or
   wherein flow cytometry is used for assaying said sample for membrane integrity and/or for assaying said sample for sperm DNA fragmentation, and/or
   wherein assaying a sample comprising spermatozoa for membrane integrity includes determining the number of spermatozoa not showing membrane integrity and/or determining the number of spermatozoa showing membrane integrity, and/or
wherein assaying a sample comprising spermatozoa for sperm DNA fragmentation includes determining the number of spermatozoa comprising fragmented DNA and/or determining the number of spermatozoa not comprising fragmented DNA,
and/or wherein the number of membrane intact DNA fragmented spermatozoa is set in ratio to the total number of membrane intact spermatozoa, thereby indicating the DNA and membrane integrity (DMI) ratio:

$$DMI\ ratio = \frac{membrane\ intact\ DNA\ fragmented\ spermatozoa}{total\ membrane\ intact\ spermatozoa}$$, and/or

wherein functional spermatozoa are potentially fertile spermatozoa, and/or
wherein non-functional spermatozoa are potentially infertile spermatozoa.

**7.** The method of any one of the preceding claims, wherein said stratifying or determining comprises the steps of:

1) assaying spermatozoa for membrane integrity (a) and DNA fragmentation (b);
2) sorting the spermatozoa according to the dye used for (a) and (b);
3) selecting the majority of spermatozoa according to their FSC-A and SSC-A signal;
4) analyzing the fluorescence values in a histogram representing (a) for individual spermatozoa;
5) analyzing the fluorescence values in a histogram representing (b) for individual spermatozoa;
5)i) calculating the %DFI from the histogram representing (b) for individual spermatozoa;
6) combining the fluorescence values of (a) and (b) obtained in the steps 4) and 5) per sample in a bivariate logarithmic density plot of the fluorescence intensity; and
6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds.

**8.** The method of claim 7, wherein calculating the percentage of the DNA fragmentation index (%DFI) per sample, is established by determining the number of spermatozoa with moderate and high DNA fragmentation in relation to the number of spermatozoa with low DNA fragmentation from the fluorescence values in the histogram representing (b) of individual spermatozoa of said sample analyzed in step 5), and/or wherein the spermatozoa of said sample are stratified or determined according to claim 1.

**9.** A method for stratifying or determining spermatozoa comprised in a sample from a subject, wherein the method comprises the steps of:

1) assaying spermatozoa for membrane integrity (a) and DNA fragmentation (b);
2) sorting the spermatozoa according to the dye used for (a) and (b);
3) selecting the majority of spermatozoa according to their FSC-A and SSC-A signal;
4) analyzing the fluorescence values in a histogram representing (a) for individual spermatozoa;
5) analyzing the fluorescence values in a histogram representing (b) for individual spermatozoa;
6) combining the fluorescence values of (a) and (b) obtained in the steps 4) and 5) per sample in a bivariate logarithmic density plot of the fluorescence intensity; and
6)i) establishing a fluorescence level threshold for membrane intact and membrane non-intact spermatozoa, and a fluorescence level threshold for spermatozoa with and without DNA fragmentation, by using spermatozoa of a calibration sample or calibration beads to stratify or determine the spermatozoa according to said thresholds.

**10.** The method of any one of claims 7, 8 or 9, wherein the spermatozoa are thereby stratified or determined as follows:

I. membrane non-intact spermatozoa, having non-fragmented DNA, with a spermatozoa number between about 20 % and about 60 % of the total spermatozoa number, preferably about 35 % of the total spermatozoa number measured per sample;
II. membrane non-intact spermatozoa, having fragmented DNA, with a spermatozoa number of at least about 1 % of the total spermatozoa number measured per sample;
III. membrane intact spermatozoa, having non-fragmented DNA, with a spermatozoa number between about 45 % and about 75 % of the total spermatozoa number, preferably about 55 % of the total spermatozoa number measured per sample; and
IV. membrane intact spermatozoa having fragmented DNA, with a spermatozoa number of at least about 1 % of the total spermatozoa number measured per sample, preferably wherein the spermatozoa numbers of I to IV allow the calculation of the DNA and membrane integrity (DMI) ratio according to

wherein the number of membrane intact DNA fragmented spermatozoa is set in ratio to the total number of membrane intact spermatozoa, thereby indicating the DNA and membrane integrity (DMI) ratio:

$$DMI\ ratio = \frac{membrane\ intact\ DNA\ fragmented\ spermatozoa}{total\ membrane\ intact\ spermatozoa}.$$

**11.** The method of any one of the preceding claims, wherein a DNA intercalating dye is applied for the evaluation of DNA fragmentation, and/or

wherein the DNA intercalating dye applied for the evaluation of DNA fragmentation is a fluorescent dye, and/or
wherein the fluorescent DNA intercalating dye, applied for the evaluation of DNA fragmentation, is acridine orange (AO), and/or
wherein the dye differentiating live and dead cells is reactive with amines, and/or
wherein the dye differentiating live and dead cells, which is reactive with amines, is a fluorescent dye, and/or
wherein the dye for assaying DNA fragmentation and the dye for assessing membrane integrity have a different emission spectrum, thereby avoiding a spectral overlap.

**12.** A method for stratifying spermatozoa comprised in a sample from a subject, wherein the method comprises

(a) assaying spermatozoa comprised by said sample for membrane integrity;
(b) assaying spermatozoa comprised by said sample for DNA fragmentation; and wherein the spermatozoa are stratified as
membrane non-intact spermatozoa with prevalent double stranded DNA;
membrane non-intact spermatozoa with prevalent single stranded DNA or RNA;
membrane intact spermatozoa with prevalent double stranded DNA; and
membrane intact spermatozoa with prevalent single stranded DNA or RNA.

**13.** The method of any one of the preceding claims, wherein a DNA intercalating dye is applied for the evaluation of single and double stranded DNA, and/or

wherein the DNA intercalating dye applied for the evaluation of single and double stranded DNA is a fluorescent dye, and/or
wherein the dye differentiating membrane intact and membrane not-intact spermatozoa is reactive with amines, and/or
wherein the dye differentiating membrane intact and membrane not-intact spermatozoa, which is reactive with amines, is a fluorescent dye, and/or
wherein the dye for the evaluation of single and double stranded DNA and the dye differentiating membrane intact and membrane not-intact spermatozoa, which is reactive with amines, have a different emission spectrum, thereby avoiding a spectral overlap, and/or
wherein step (a) and step (b) are performed simultaneously or subsequently irrespective of the order, whereas step (a) is preferably performed prior to step (b), and/or wherein the subject is a mammal, bird or fish.

**Patentansprüche**

**1.** Verfahren zur Stratifizierung von Spermatozoen, die in einer von einem Subjekt erhaltenen Probe enthalten sind, umfassend

(a) Testen der Membranintegrität der in der Probe enthaltenen Spermatozoen; und
(b) Testen der DNA-Fragmentierung der in der Probe enthaltenen Spermatozoen,
wobei die Stratifizierung eine Vorhersage ermöglicht, ob das Subjekt Spermatozoen haben wird, die funktionsfähig sein können, wobei die Spermatozoen wie folgt stratifiziert werden:

(i) Spermatozoen mit nicht-intakter Membran, die einen DNA-Fragmentierungsindex (%DFI)-Anteil von höchstens etwa 5 % aufweisen;
(ii) Spermatozoen mit nicht-intakter Membran, die einen DNA-Fragmentierungsindex (%DFI)-Anteil von mindestens etwa 1 % aufweisen;
(iii) Spermatozoen mit intakter Membran, die einen DNA-Fragmentierungsindex (%DFI)-Anteil von höchstens etwa 5 % aufweisen;
(iv) Spermatozoen mit intakter Membran, die einen DNA-Fragmentierungsindex (%DFI) von mindestens etwa 1 % aufweisen;

wobei der Prozentsatz des DNA-Fragmentierungsindex (%DFI) der Probe zwischen etwa 4 %DFI und etwa 75 % DFI liegt, wobei das Vorhandensein von Spermatozoen gemäß (iii) darauf hindeutet, dass die Person Spermatozoen aufweisen wird, die funktionsfähig sein können, und wobei das Vorhandensein von Spermatozoen gemäß (i), (ii) und/oder (iv) vorhersagt, dass die Person Spermatozoen aufweisen wird, die möglicherweise nicht funktionsfähig sind.

**2.** Verfahren nach Anspruch 1, wobei eine Spermaprobe mit einem %DFI zwischen etwa 4 %DFI und etwa 30 %DFI überwiegend Spermatozoen enthält, die funktionsfähig sein können, oder wobei eine Spermaprobe mit einem %DFI zwischen etwa 30 %DFI und etwa 75 %DFI überwiegend Spermatozoen enthält, die nicht funktionsfähig sein können.

**3.** Verfahren nach einem der vorhergehenden Ansprüche, wobei, wenn die Person überwiegend Spermatozoen der Kategorie (i), (ii) und/oder (iv) aufweist, diese Spermatozoen für die IVF oder ICSI zu verwenden sind.

**4.** Verfahren zur Bestimmung, ob in einer von einem Subjekt erhaltenen Probe funktionsfähige Spermatozoen vorhanden sind, umfassend

(a) Testen der in der Probe enthaltenen Spermatozoen auf Membranintegrität; und
(b) Testen der in der Probe enthaltenen Spermatozoen auf DNA-Fragmentierung;

wobei das Vorhandensein von Spermatozoen mit intakter Membran, die einen DNA-Fragmentierungsindex (%DFI) von höchstens etwa 5 % in der Probe aufweisen, vorhersagt, dass das Subjekt Spermatozoen haben wird, die funktionsfähig sein können.

**5.** Verfahren zur Bestimmung, ob nicht funktionsfähige Spermatozoen in einer Probe von einem Subjekt vorhanden sind, umfassend

(a) Untersuchen von Spermatozoen, die in der Probe enthalten sind, auf Membranintegrität; und
(b) Untersuchen der Spermien in der Probe auf DNA-Fragmentierung;
wobei das Vorhandensein von Spermien mit nicht-intakter Membran, die einen DNA-Fragmentierungsindex (% DFI) von höchstens etwa 5 % in der Probe aufweisen, darauf hindeutet, dass das Subjekt Spermien haben wird, die möglicherweise nicht funktionsfähig sind,
wobei das Vorhandensein von Spermatozoen mit nicht-intakter Membran, die einen DNA-Fragmentierungsindex (%DFI) von mindestens etwa 1 % in der Probe aufweisen, vorhersagt, dass das Subjekt Spermatozoen haben wird, die möglicherweise nicht funktionsfähig sind, und/oder
wobei das Vorhandensein von Spermatozoen mit intakter Membran, die einen DNA-Fragmentierungsindex (% DFI) von mindestens etwa 1 % in der Probe aufweisen, vorhersagt, dass das Subjekt Spermatozoen haben wird, die möglicherweise nicht funktionsfähig sind.

**6.** Verfahren nach einem der vorhergehenden Ansprüche, wobei funktionelle und/oder nicht-funktionelle Spermatozoen einem oder mehreren Lebensfähigkeitstests unterzogen werden, und/oder

wobei die Membranintegrität durch einen Farbstoff untersucht wird, der zwischen lebenden und toten Zellen unterscheidet, und/oder
wobei DNA-Schäden durch einen Spermien-DNA-Fragmentierungstest untersucht werden, und/oder
wobei die Durchflusszytometrie zur Untersuchung der Probe auf Membranintegrität und/oder zur Untersuchung der Probe auf Spermien-DNA-Fragmentierung verwendet wird, und/oder
wobei die Untersuchung einer Spermatozoen enthaltenden Probe auf Membranintegrität die Bestimmung der Anzahl von Spermatozoen, die keine Membranintegrität aufweisen, und/oder die Bestimmung der Anzahl von Spermatozoen, die Membranintegrität aufweisen, umfasst, und/oder

wobei die Untersuchung einer Spermien enthaltenden Probe auf Spermien-DNA-Fragmentierung die Bestimmung der Anzahl von Spermien mit fragmentierter DNA und/oder die Bestimmung der Anzahl von Spermien ohne fragmentierte DNA umfasst,
und/oder wobei die Anzahl von Spermien mit intakter Membran und fragmentierter DNA ins Verhältnis zur Gesamtzahl von Spermien mit intakter Membran gesetzt wird, wodurch das DNA- und Membranintegritätsverhältnis (DMI) angegeben wird:

DMI-Verhältnis = (membranintakte DNA-fragmentierte Spermatozoen) / (gesamte membranintakte Spermatozoen), und/oder
wobei funktionelle Spermatozoen potenziell fruchtbare Spermatozoen sind, und/oder wobei nicht-funktionelle Spermatozoen potenziell unfruchtbare Spermatozoen sind.

**7.** Verfahren nach einem der vorhergehenden Ansprüche, wobei das genannte Stratifizieren oder Bestimmen die folgenden Schritte umfasst:

1) Untersuchen der Spermatozoen auf Membranintegrität (a) und DNA-Fragmentierung (b);
2) Sortieren der Spermatozoen gemäß dem für (a) und (b) verwendeten Farbstoff;
3) Auswählen der Mehrheit der Spermatozoen gemäß ihrem FSC-A- und SSC-A-Signal;
4) Analysieren der Fluoreszenzwerte in einem Histogramm, das (a) für einzelne Spermatozoen darstellt;
5) Analyse der Fluoreszenzwerte in einem Histogramm, das (b) für einzelne Spermatozoen darstellt;
5)i) Berechnung des %DFI aus dem Histogramm, das (b) für einzelne Spermatozoen darstellt;
6) Kombination der Fluoreszenzwerte von (a) und (b), die in den Schritten 4) und 5) pro Probe erhalten wurden, in einem bivariaten logarithmischen Dichteplot der Fluoreszenzintensität; und
6)i) Festlegung eines Fluoreszenzschwellenwerts für Spermien mit intakter und nicht-intakter Membran und eines Fluoreszenzschwellenwerts für Spermien mit und ohne DNA-Fragmentierung unter Verwendung von Spermien einer Kalibrierungsprobe oder Kalibrierungskügelchen zur Stratifizierung oder Bestimmung der Spermien gemäß den Schwellenwerten.

**8.** Verfahren nach Anspruch 7, wobei die Berechnung des Prozentsatzes des DNA-Fragmentierungsindex (%DFI) pro Probe durch Bestimmung der Anzahl von Spermatozoen mit mäßiger und hoher DNA-Fragmentierung im Verhältnis zur Anzahl von Spermatozoen mit geringer DNA-Fragmentierung aus den Fluoreszenzwerten in dem Histogramm, das (b) der einzelnen Spermatozoen der in Schritt 5 analysierten Probe darstellt, und/oder wobei die Spermatozoen der Probe gemäß Anspruch 1 stratifiziert oder bestimmt werden.

**9.** Verfahren zum Stratifizieren oder Bestimmen von Spermatozoen, die in einer Probe von einem Subjekt enthalten sind, wobei das Verfahren die folgenden Schritte umfasst:

1) Untersuchen der Spermatozoen auf Membranintegrität (a) und DNA-Fragmentierung (b);
2) Sortieren der Spermatozoen gemäß dem für (a) und (b) verwendeten Farbstoff;
3) Auswählen der Mehrheit der Spermatozoen gemäß ihrem FSC-A- und SSC-A-Signal;
4) Analyse der Fluoreszenzwerte in einem Histogramm, das (a) für einzelne Spermien darstellt;
5) Analyse der Fluoreszenzwerte in einem Histogramm, das (b) für einzelne Spermien darstellt;
6) Kombination der Fluoreszenzwerte von (a) und (b), die in den Schritten 4) und 5) pro Probe in einem bivariaten logarithmischen Dichteplot der Fluoreszenzintensität erhalten wurden; und
6)i) Festlegung eines Fluoreszenzschwellenwerts für Spermatozoen mit intakter und nicht-intakter Membran und eines Fluoreszenzschwellenwerts für Spermatozoen mit und ohne DNA-Fragmentierung unter Verwendung von Spermatozoen einer Kalibrierungsprobe oder Kalibrierungskügelchen zur Stratifizierung oder Bestimmung der Spermatozoen gemäß den Schwellenwerten.

**10.** Verfahren nach einem der Ansprüche 7, 8 oder 9, wobei die Spermatozoen dadurch wie folgt stratifiziert oder bestimmt werden:

I. Membran-nicht-intakte Spermatozoen mit nicht-fragmentierter DNA mit einer Spermatozoenanzahl zwischen etwa 20 % und etwa 60 % der gesamten Spermatozoenanzahl, bevorzugt etwa 35 % der gesamten Spermatozoenanzahl, gemessen pro Probe;
II. Spermatozoen mit nicht-intakter Membran und fragmentierter DNA, mit einer SpermatozoenAnzahl von mindestens etwa 1 % der Gesamtzahl der Spermatozoen, gemessenen pro Probe;
III. Spermatozoen mit intakter Membran und nicht fragmentierter DNA, mit einer Spermatozoenanzahl zwischen etwa 45 % und etwa 75 % der Gesamtzahl der Spermatozoen, bevorzugt etwa 55 % der Gesamtzahl der Spermatozoen, gemessen pro Probe; und
IV. Membran-intakte Spermatozoen mit fragmentierter DNA, mit einer Spermatozoenanzahl von mindestens etwa 1 % der gesamten Spermatozoenanzahl, gemessen pro Probe, wobei bevorzugt die Spermatozoenanzahlen von I bis IV die Berechnung des DNA- und Membranintegritätsverhältnisses (DMI) gemäß

wobei die Anzahl der Spermatozoen mit intakter Membran und fragmentierter DNA ins Verhältnis zur Gesamtzahl der Spermatozoen mit intakter Membran gesetzt wird, wodurch das Verhältnis von DNA- und Membranintegrität (DMI) angegeben wird:

DMI-Verhältnis = (Spermatozoen mit intakter Membran und fragmentierter DNA) / (Gesamtzahl der Spermatozoen mit intakter Membran).

**11.** Verfahren nach einem der vorhergehenden Ansprüche, wobei ein DNA-interkalierender Farbstoff zur Bewertung der DNA-Fragmentierung angewendet wird, und/oder

wobei der zur Bewertung der DNA-Fragmentierung angewendete DNA-interkalierende Farbstoff ein Fluoreszenzfarbstoff ist, und/oder
wobei der zur Bewertung der DNA-Fragmentierung angewendete fluoreszierende DNA-interkalierende Farbstoff Acridinorange (AO) ist, und/oder
wobei der Farbstoff, der lebende und tote Zellen unterscheidet, mit Aminen reagiert, und/oder
wobei der Farbstoff, der lebende und tote Zellen unterscheidet und mit Aminen reagiert, ein Fluoreszenzfarbstoff ist, und/oder
wobei der Farbstoff zum Untersuchen der DNA-Fragmentierung und der Farbstoff zum Beurteilen der Membranintegrität ein unterschiedliches Emissionsspektrum aufweisen, wodurch eine spektrale Überlappung vermieden wird.

**12.** Verfahren zur Stratifizierung von Spermatozoen, die in einer Probe von einem Subjekt enthalten sind, wobei das Verfahren umfasst:

(a) Untersuchen der in der Probe enthaltenen Spermatozoen auf Membranintegrität;
(b) Untersuchen der in der Probe enthaltenen Spermatozoen auf DNA-Fragmentierung; und wobei die Spermatozoen als
membran-nicht-intakte Spermatozoen mit überwiegend doppelsträngiger DNA;
Spermatozoen mit nicht-intakter Membran und überwiegend einzelsträngiger DNA oder RNA,
Spermatozoen mit intakter Membran und überwiegend doppelsträngiger DNA; und
Spermatozoen mit intakter Membran und überwiegend einzelsträngiger DNA oder RNA stratifiziert werden.

**13.** Verfahren nach einem der vorhergehenden Ansprüche, wobei ein DNA-interkalierender Farbstoff zur Bewertung von einzelsträngiger und doppelsträngiger DNA angewendet wird, und/oder

wobei der zur Bewertung von einzelsträngiger und doppelsträngiger DNA angewendete DNA-interkalierende Farbstoff ein Fluoreszenzfarbstoff ist, und/oder
wobei der Farbstoff, der zwischen intakten und nicht-intakten Spermatozoenmembranen unterscheidet, mit Aminen reagiert, und/oder
wobei der Farbstoff, der zwischen intakten und nicht-intakten Spermatozoenmembranen unterscheidet und mit Aminen reagiert, ein Fluoreszenzfarbstoff ist, und/oder
wobei der Farbstoff zur Bewertung von einzelsträngiger und doppelsträngiger DNA und der Farbstoff, der zwischen intakten und nicht-intakten Spermatozoenmembranen unterscheidet und mit Aminen reagiert, ein unterschiedliches Emissionsspektrum aufweisen, wodurch eine spektrale Überlappung vermieden wird, und/oder
wobei Schritt (a) und Schritt (b) gleichzeitig oder nacheinander unabhängig von der Reihenfolge durchgeführt werden, wobei Schritt (a) bevorzugt vor Schritt (b) durchgeführt wird, und/oder
wobei das Subjekt ein Säugetier, ein Vogel oder ein Fisch ist.

## Revendications

1. Méthode de stratification des spermatozoïdes contenus dans un échantillon obtenu à partir d'un sujet, comprenant

   (a) l'analyse des spermatozoïdes compris dans ledit échantillon pour l'intégrité de la membrane; et
   (b) l'analyse des spermatozoïdes compris dans ledit échantillon pour la fragmentation de l'ADN,
   dans lequel la stratification permet de prédire si ledit sujet aura des spermatozoïdes susceptibles d'être fonctionnels, les spermatozoïdes étant stratifiés comme suit:

   (i) spermatozoïdes non intacts au niveau de la membrane, ayant un indice de fragmentation de l'ADN (%DFI) d'au plus 5 %;
   (ii) spermatozoïdes non intacts à la membrane, dont l'indice de fragmentation de l'ADN (%DFI) est d'au moins 1 % environ;
   (iii) spermatozoïdes intacts à la membrane, présentant un indice de fragmentation de l'ADN (%DFI) d'au plus 5 % environ;
   (iv) spermatozoïdes intacts à la membrane, présentant un indice de fragmentation de l'ADN (%DFI) d'au moins 1 % environ;

   dans lequel le pourcentage de l'indice de fragmentation de l'ADN (%DFI) dudit échantillon est compris entre environ 4 %DFI et environ 75 %DFI, dans lequel la présence de spermatozoïdes conformément à (iii) indique que le sujet aura des spermatozoïdes qui peuvent être fonctionnels, et dans lequel la présence de spermatozoïdes conformément à (i), (ii) et/ou (iv) prédit que le sujet aura des spermatozoïdes qui peuvent ne pas être fonctionnels.

2. La méthode de la revendication 1, dans laquelle un échantillon de sperme dont le %DFI est compris entre environ 4 % DFI et environ 30 %DFI contient principalement des spermatozoïdes qui peuvent être fonctionnels, ou dans laquelle un échantillon de sperme dont le %DFI est compris entre environ 30 %DFI et environ 75 %DFI contient principalement des spermatozoïdes qui peuvent ne pas être fonctionnels.

3. La méthode de l'une des revendications précédentes, dans laquelle, si le sujet a principalement des spermatozoïdes de la catégorie (i), (ii) et/ou (iv), ces spermatozoïdes doivent être utilisés pour la FIV ou l'ICSI.

4. Méthode permettant de déterminer si des spermatozoïdes fonctionnels sont présents dans un échantillon obtenu à partir d'un sujet, comprenant

   (a) l'analyse des spermatozoïdes compris dans ledit échantillon pour déterminer l'intégrité de la membrane; et
   (b) l'analyse de la fragmentation de l'ADN des spermatozoïdes compris dans ledit échantillon;

   dans lequel la présence de spermatozoïdes à membrane intacte ayant un indice de fragmentation de l'ADN (%DFI) d'au plus 5 % dans ledit échantillon permet de prédire que le sujet aura des spermatozoïdes qui peuvent être fonctionnels.

5. Méthode permettant de déterminer si des spermatozoïdes non fonctionnels sont présents dans un échantillon provenant d'un sujet, comprenant

   (a) l'analyse des spermatozoïdes compris dans ledit échantillon pour déterminer l'intégrité de la membrane; et
   (b) l'analyse des spermatozoïdes compris dans ledit échantillon pour la fragmentation de l'ADN;
   dans lequel la présence de spermatozoïdes dont la membrane n'est pas intacte et dont l'indice de fragmentation de l'ADN (%DFI) est d'au plus 5 % dans ledit échantillon permet de prédire que le sujet aura des spermatozoïdes qui peuvent ne pas être fonctionnels,
   dans lequel la présence de spermatozoïdes sans membrane intacte ayant un indice de fragmentation de l'ADN (%DFI) d'au moins 1 % environ dans ledit échantillon permet de prédire que le sujet aura des spermatozoïdes qui pourraient ne pas être fonctionnels, et/ou
   dans lequel la présence de spermatozoïdes à membrane intacte ayant un indice de fragmentation de l'ADN (% DFI) d'au moins 1 % environ dans ledit échantillon permet de prédire que le sujet aura des spermatozoïdes qui peuvent ne pas être fonctionnels.

6. La méthode de l'une des revendications précédentes, dans laquelle les spermatozoïdes fonctionnels et/ou non fonctionnels sont soumis à un ou plusieurs tests de viabilité, et/ou

dans lequel l'intégrité de la membrane est évaluée par un colorant différenciant les cellules vivantes des cellules mortes, et/ou
dans lequel les dommages à l'ADN sont évalués par un test de fragmentation de l'ADN des spermatozoïdes, et/ou la cytométrie en flux est utilisée pour évaluer l'intégrité de la membrane et/ou la fragmentation de l'ADN des spermatozoïdes de l'échantillon, et/ou
dans lequel l'analyse d'un échantillon comprenant des spermatozoïdes pour l'intégrité de la membrane comprend la détermination du nombre de spermatozoïdes ne présentant pas d'intégrité de la membrane et/ou la détermination du nombre de spermatozoïdes présentant une intégrité de la membrane, et/ou
dans lequel l'analyse d'un échantillon de spermatozoïdes pour la fragmentation de l'ADN des spermatozoïdes consiste à déterminer le nombre de spermatozoïdes comprenant de l'ADN fragmenté et/ou à déterminer le nombre de spermatozoïdes ne comprenant pas d'ADN fragmenté,
et/ou dans lequel le nombre de spermatozoïdes à l'ADN membranaire intact fragmenté est rapporté au nombre total de spermatozoïdes à la membrane intacte, ce qui indique le rapport d'intégrité de l'ADN et de la membrane (DMI):

Rapport DMI = (spermatozoïdes fragmentés à l'ADN intact à la membrane) / (total des spermatozoïdes à la membrane intacte), et/ou

dans lequel les spermatozoïdes fonctionnels sont des spermatozoïdes potentiellement fertiles, et/ou
les spermatozoïdes non fonctionnels étant des spermatozoïdes potentiellement infertiles.

**7.** La méthode de l'une des revendications précédentes, dans laquelle la stratification ou la détermination comprend les étapes suivantes

1) l'analyse des spermatozoïdes pour l'intégrité de la membrane (a) et la fragmentation de l'ADN (b);
2) trier les spermatozoïdes en fonction du colorant utilisé pour (a) et (b);
3) sélection de la majorité des spermatozoïdes en fonction de leur signal FSC-A et SSC-A;
4) analyse des valeurs de fluorescence dans un histogramme représentant (a) pour les spermatozoïdes individuels;
5) analyser les valeurs de fluorescence dans un histogramme représentant (b) pour les spermatozoïdes individuels;
5)i) calculer le %DFI à partir de l'histogramme représentant (b) pour les spermatozoïdes individuels;
6) combiner les valeurs de fluorescence de a) et b) obtenues aux étapes 4) et 5) par échantillon dans un diagramme de densité logarithmique bivarié de l'intensité de fluorescence; et
6)i) établir un seuil de niveau de fluorescence pour les spermatozoïdes à membrane intacte et à membrane non intacte, et un seuil de niveau de fluorescence pour les spermatozoïdes avec et sans fragmentation de l'ADN, en utilisant des spermatozoïdes d'un échantillon d'étalonnage ou des billes d'étalonnage pour stratifier ou déterminer les spermatozoïdes en fonction desdits seuils.

**8.** Méthode de la revendication 7, dans laquelle le calcul du pourcentage de l'indice de fragmentation de l'ADN (%DFI) par échantillon est établi en déterminant le nombre de spermatozoïdes présentant une fragmentation modérée et élevée de l'ADN par rapport au nombre de spermatozoïdes présentant une faible fragmentation de l'ADN à partir des valeurs de fluorescence dans l'histogramme représentant (b) des spermatozoïdes individuels de l'échantillon analysé à l'étape 5), et/ou dans laquelle les spermatozoïdes de l'échantillon sont stratifiés ou déterminés selon la revendication 1.

**9.** Méthode de stratification ou de détermination des spermatozoïdes contenus dans un échantillon provenant d'un sujet, dans laquelle la méthode comprend les étapes suivantes

1) analyser les spermatozoïdes pour l'intégrité de la membrane (a) et la fragmentation de l'ADN (b) ;
2) trier les spermatozoïdes en fonction du colorant utilisé pour (a) et (b);
3) sélection de la majorité des spermatozoïdes en fonction de leur signal FSC-A et SSC-A;
4) analyse des valeurs de fluorescence dans un histogramme représentant (a) pour les spermatozoïdes individuels;
5) analyser les valeurs de fluorescence dans un histogramme représentant (b) pour les spermatozoïdes individuels;

6) combiner les valeurs de fluorescence de a) et b) obtenues aux étapes 4) et 5) par échantillon dans un diagramme de densité logarithmique bivarié de l'intensité de fluorescence; et
6)i) établir un seuil de niveau de fluorescence pour les spermatozoïdes à membrane intacte et à membrane non intacte, et un seuil de niveau de fluorescence pour les spermatozoïdes avec et sans fragmentation de l'ADN, en utilisant des spermatozoïdes d'un échantillon d'étalonnage ou des billes d'étalonnage pour stratifier ou déterminer les spermatozoïdes en fonction desdits seuils.

**10.** La méthode de l'une des revendications 7, 8 ou 9, dans laquelle les spermatozoïdes sont stratifiés ou déterminés comme suit :

I. spermatozoïdes non intacts sur la membrane, ayant un ADN non fragmenté, avec un nombre de spermatozoïdes compris entre environ 20 % et environ 60 % du nombre total de spermatozoïdes, de préférence environ 35 % du nombre total de spermatozoïdes mesuré par échantillon;
II. spermatozoïdes non intacts sur la membrane, dont l'ADN est fragmenté et dont le nombre de spermatozoïdes représente au moins 1 % environ du nombre total de spermatozoïdes mesuré par échantillon;
III. spermatozoïdes intacts sur membrane, dont l'ADN n'est pas fragmenté, dont le nombre de spermatozoïdes est compris entre environ 45 % et environ 75 % du nombre total de spermatozoïdes, de préférence environ 55 % du nombre total de spermatozoïdes mesuré par échantillon; et
IV. spermatozoïdes à membrane intacte ayant un ADN fragmenté, avec un nombre de spermatozoïdes d'au moins 1 % environ du nombre total de spermatozoïdes mesuré par échantillon, de préférence dans lequel les nombres de spermatozoïdes de I à IV permettent le calcul du ratio d'intégrité de l'ADN et de la membrane (DMI) selon la méthode suivante,

dans lequel le nombre de spermatozoïdes fragmentés à l'ADN membranaire intact est rapporté au nombre total de spermatozoïdes à la membrane intacte, indiquant ainsi le rapport ADN et intégrité membranaire (DMI):

Rapport DMI = (spermatozoïdes à ADN fragmenté intacts à la membrane) / (total des spermatozoïdes intacts à la membrane).

**11.** La méthode de l'une des revendications précédentes, dans laquelle un colorant d'intercalation de l'ADN est appliqué pour l'évaluation de la fragmentation de l'ADN, et/ou

dans lequel le colorant d'intercalation de l'ADN appliqué pour l'évaluation de la fragmentation de l'ADN est un colorant fluorescent, et/ou
dans lequel le colorant fluorescent d'intercalation de l'ADN, appliqué pour l'évaluation de la fragmentation de l'ADN, est l'acridine orange (AO), et/ou
dans lequel le colorant permettant de différencier les cellules vivantes des cellules mortes réagit avec les amines, et/ou
dans lequel le colorant différenciant les cellules vivantes et mortes, qui est réactif avec les amines, est un colorant fluorescent, et/ou
dans lequel le colorant permettant d'évaluer la fragmentation de l'ADN et le colorant permettant d'évaluer l'intégrité de la membrane ont un spectre d'émission différent, ce qui permet d'éviter un chevauchement spectral.

**12.** Méthode de stratification des spermatozoïdes contenus dans un échantillon provenant d'un sujet, dans laquelle la méthode consiste à

(a) l'analyse des spermatozoïdes compris dans ledit échantillon pour l'intégrité de la membrane;
(b) l'analyse de la fragmentation de l'ADN des spermatozoïdes compris dans ledit échantillon; et
dans lequel les spermatozoïdes sont stratifiés comme suit
spermatozoïdes non intacts au niveau de la membrane avec ADN double brin prévalent;
spermatozoïdes membranaires non intacts avec prédominance d'ADN ou d'ARN simple brin;
spermatozoïdes intacts à la membrane avec ADN double brin prévalent; et
spermatozoïdes intacts à la membrane avec prévalence d'ADN ou d'ARN simple brin.

**13.** La méthode de l'une des revendications précédentes, dans laquelle un colorant intercalant l'ADN est appliqué pour l'évaluation de l'ADN simple et double brin, et/ou

dans lequel le colorant d'intercalation de l'ADN appliqué pour l'évaluation de l'ADN simple et double brin est un colorant fluorescent, et/ou
dans lequel le colorant permettant de différencier les spermatozoïdes dont la membrane est intacte de ceux dont la membrane n'est pas intacte réagit avec les amines, et/ou
dans lequel le colorant permettant de différencier les spermatozoïdes à membrane intacte des spermatozoïdes à membrane non intacte, qui est réactif avec les amines, est un colorant fluorescent, et/ou
dans lequel le colorant pour l'évaluation de l'ADN simple et double brin et le colorant différenciant les spermatozoïdes à membrane intacte et à membrane non intacte, qui est réactif avec les amines, ont un spectre d'émission différent, ce qui permet d'éviter un chevauchement spectral, et/ou
l'étape (a) et l'étape (b) sont réalisées simultanément ou ultérieurement, quel que soit l'ordre, l'étape (a) étant de préférence réalisée avant l'étape (b), et/ou
le sujet est un mammifère, un oiseau ou un poisson.

Figure 1

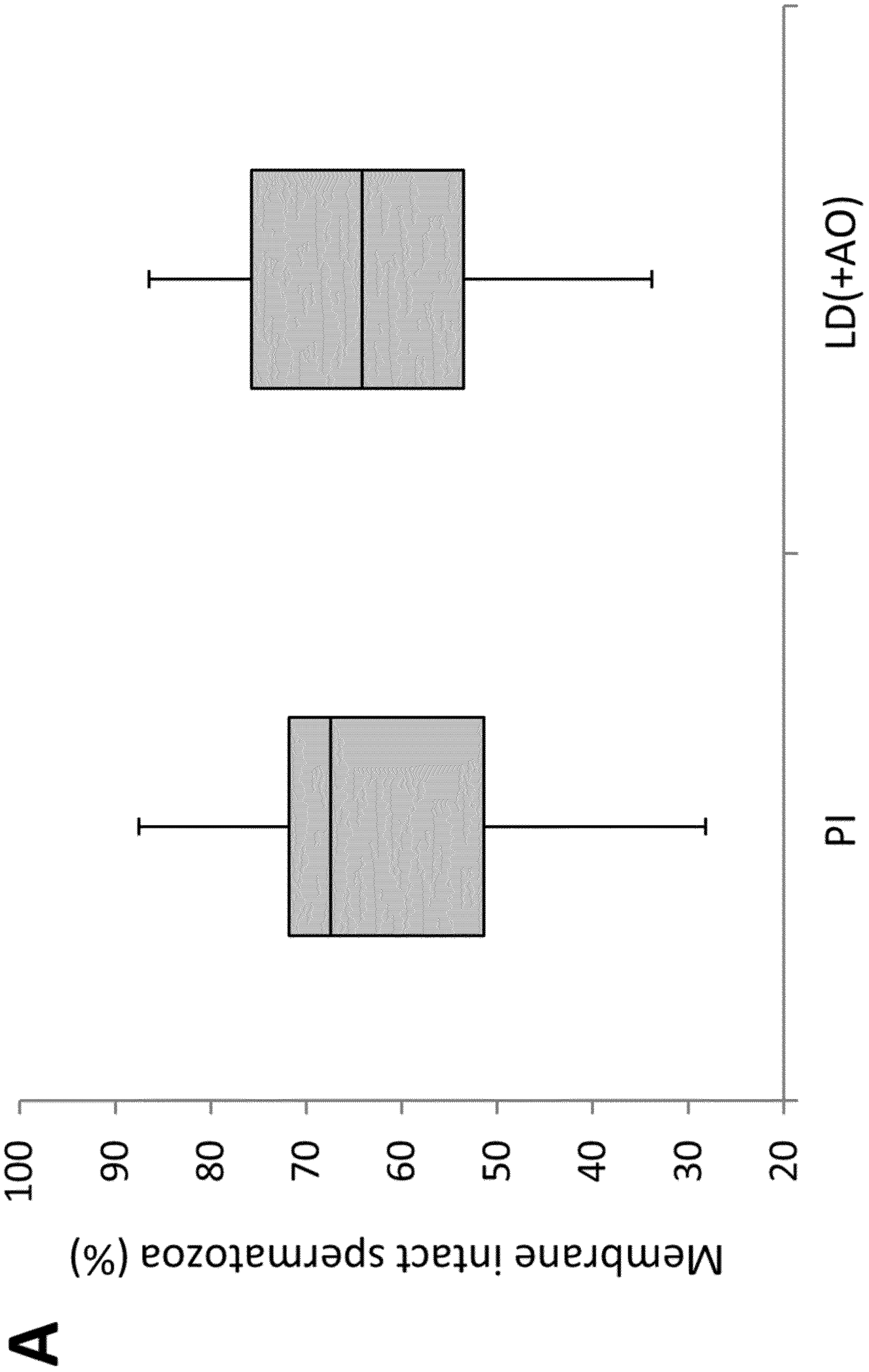
A
Membrane intact spermatozoa (%)
100
90
80
70
60
50
40
30
20
PI
LD(+AO)

Figure 1 (cont.)

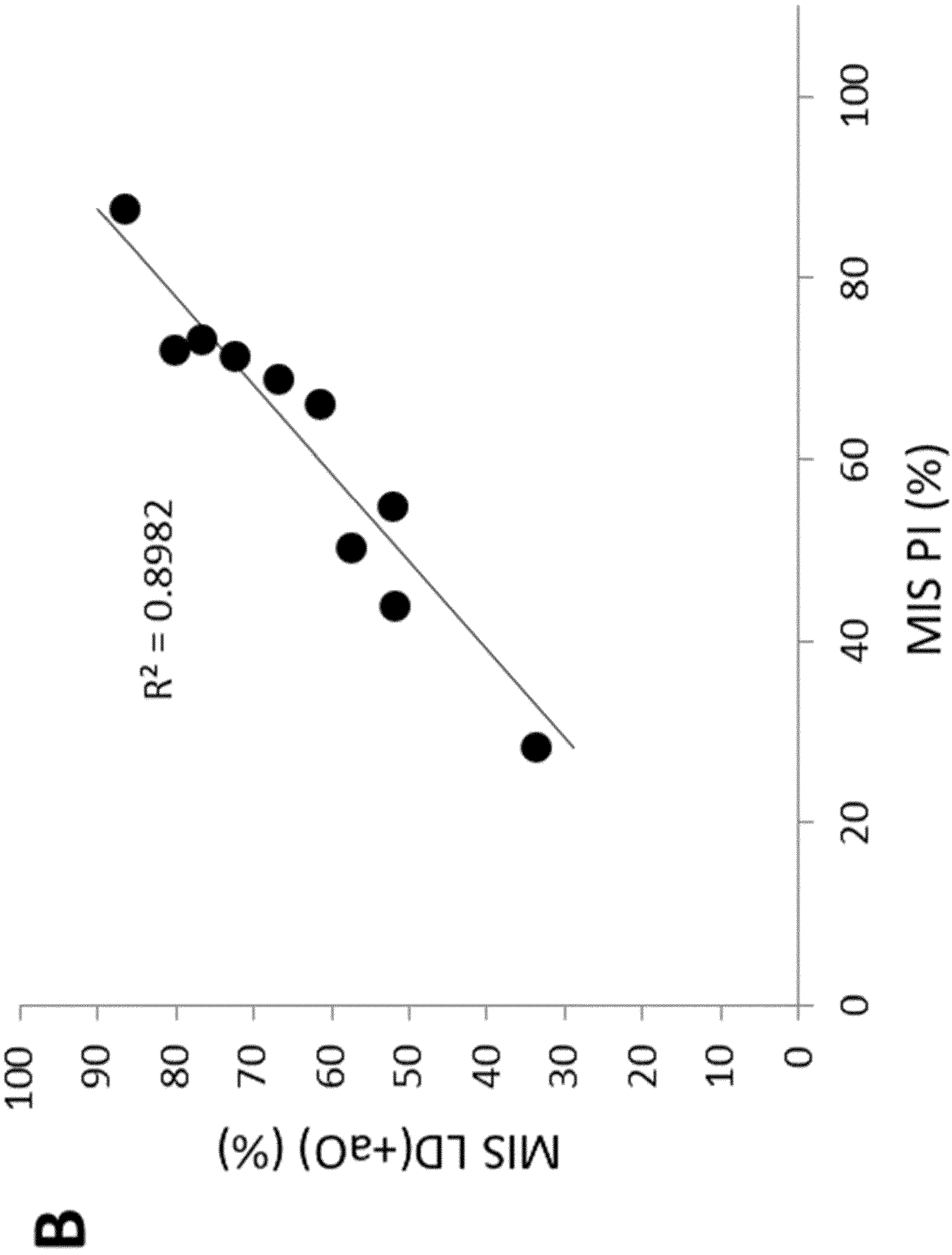

Figure 2

A

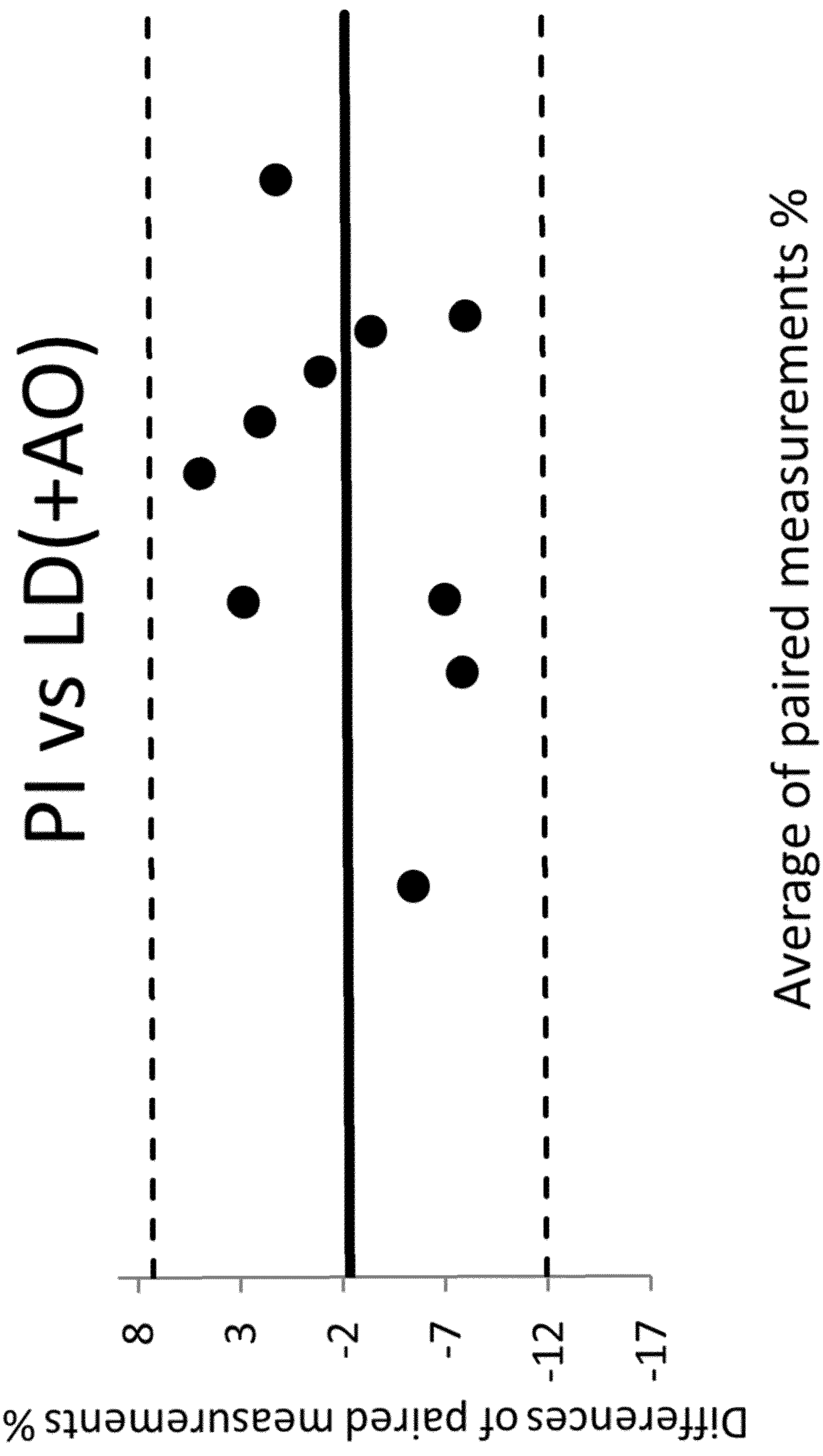
PI vs LD(+AO)
Average of paired measurements %
8
3
-2
-7
-12
-17
Differences of paired measurements %

Figure 2 (cont.)

**B**

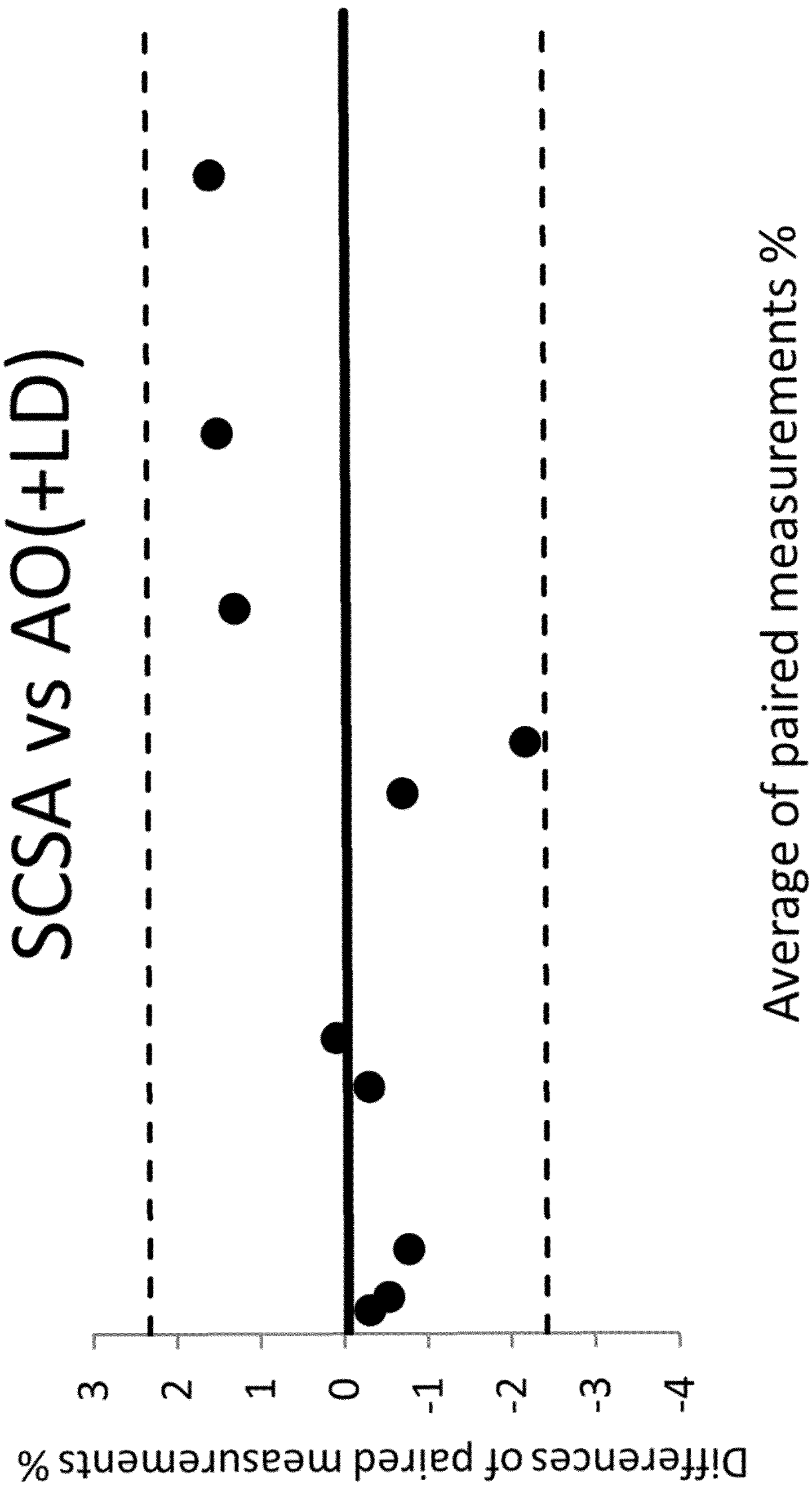

Figure 3

A

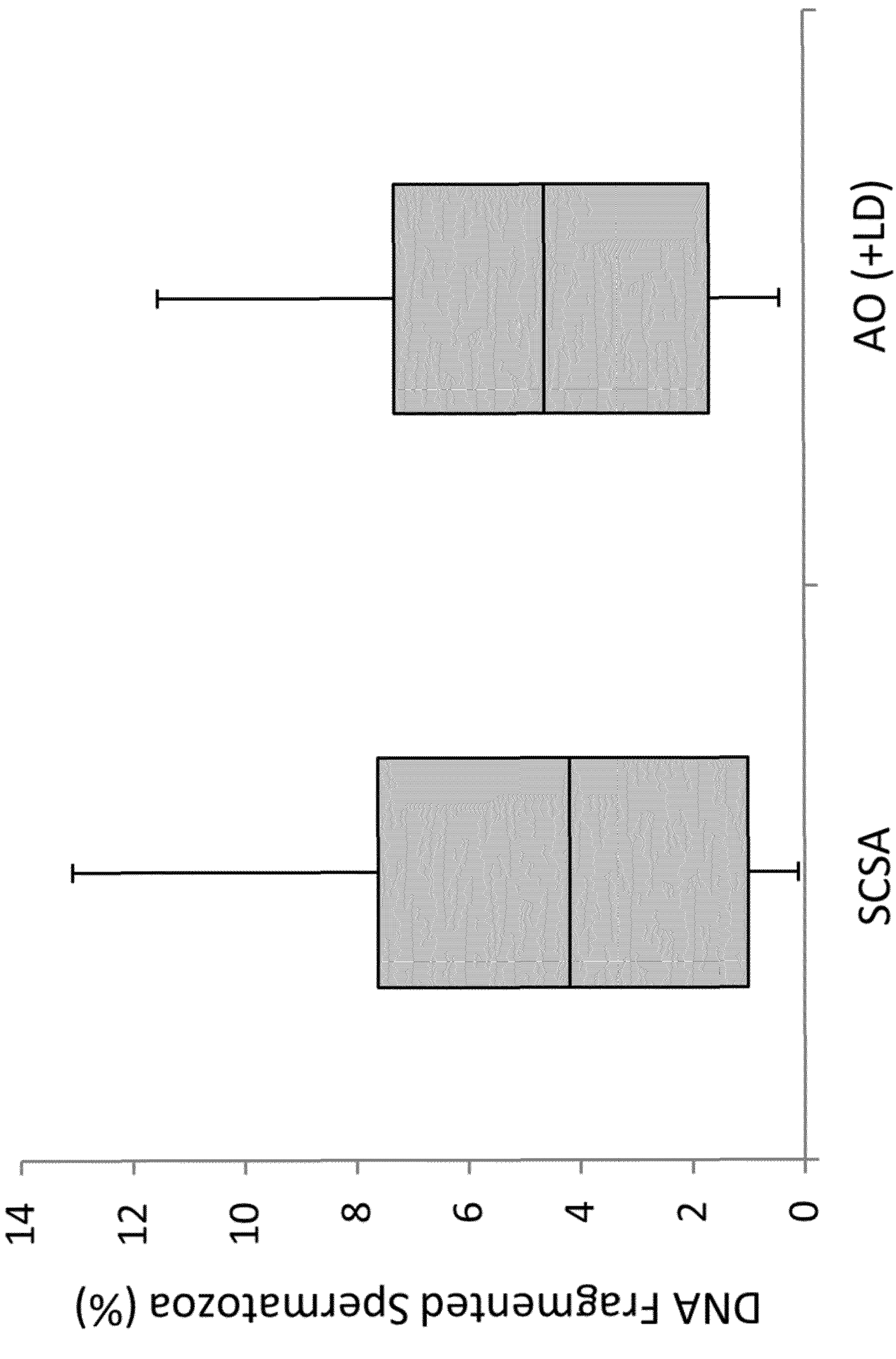

**Figure 3 (cont.)**

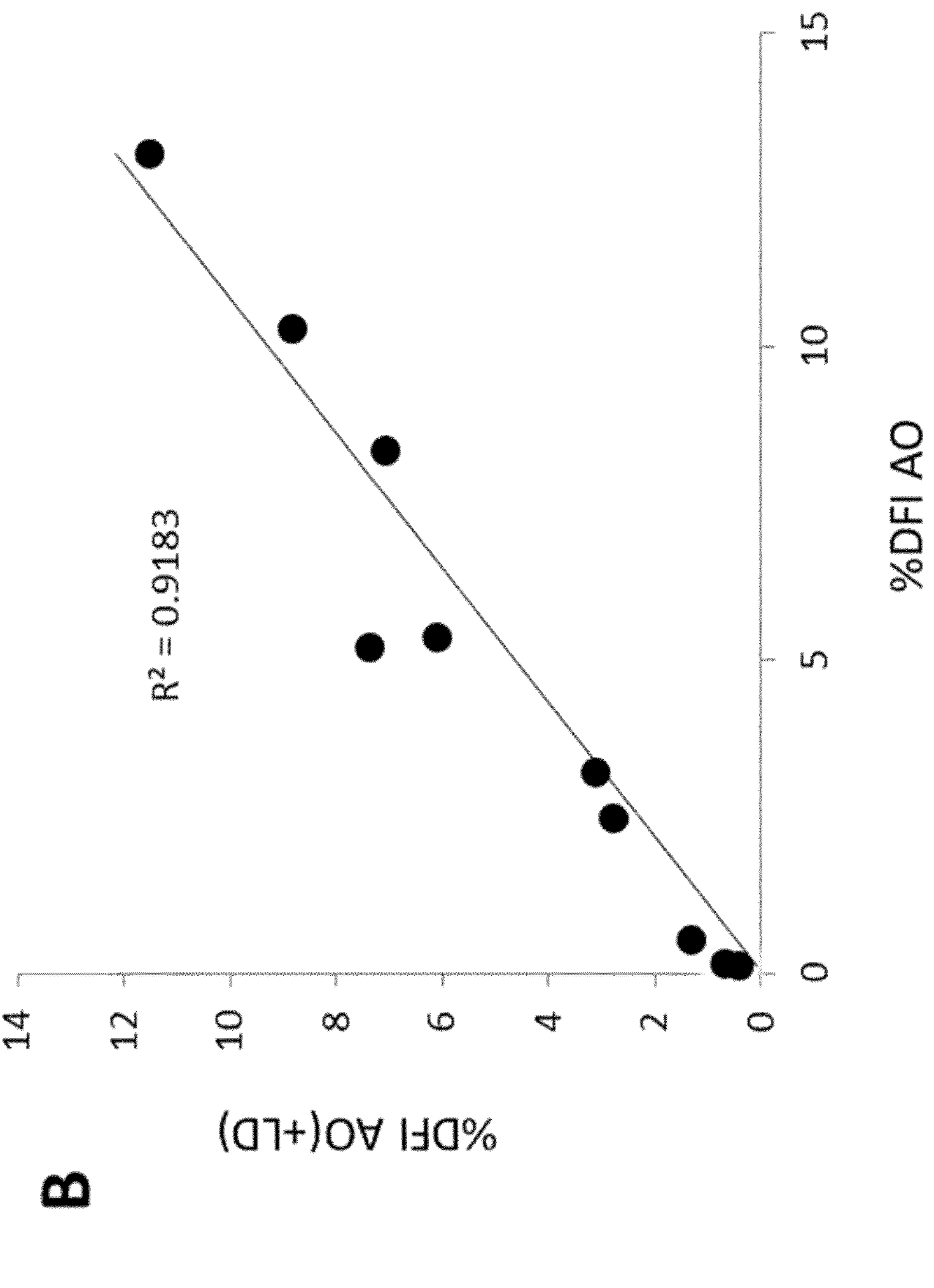

Figure 4

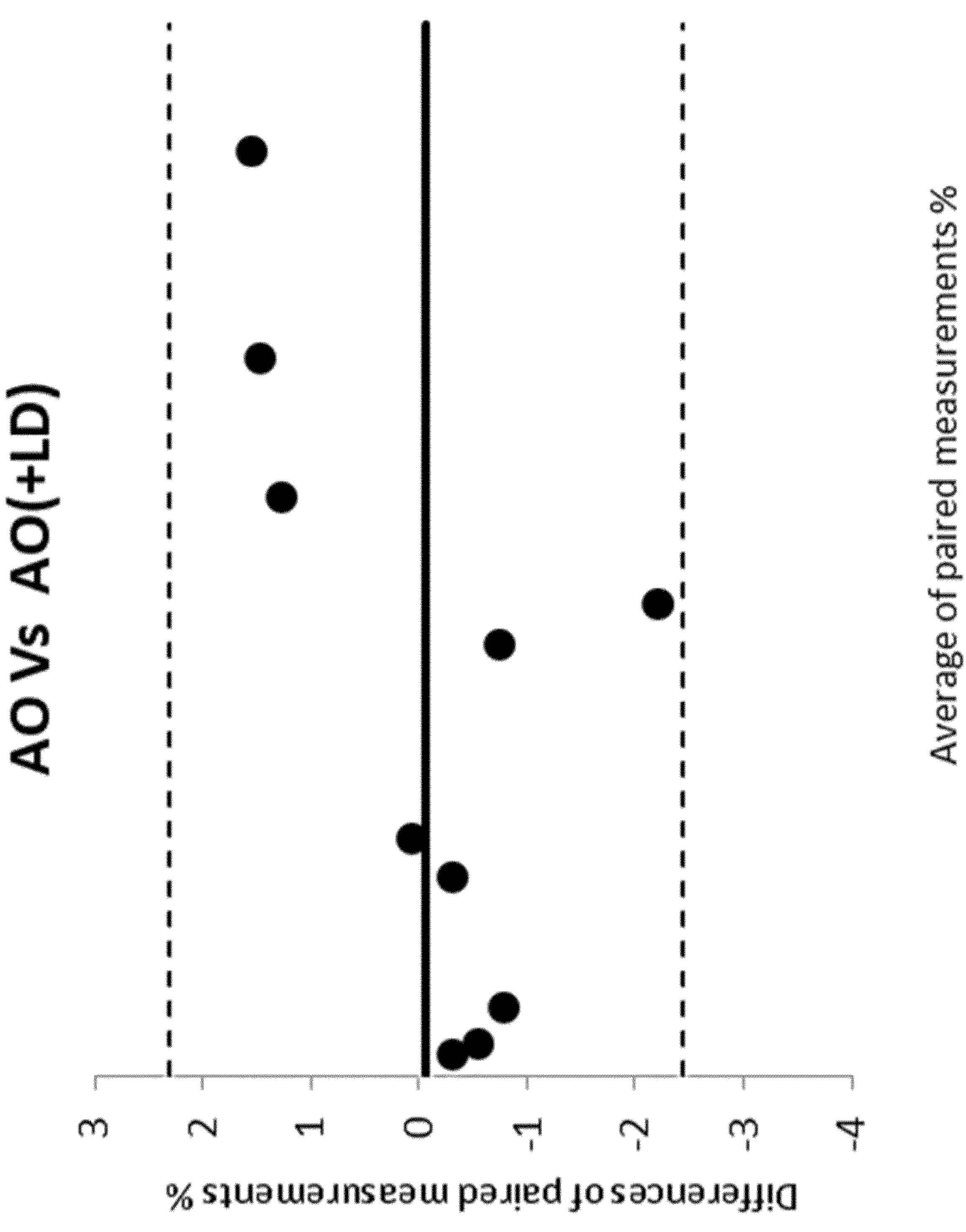
AO Vs AO(+LD)
Average of paired measurements %
Differences of paired measurements %
3
2
1
0
-1
-2
-3
-4

Figure 5

A

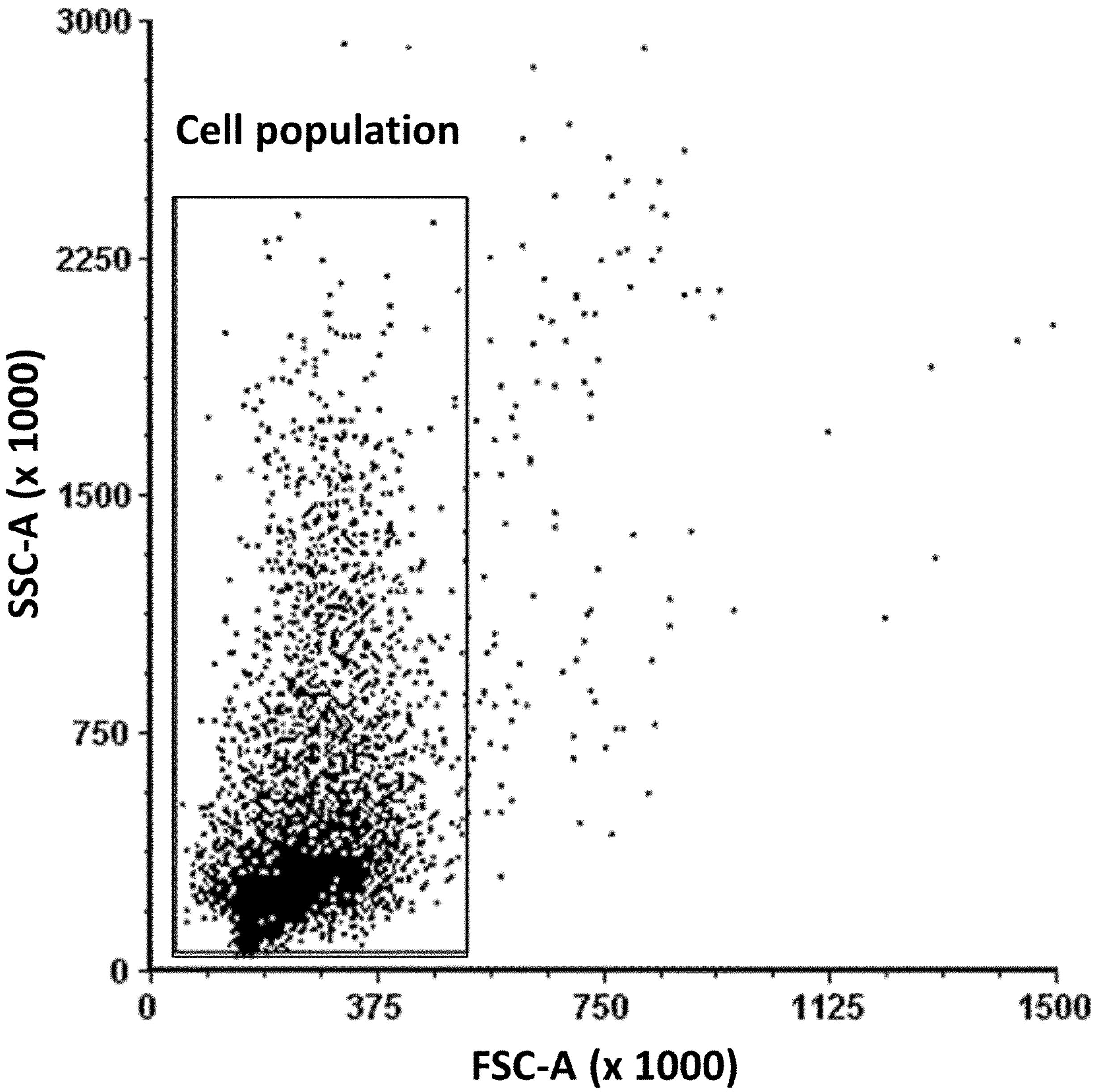
Cell population
3000
2250
1500
750
0
SSC-A (x 1000)
0
375
750
1125
1500
FSC-A (x 1000)

Figure 5 (cont.)

B

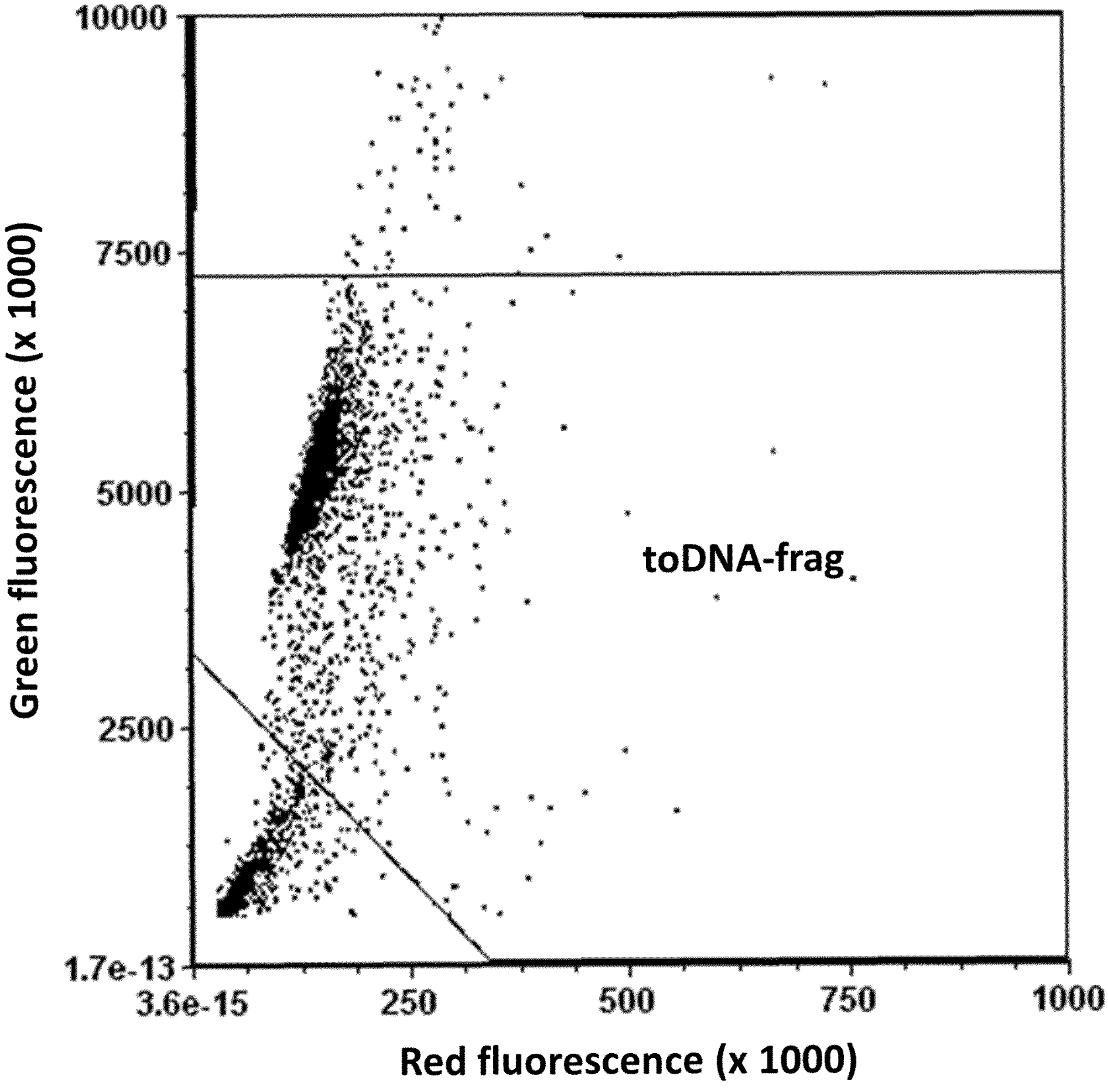

Figure 5 (cont.)

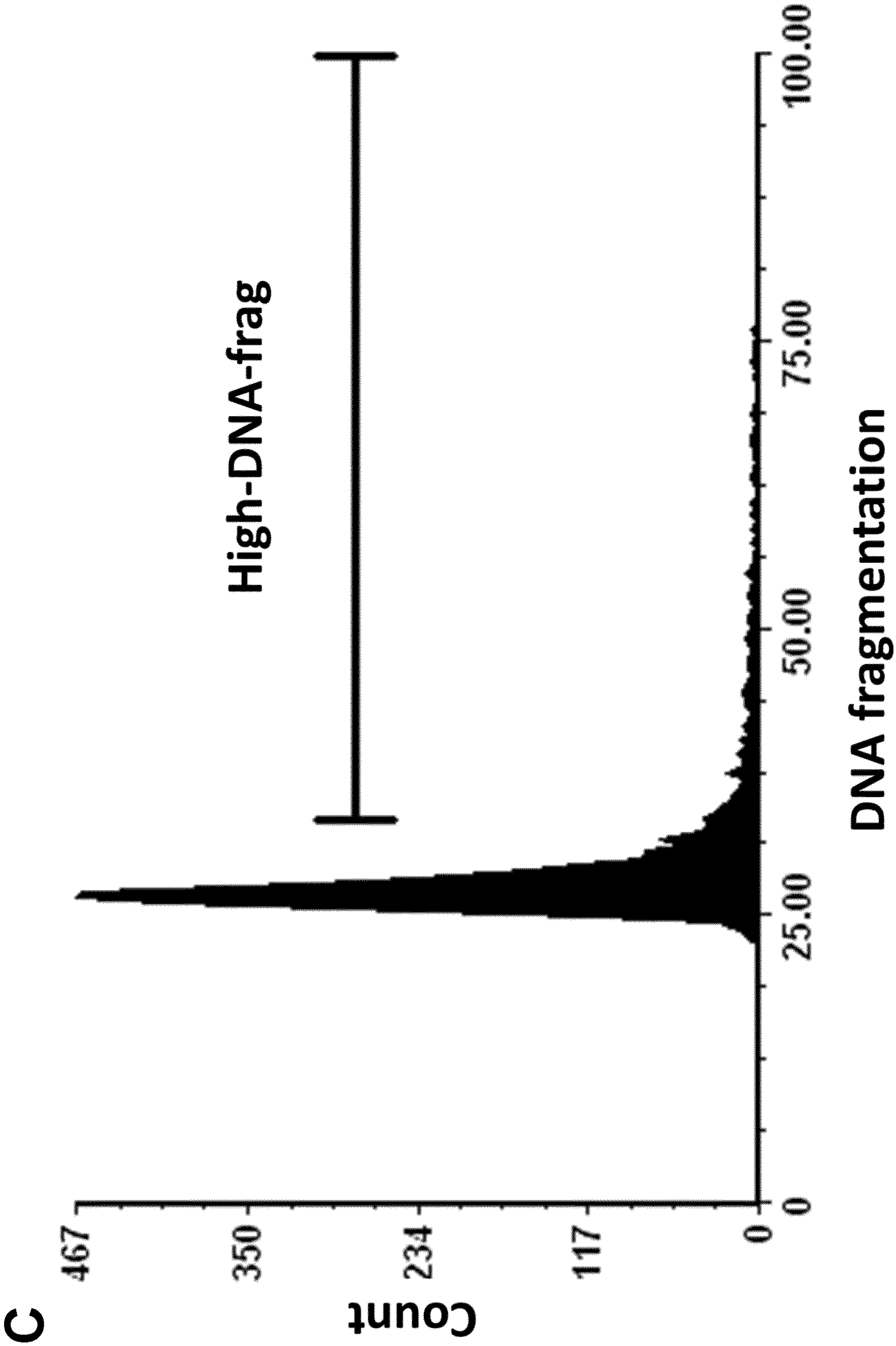

Figure 5 (cont.)

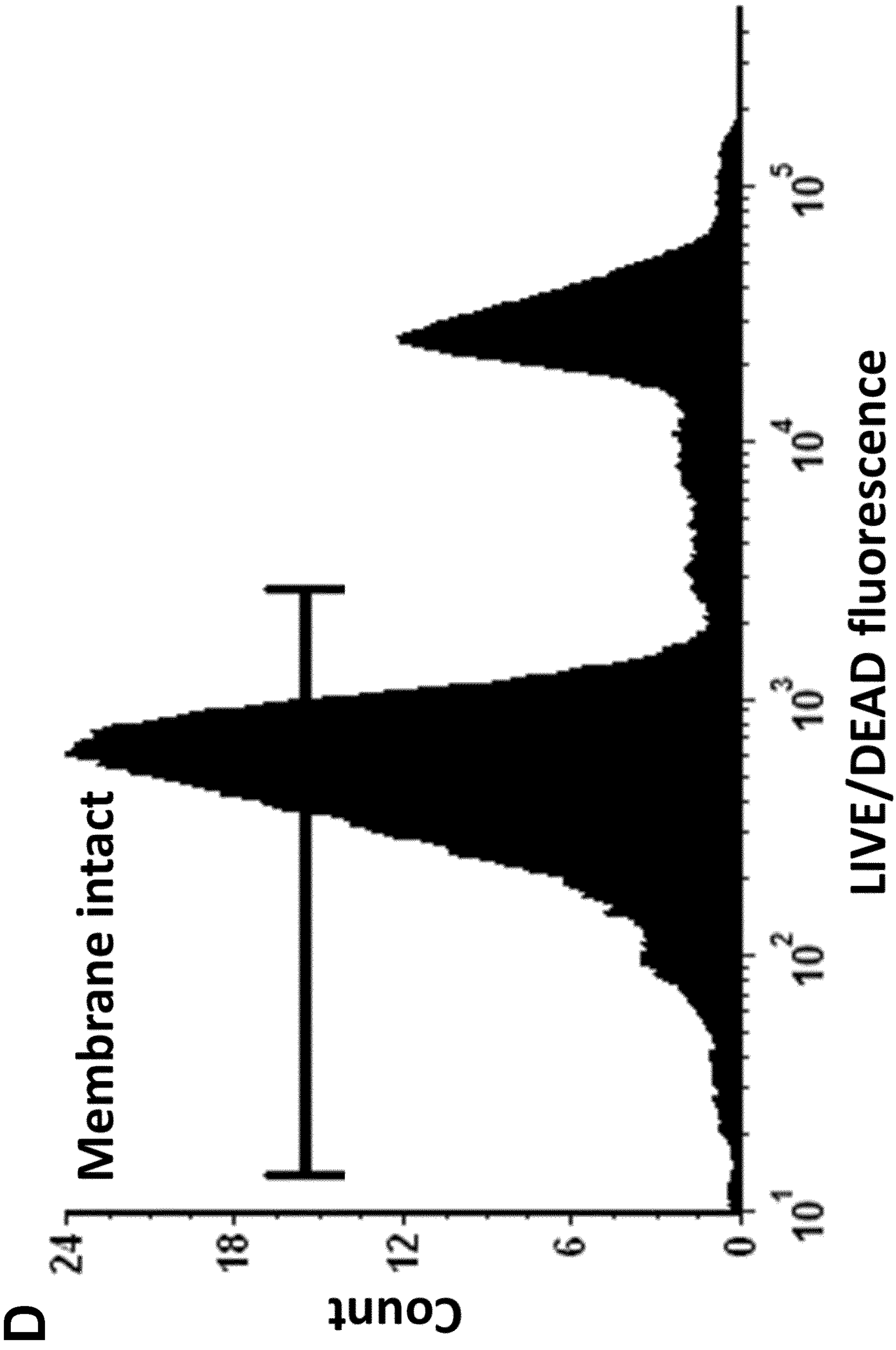

Figure 6

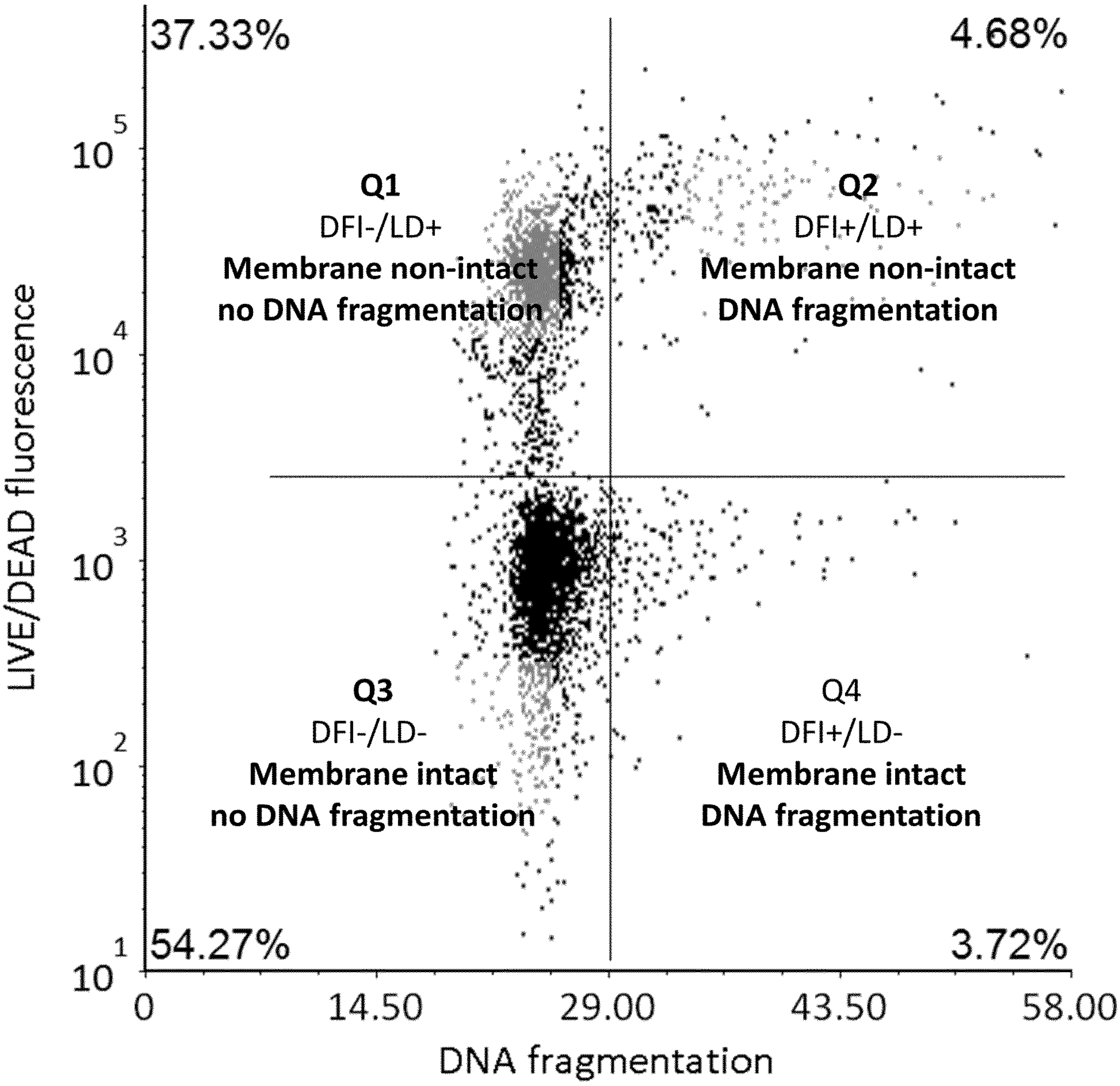
37.33%
4.68%
54.27%
3.72%
Q1
DFI-/LD+
Membrane non-intact
no DNA fragmentation
Q2
DFI+/LD+
Membrane non-intact
DNA fragmentation
Q3
DFI-/LD-
Membrane intact
no DNA fragmentation
Q4
DFI+/LD-
Membrane intact
DNA fragmentation
LIVE/DEAD fluorescence
DNA fragmentation
0
14.50
29.00
43.50
58.00

Figure 7

## DNA and Membrane Integrity Ratio

$$DMI\ Ratio = \frac{Membrane\ intact\ with\ DNA\ fragmentation\ SPZ}{Total\ membrane\ intact\ SPZ} \times 100$$

Figure 8

A

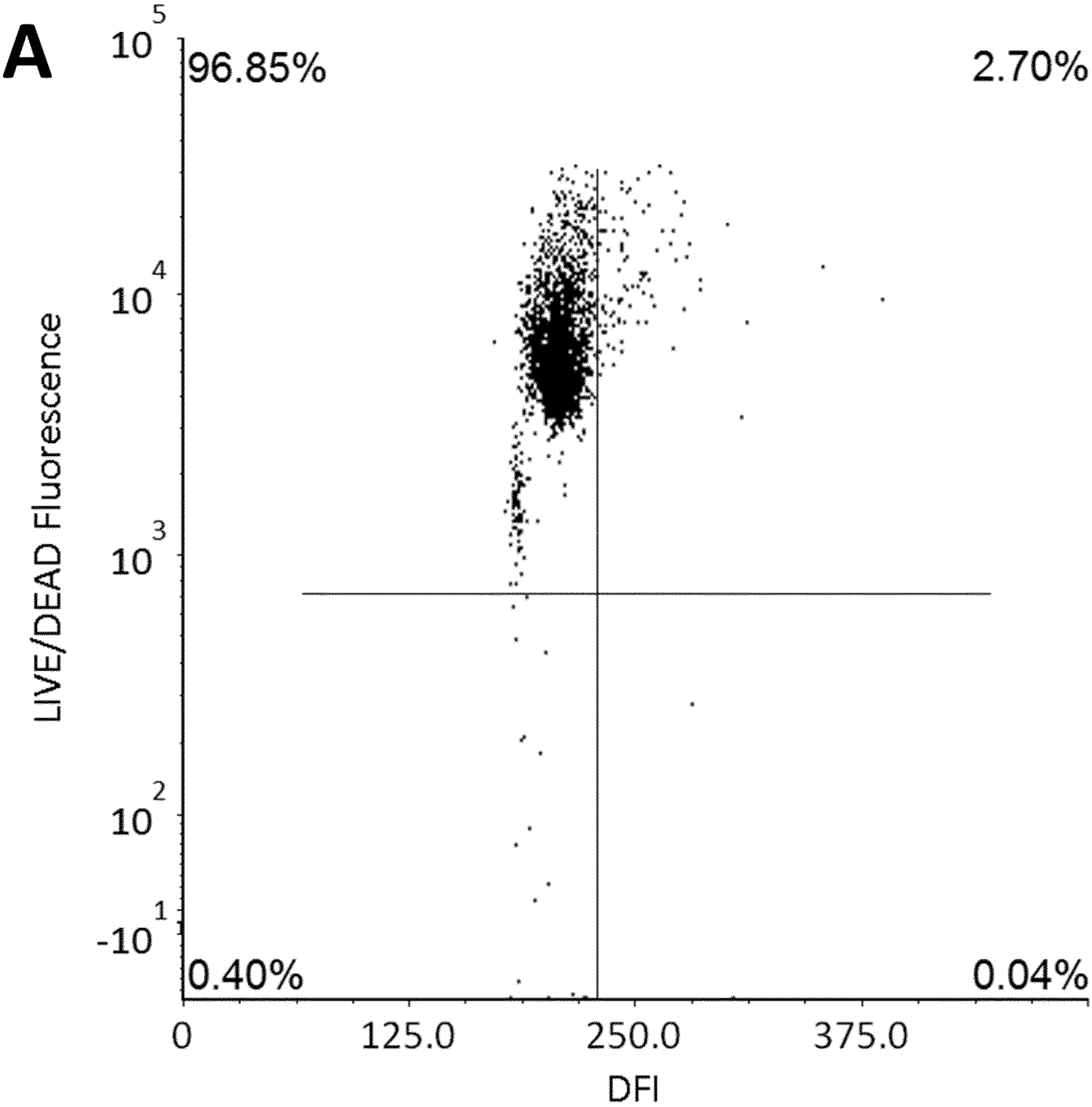
96.85%
2.70%
0.40%
0.04%
LIVE/DEAD Fluorescence
DFI
10^5
10^4
10^3
10^2
-10^1
0
125.0
250.0
375.0

Figure 8 (cont.)

B

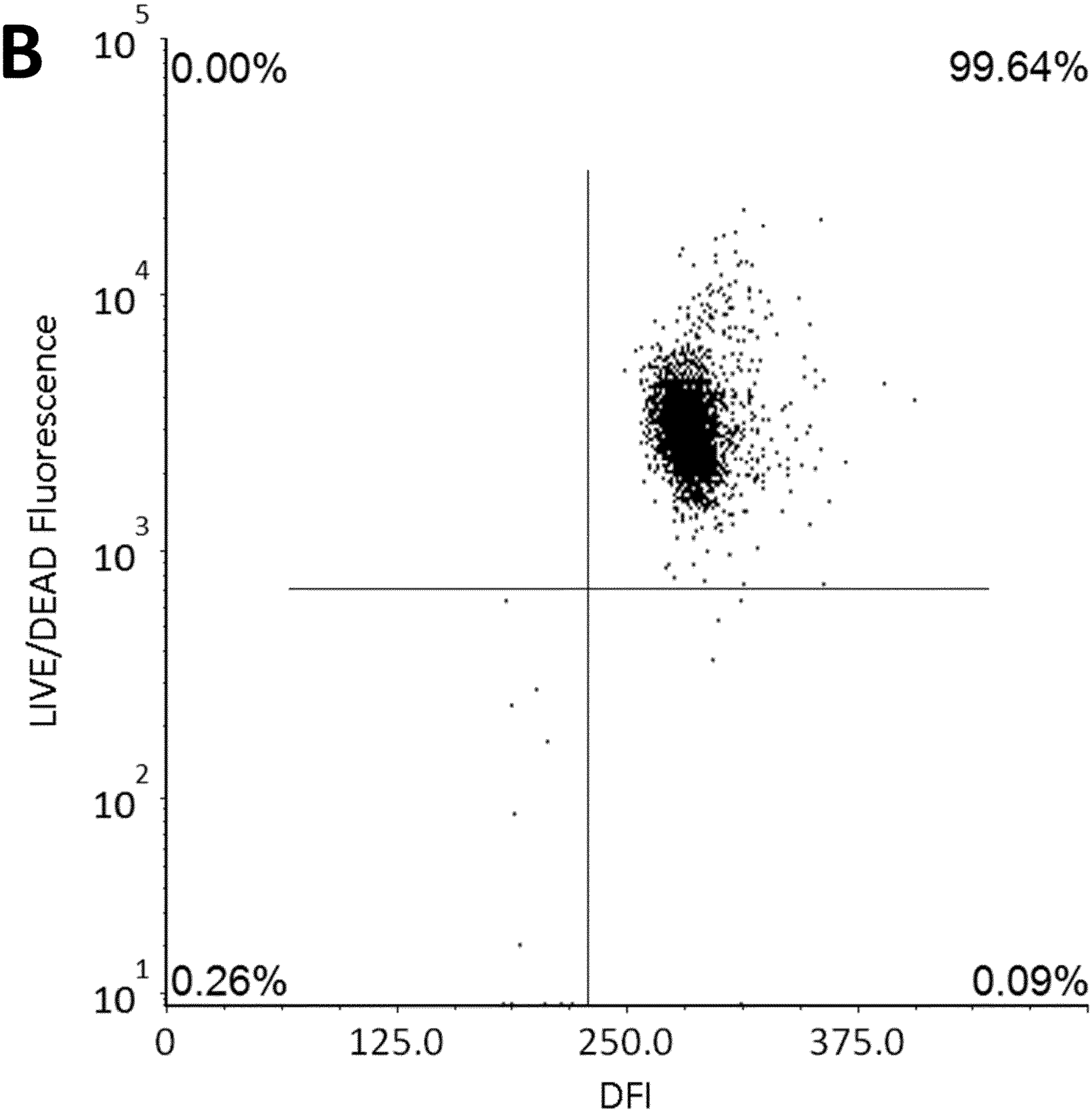

Figure 9

A

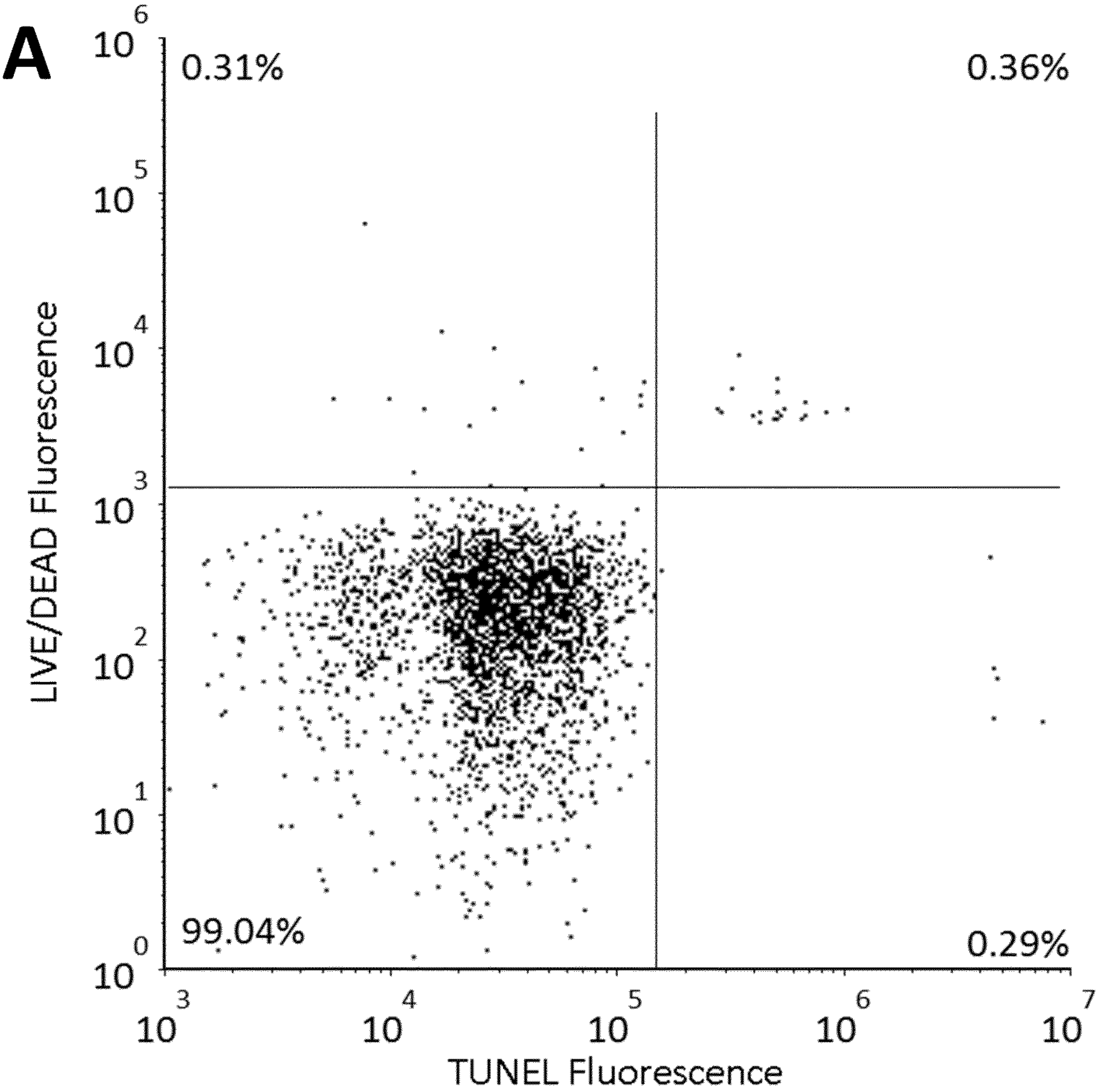

0.31%
0.36%
99.04%
0.29%
LIVE/DEAD Fluorescence
TUNEL Fluorescence

Figure 9 (cont.)

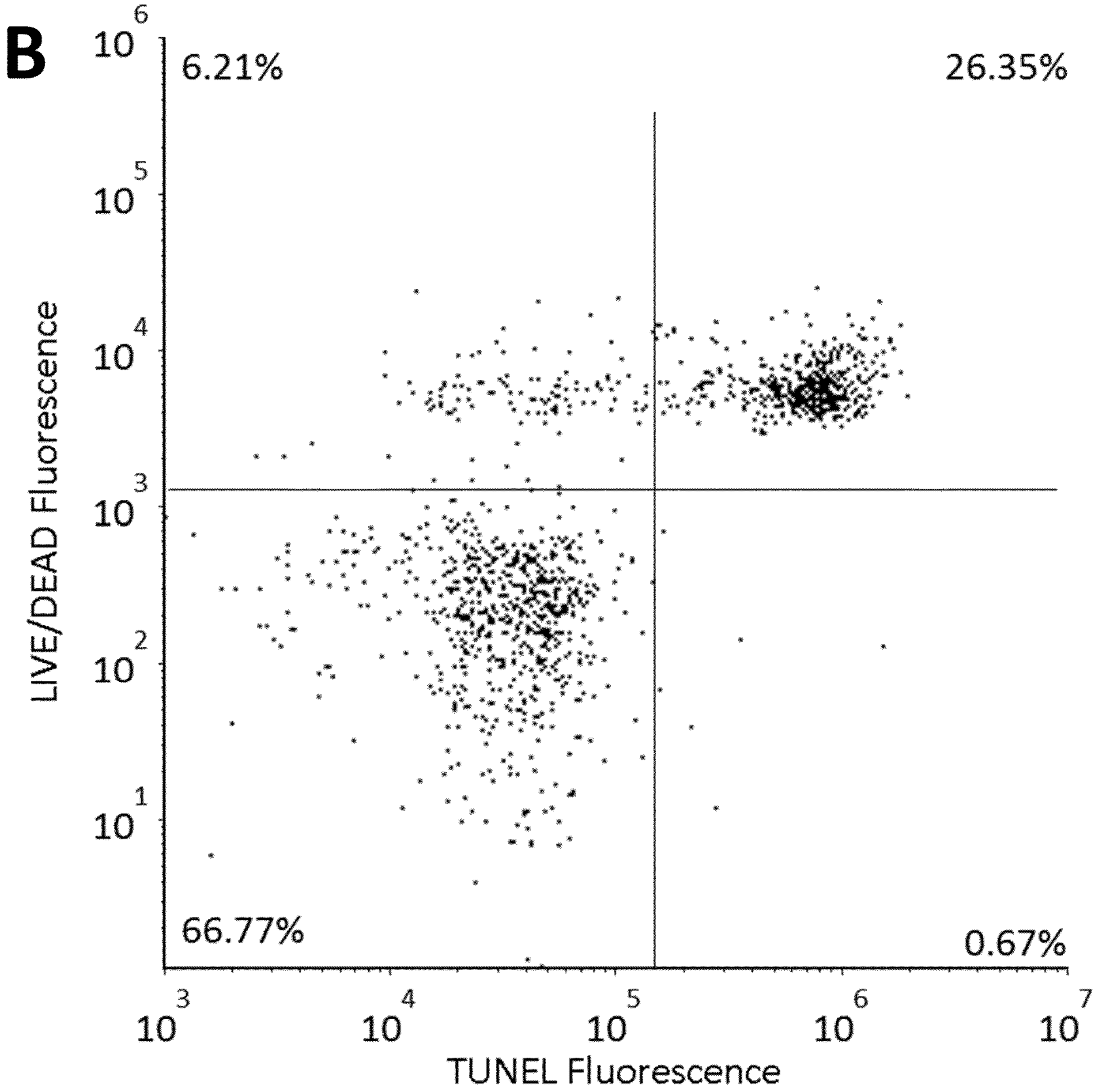

Figure 9 (cont.)

C

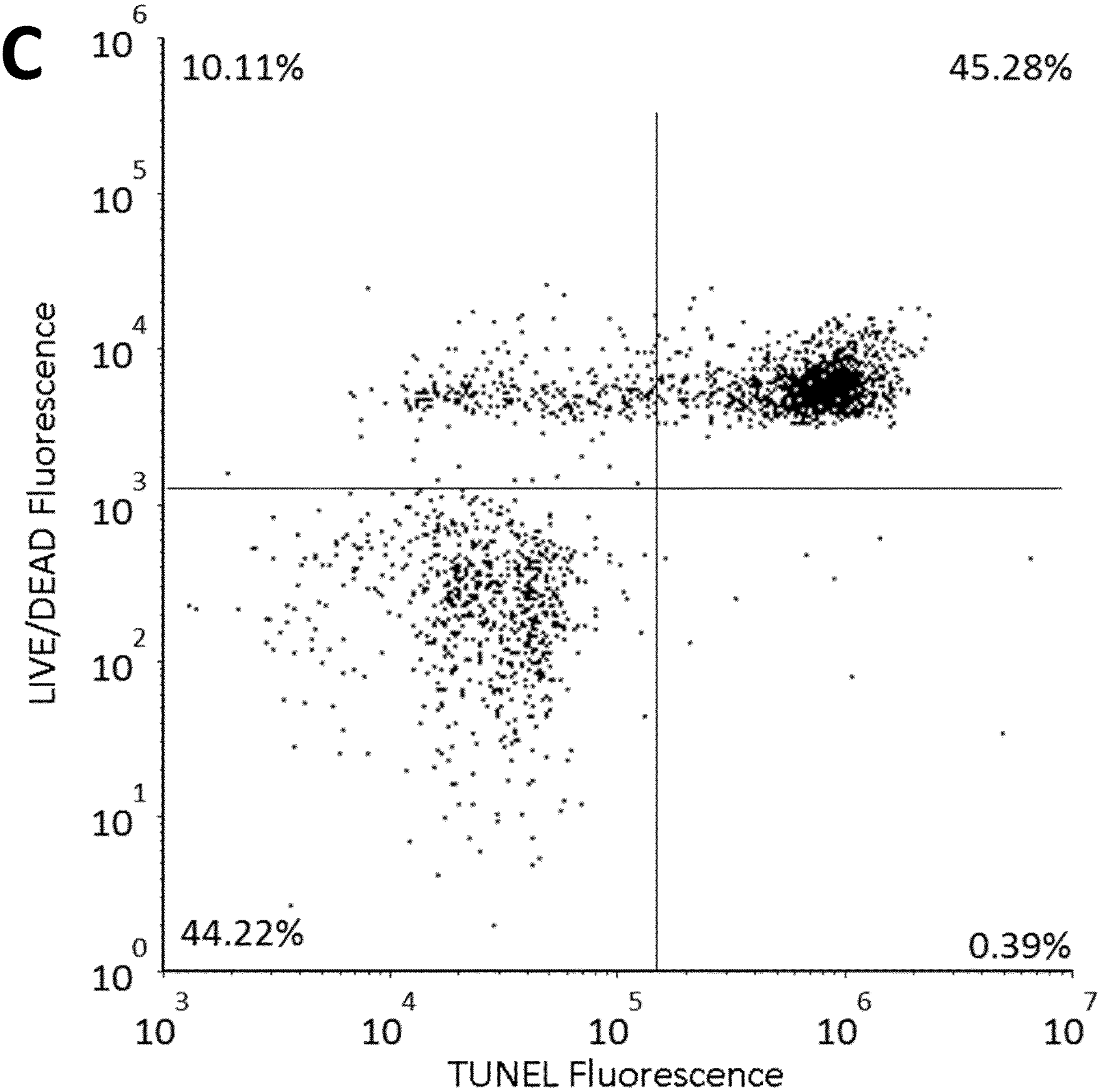

Figure 9 (cont.)

D

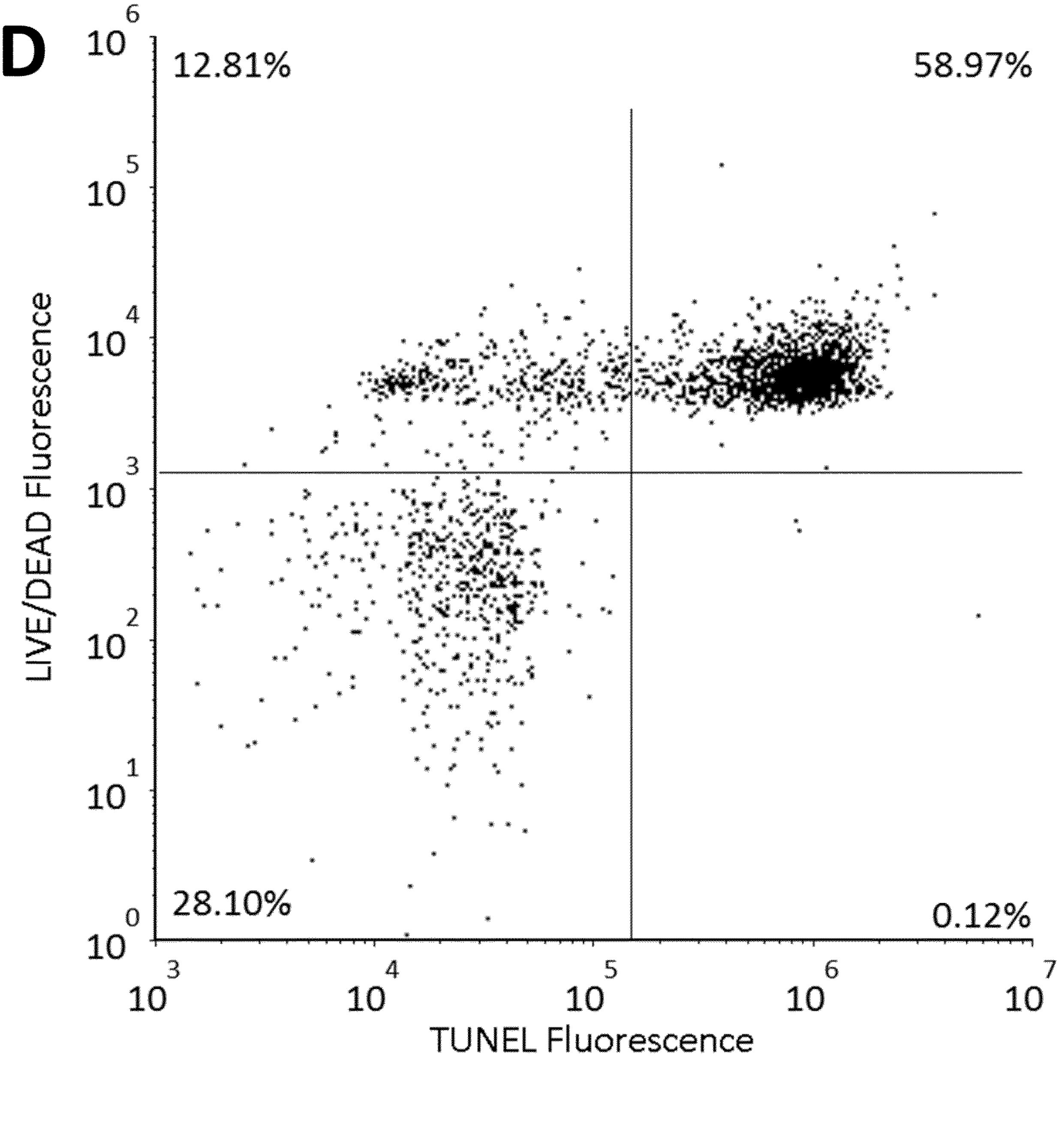

Figure 9 (cont.)

E

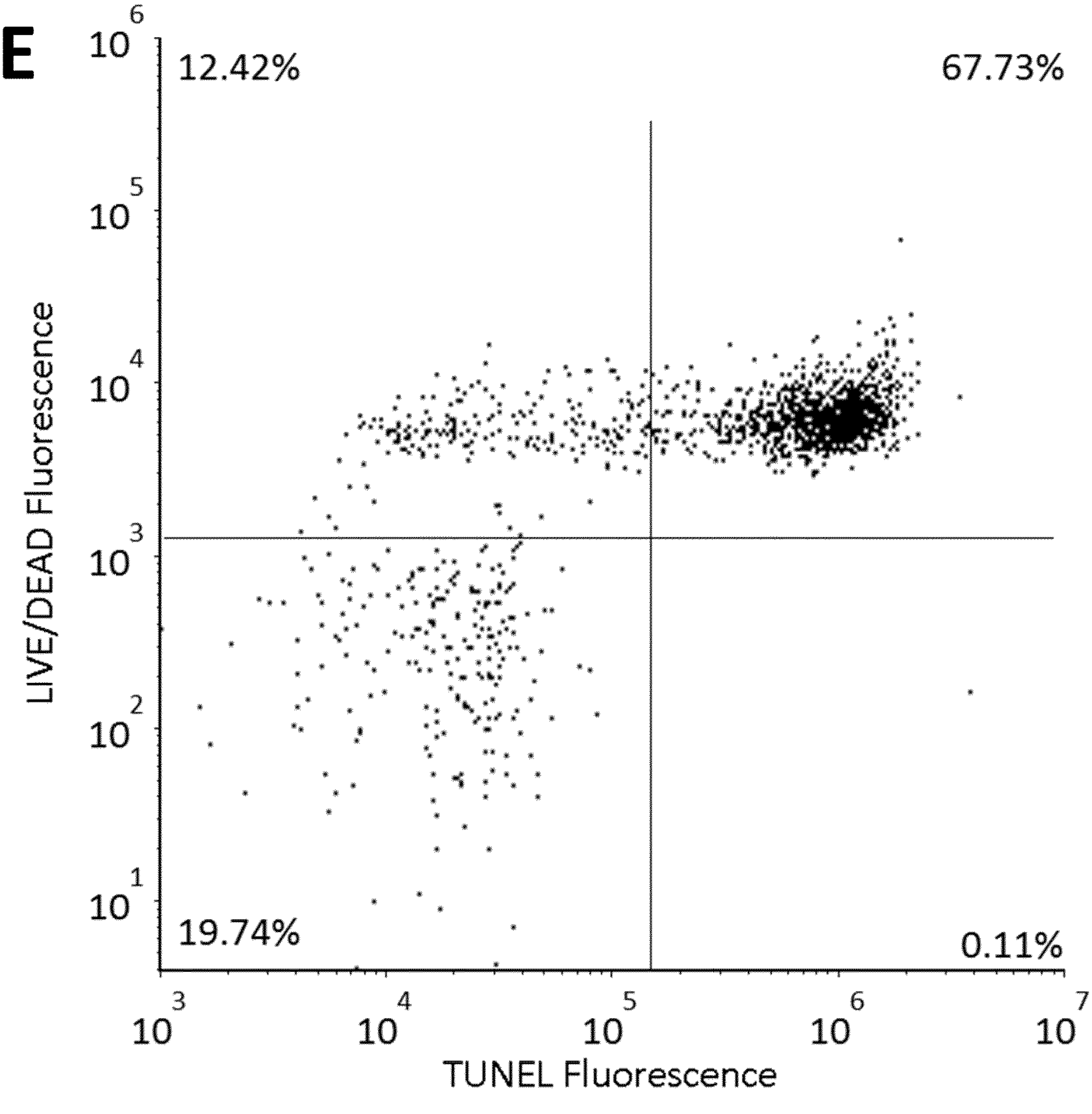

Figure 9 (cont.)

F

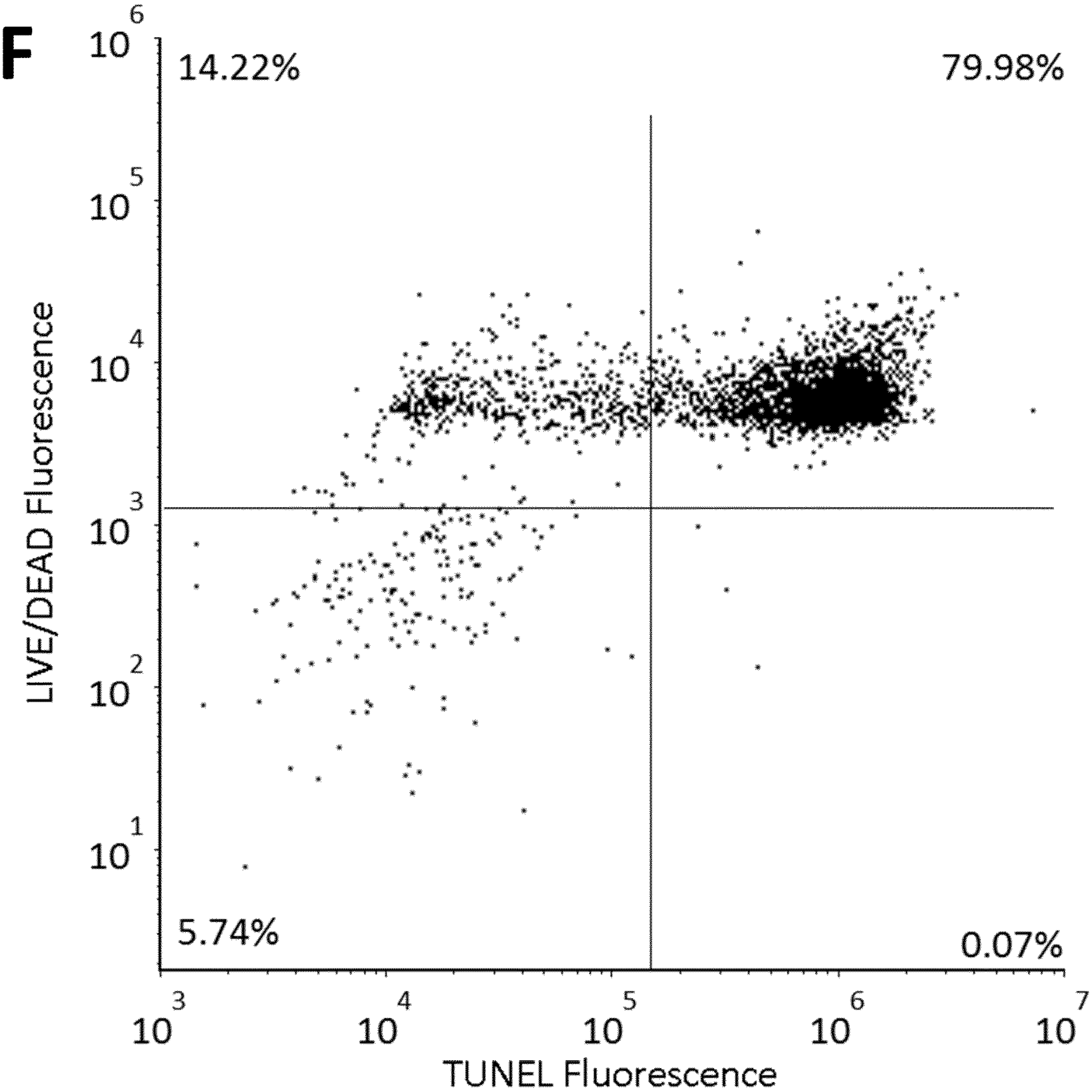

Figure 10

A

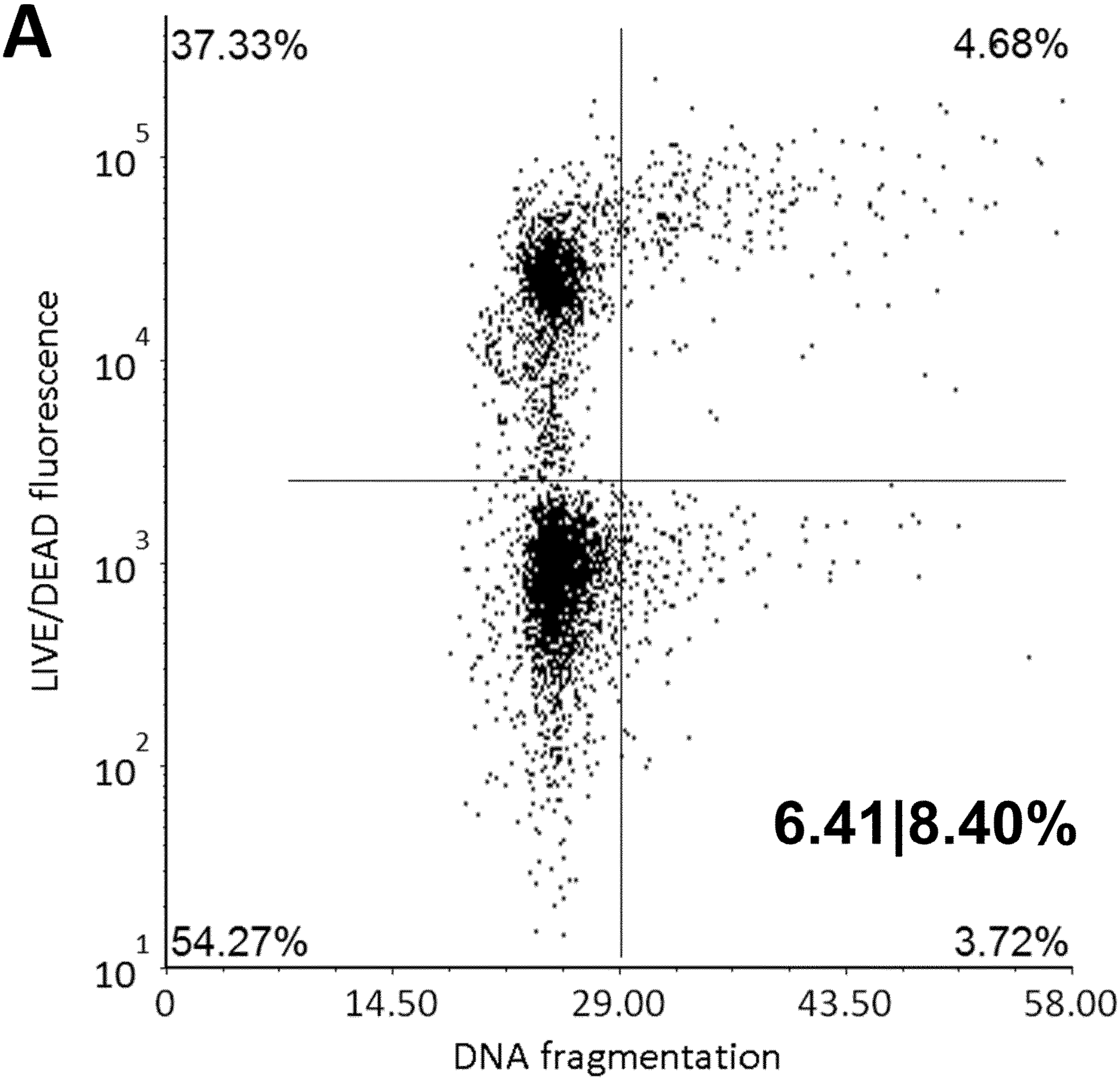
37.33%
4.68%
54.27%
3.72%
6.41|8.40%
LIVE/DEAD fluorescence
$10^5$
$10^4$
$10^3$
$10^2$
$10^1$
0
14.50
29.00
43.50
58.00
DNA fragmentation

Figure 10 (cont.)

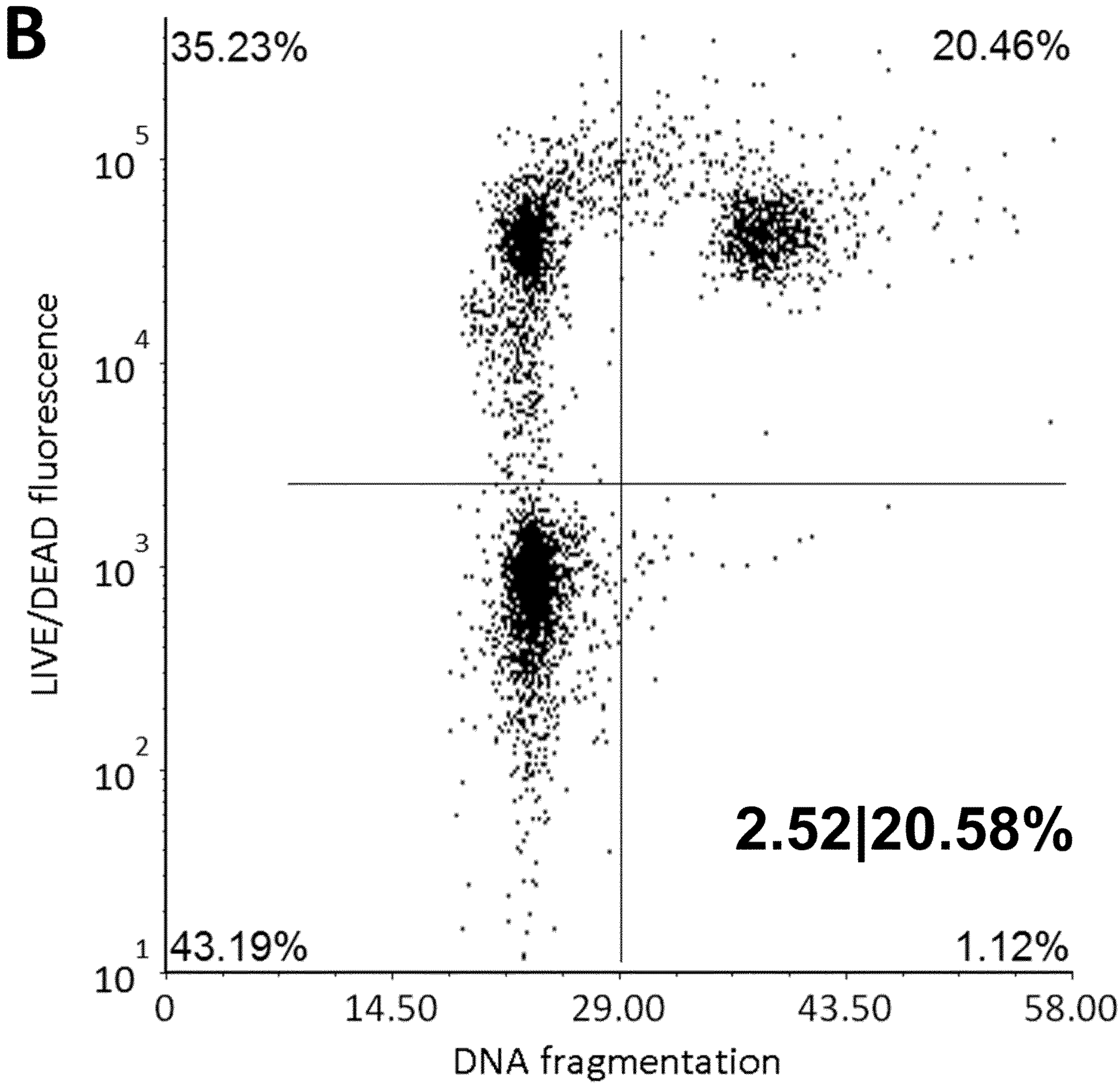

Figure 10 (cont.)

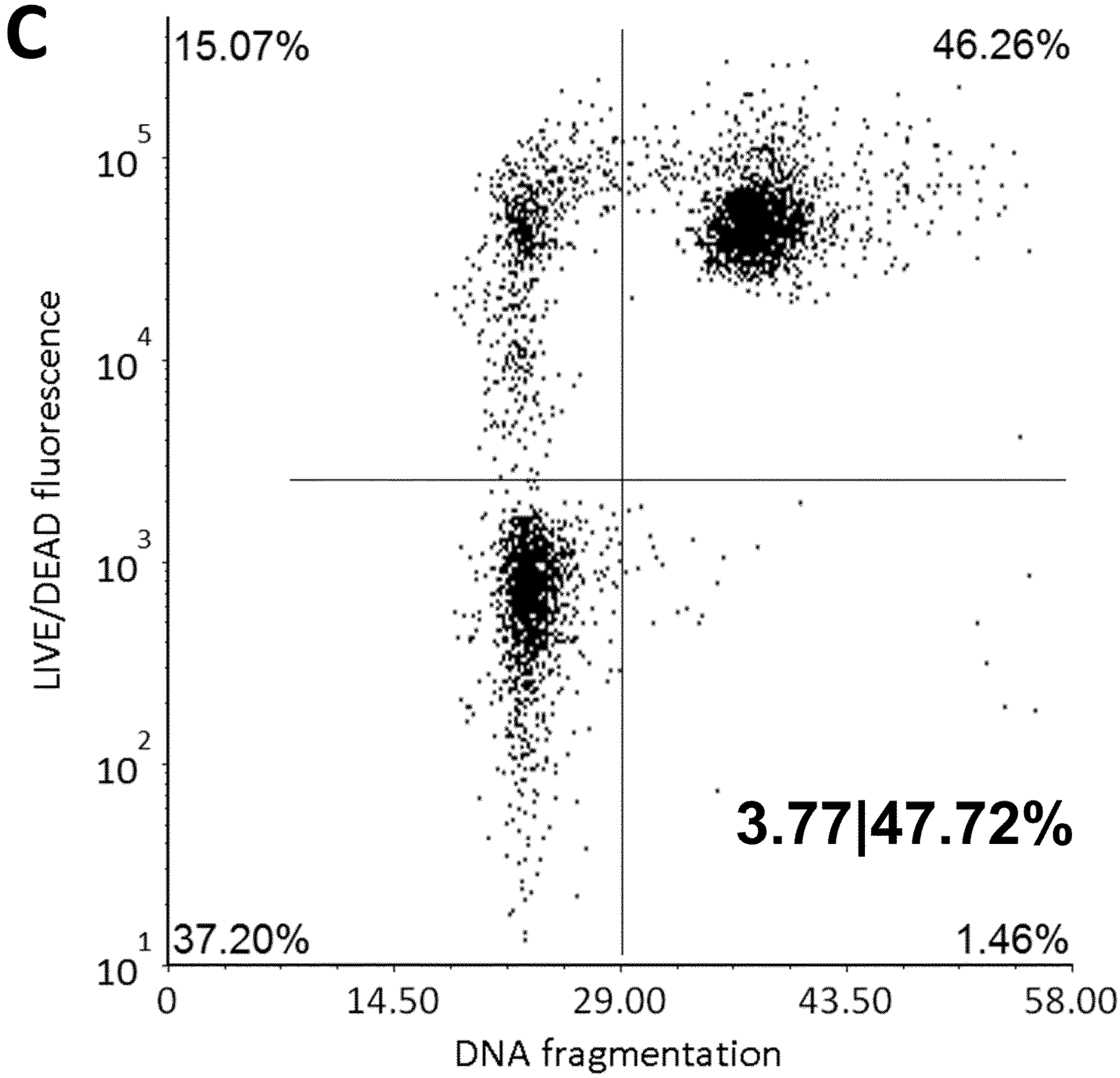

Figure 10 (cont.)

D

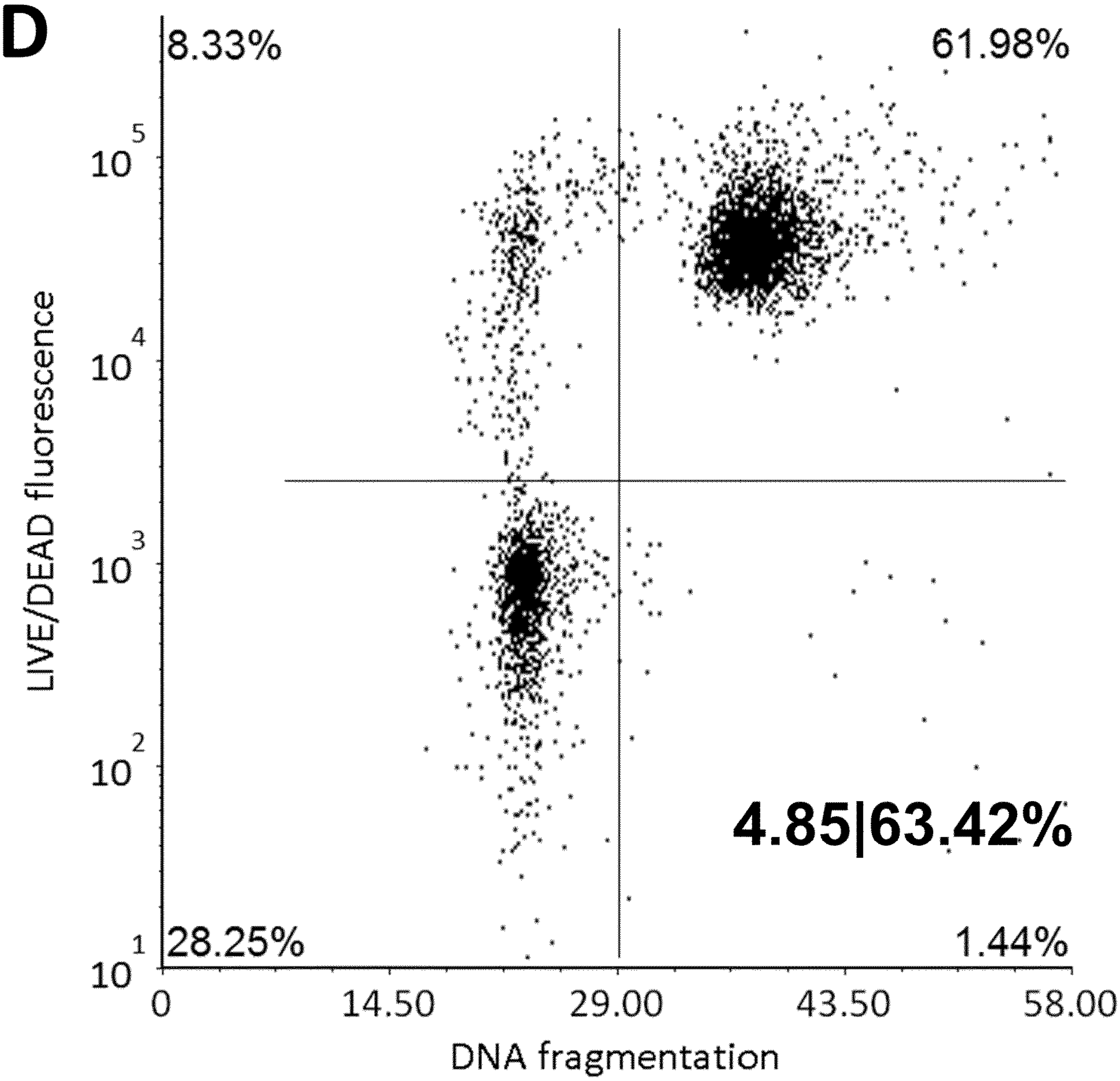

Figure 10 (cont.)

E

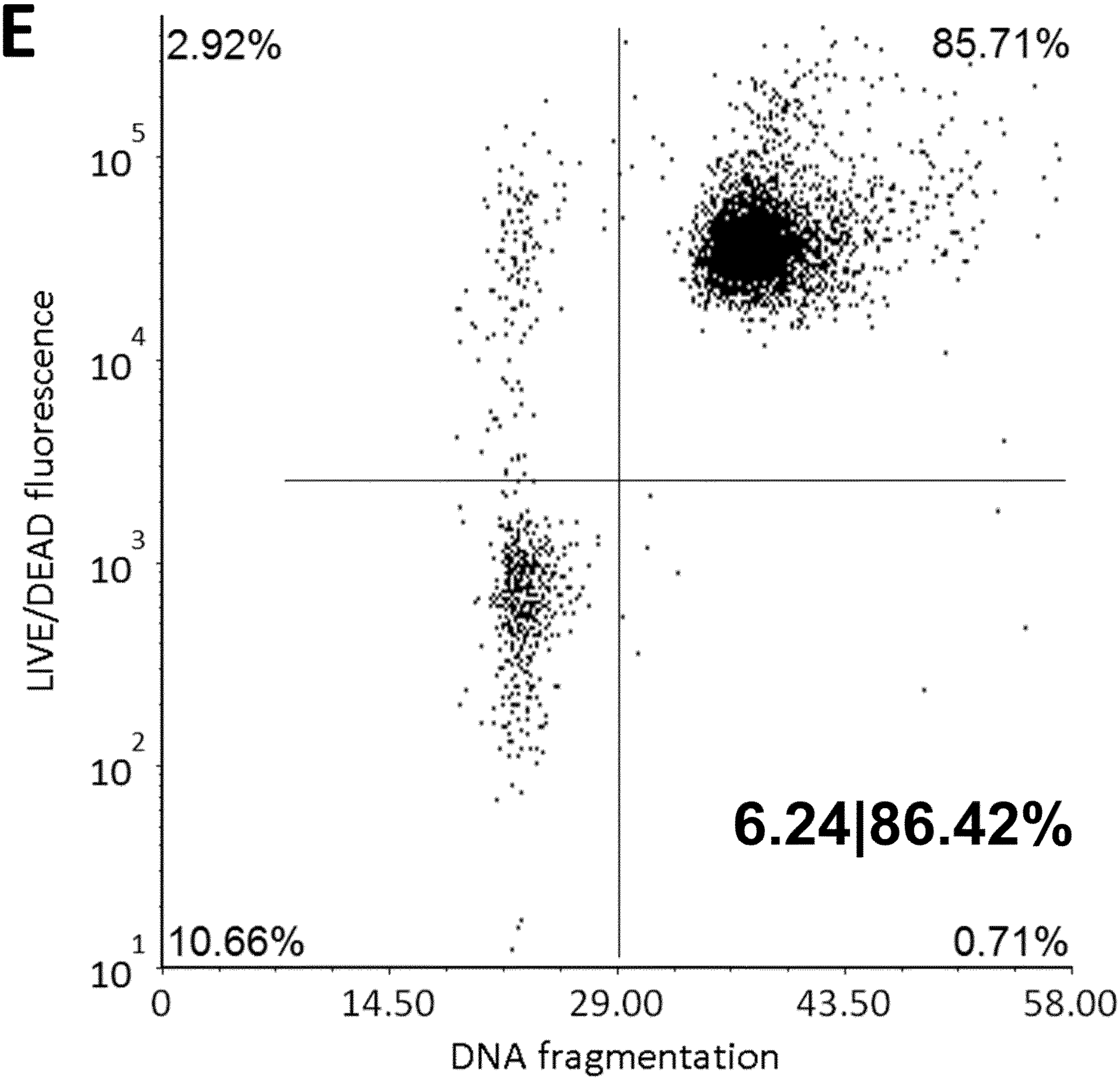

Figure 10 (cont.)

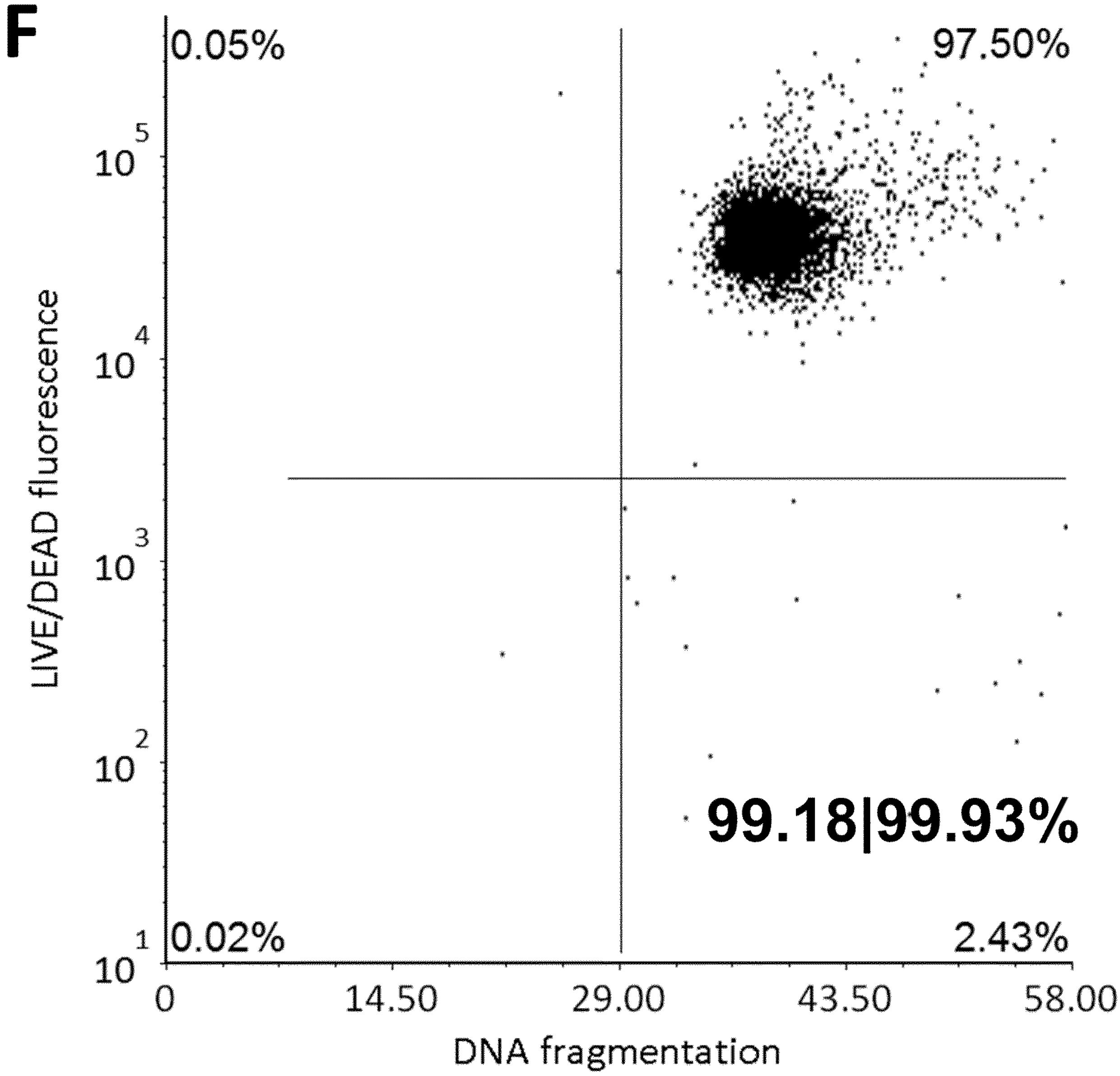

Figure 11

A

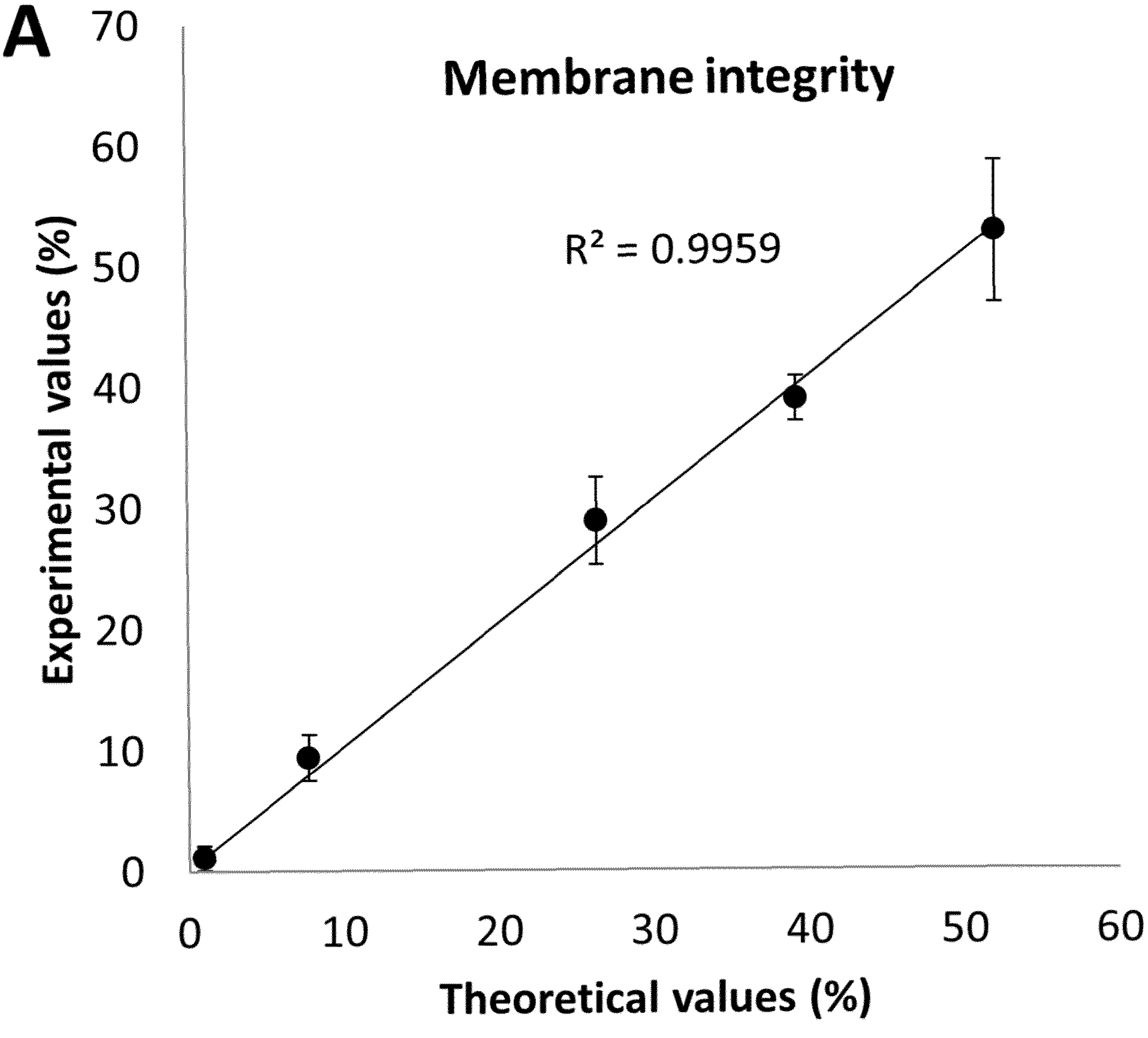
Membrane integrity
R² = 0.9959
Experimental values (%)
0
10
20
30
40
50
60
70
Theoretical values (%)
0
10
20
30
40
50
60

Figure 11 (cont.)

B

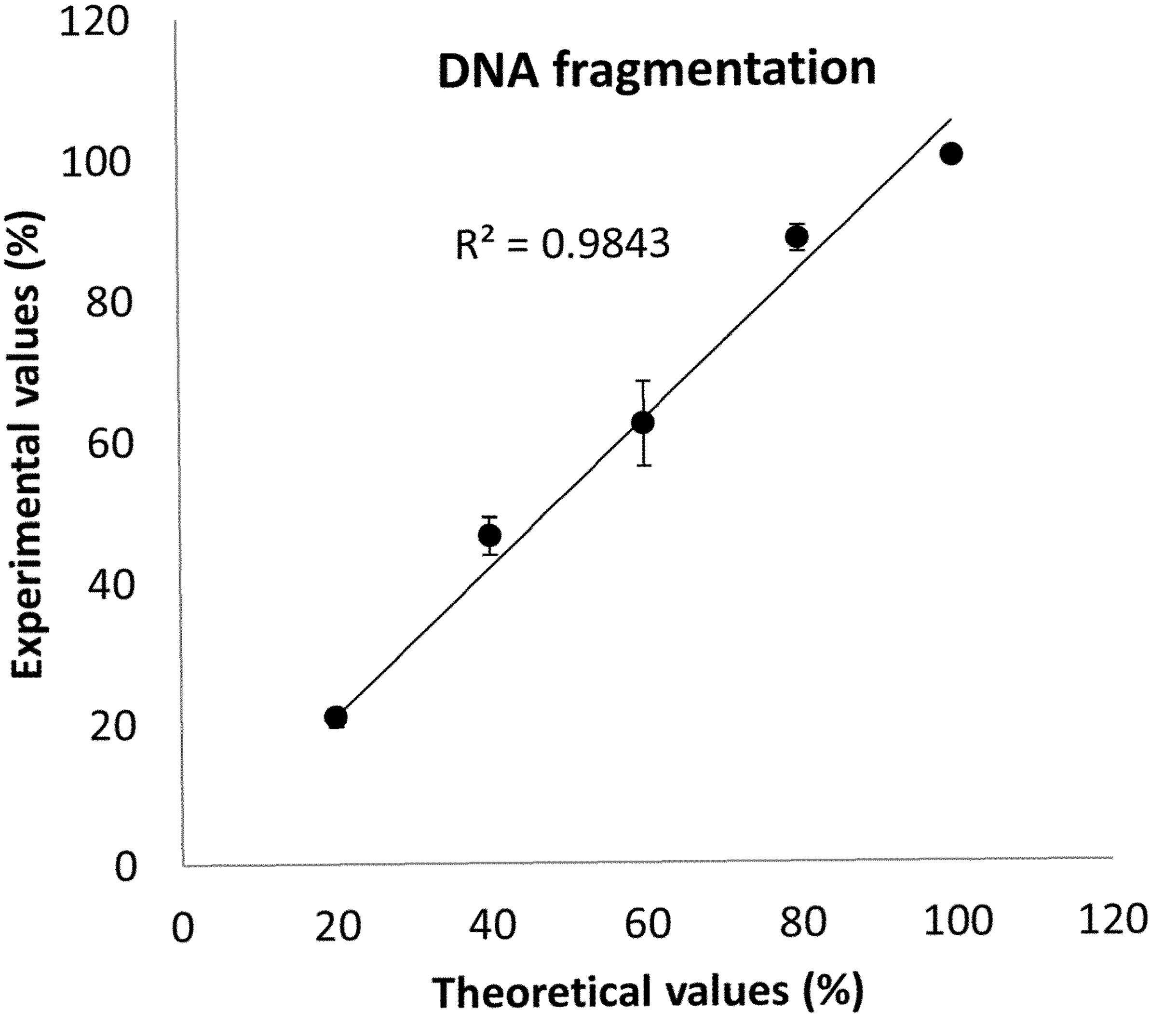

Figure 12

A

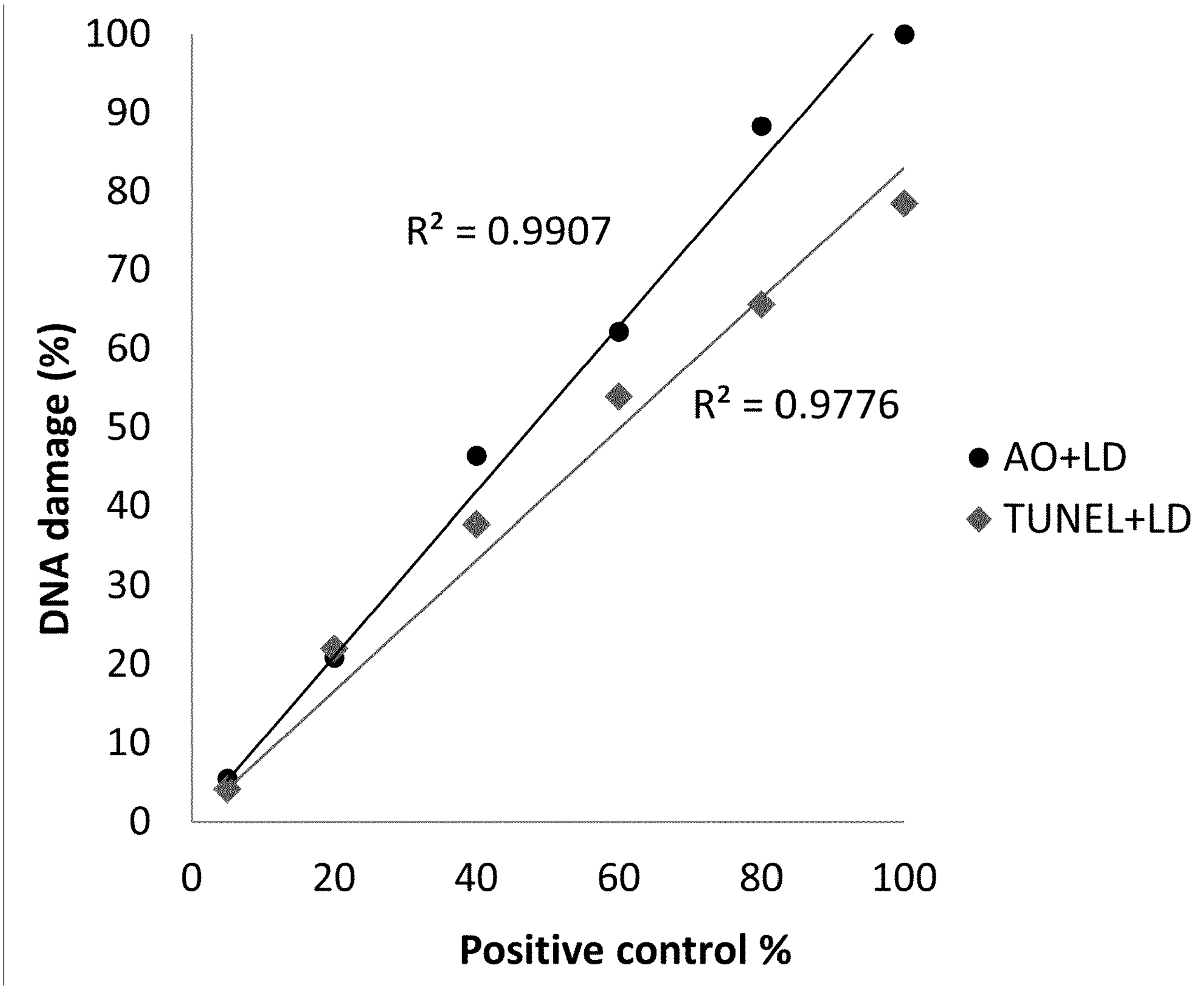
100
90
80
70
60
50
40
30
20
10
0
0
20
40
60
80
100
DNA damage (%)
Positive control %
R² = 0.9907
R² = 0.9776
AO+LD
TUNEL+LD

Figure 12 (cont.)

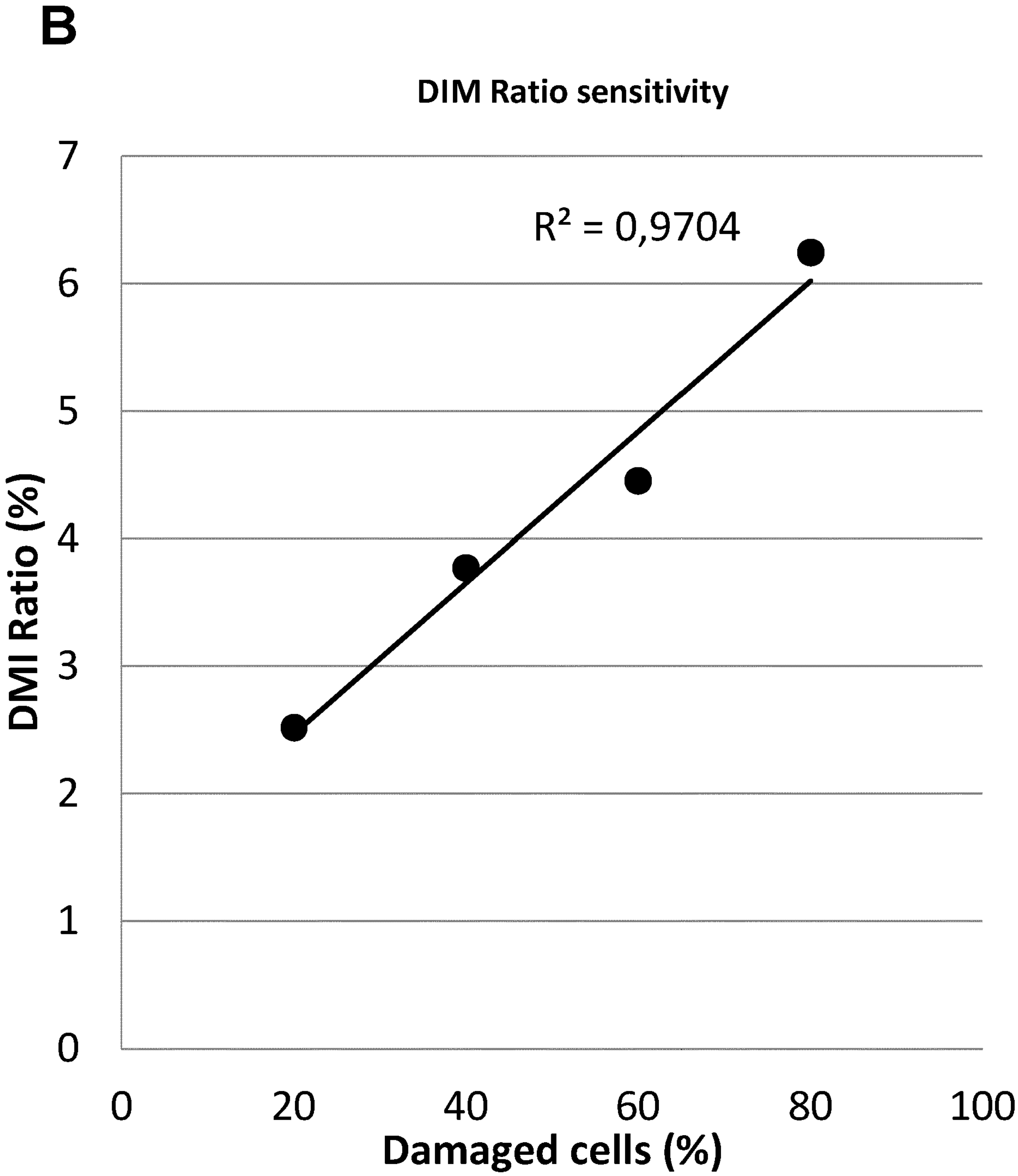

# Figure 13

## A

DNA fragmentation in human sperm assessed by Acridine Orange staining. Green sperm heads show intact DNA while Yellow to Red sperm heads show damage to DNA. https://medicine.utah.edu/surgery/andrology/research/gallery.php

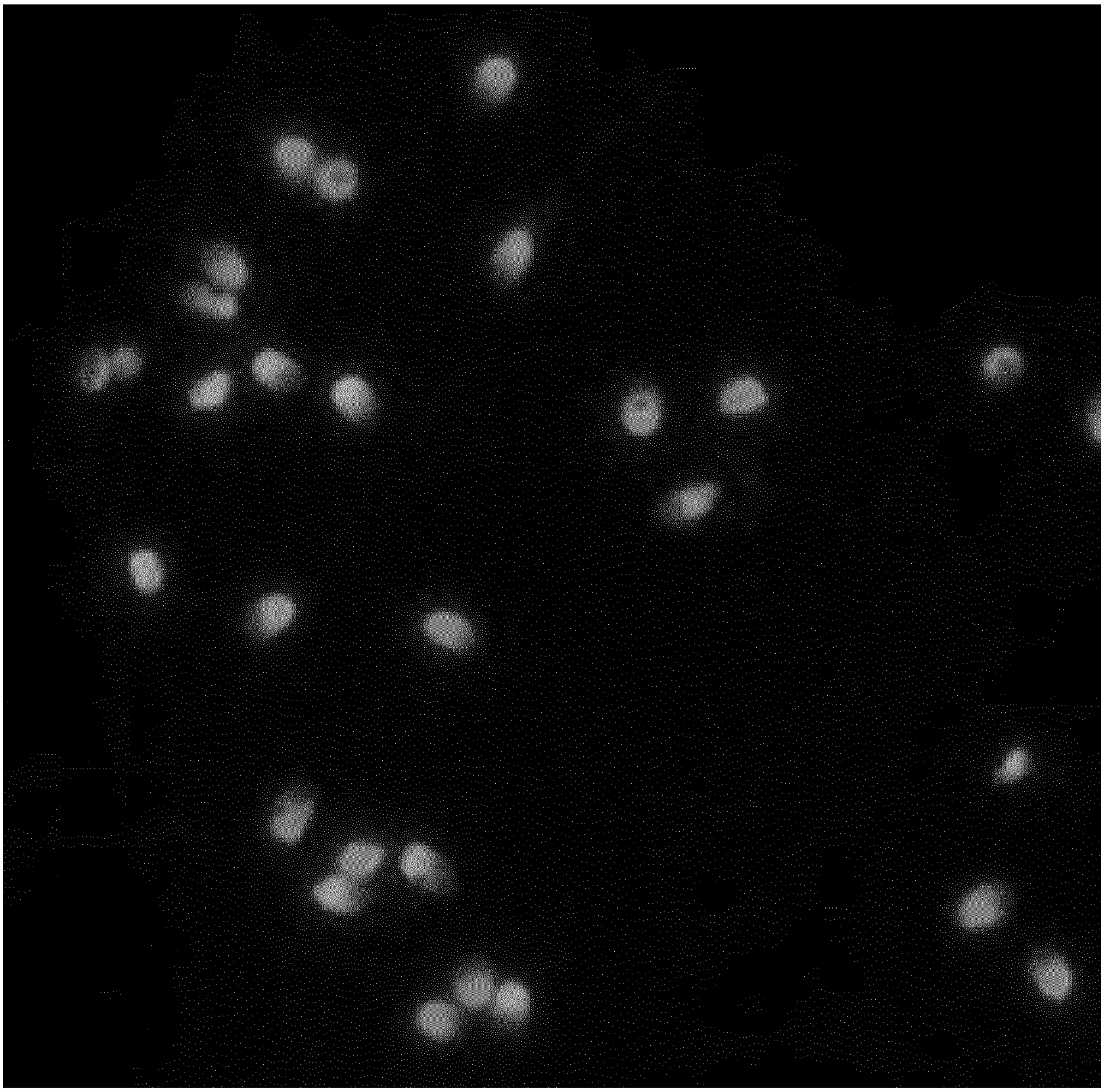

**Figure 13 (cont.)**

# B

Histogram representing individual values of DNA fragmentation (green to red fluorescence) in the analyzed sperm population.

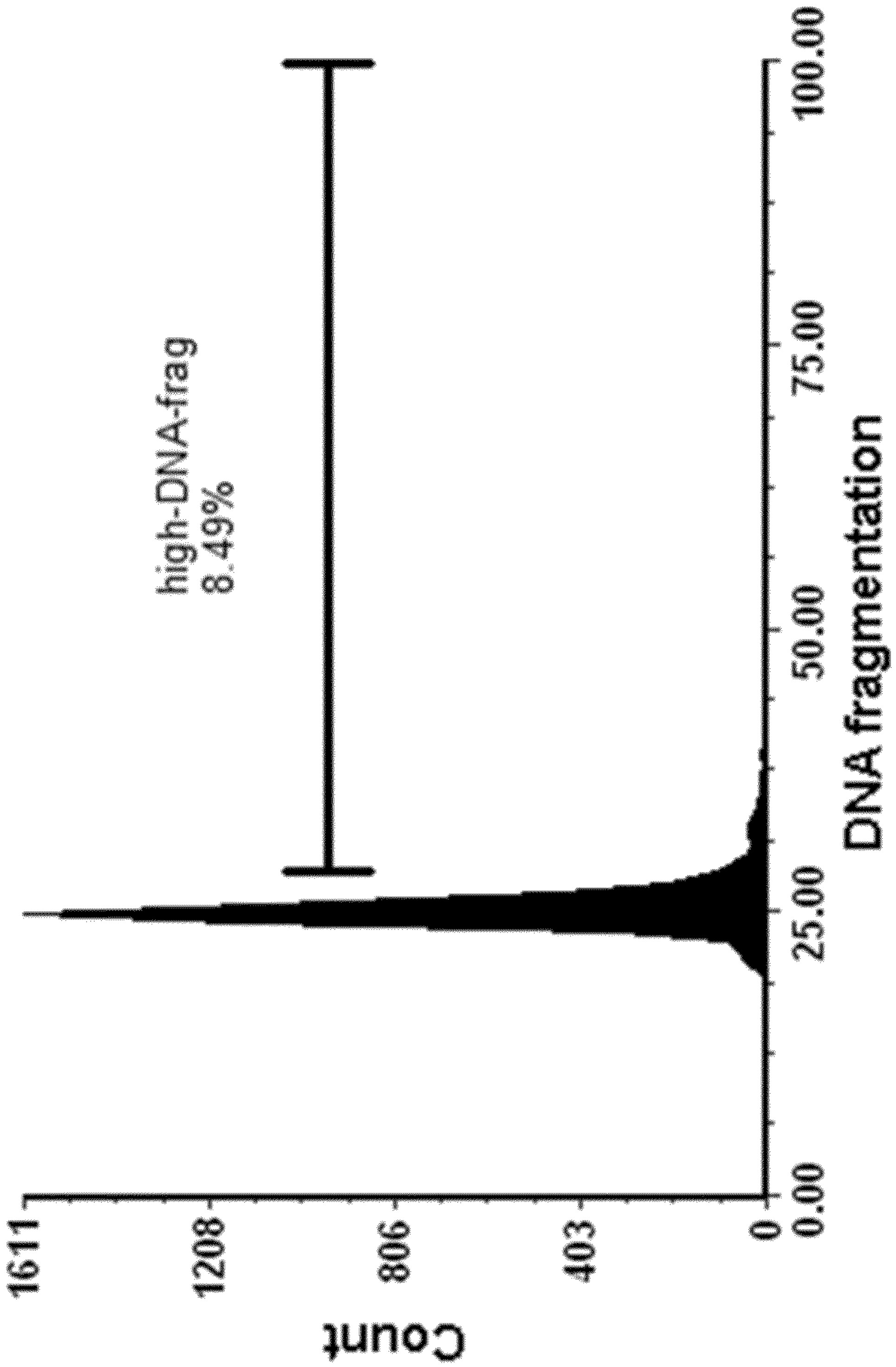

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0241906 A
- US 5150313 A
- US 5602039 A
- US 5602349 A
- US 5643796 A
- WO 9612171 A
- US 5135759 A
- US 5985216 A
- US 6071689 A
- US 6149867 A
- US 6263745 B
- WO 9933956 A
- WO 0137655 A
- US 812351
- WO 2004088283 A


### Non-patent literature cited in the description


- **MOSKOVTSEV et al.** *Archives of Andrology*, 2005, vol. 51, 33-40
- **MOSKOVTSEV S. I. et al.** *ARCHIVES OF ANDROLOGY*, 2005, vol. 51 (1), 33-40
- **CISSEN M** ; **WELY MV** ; **SCHOLTEN I** ; **MANSELL S** ; **BRUIN JPD** ; **MOL BW** ; **BRAAT D** ; **REPPING S** ; **HAMER G**. Measuring Sperm DNA Fragmentation and Clinical Outcomes of Medically Assisted Reproduction: A Systematic Review and Meta-Analysis. *PloS one*, 2016, vol. 11, e0165125-e0165125
- **COLLINS JA** ; **BARNHART KT** ; **SCHLEGEL PN**. Do sperm DNA integrity tests predict pregnancy with in vitro fertilization?. *Fertility and Sterility*, 2008, vol. 89, 823-831
- **COSSARIZZA A et al.** Guidelines for the use of flow cytomerty and cell sorting in immunological studies. *Eur J Immunol.*, 2017, vol. 47, 1584-1797
- **EVENSON D** ; **DARZYNKIEWICZ Z** ; **MELAMED M**. Relation of mammalian sperm chromatin heterogeneity to fertility. *Science*, 1980, vol. 210, 1131-1133
- Sperm Chromatin Structure Assay (SCSA). **EVENSON DP**. Spermatogenesis: Methods and Protocols. Humana Press, 2013, 147-164
- **EVENSON DP**. The Sperm Chromatin Structure Assay (SCSA®) and other sperm DNA fragmentation tests for evaluation of sperm nuclear DNA integrity as related to fertility. *Animal Reproduction Science*, 2016, vol. 169, 56-75
- **RIBEIRO SC** ; **MURATORI M** ; **DE GEYTER M** ; **DE GEYTER C**. TUNEL labeling with BrdUTP/anti-BrdUTP greatly underestimates the level of sperm DNA fragmentation in semen evaluation. *PLOS ONE*, 2017, vol. 12, e0181802
- **WHO**. WHO laboratory manual for the examination and processing of human semen. WHO Press, World Health Organization, 2010
- Thermo Fisher Scientific. *LIVE/DEAD™ Fixable Blue Dead Cell Stain Kit, for UV excitation*, 28 July 2019
- **MOSKOVTSEV SI et al.** Sperm Dna Integrity: Correlation With Sperm Plasma Membrane Integrity In Semen Evaluated For Male Infertility. *ARCHIVES OF ANDROLOGY*, 2005, vol. 51 (1), 33-40
- **QUINN P** ; **KERIN JF** ; **WARNES GM**. Improved pregnancy rate in human in vitro fertilization with the use of a medium based on the composition of human tubal fluid. *Fertil Steril*, 1985, vol. 44, 493-498
- **STRÜNKER, T.** ; **GOODWIN, N.** ; **BRENKER, C. et al.** The CatSper channel mediates progesterone-induced Ca2+ influx in human sperm. *Nature*, 2011, vol. 471, 382-386
- **GIWERCMAN A** ; **LINDSTEDT L** ; **LARSSON M et al.** Sperm chromatin structure assay as an independent predictor of fertility in vivo: a case-control study. *Int. J. Androl.*, 2010, vol. 33, 221-7